# EUROPEAN PATENT APPLICATION

(11) **EP 4 342 882 A1**
(43) Date of publication of application: **27.03.2024**
(21) Application number: 22804916.9
(22) Date of filing: 13.05.2022
(51) Int. Cl.: C07D 209/14, A61K 31/4045, A61K 45/06, A61P 35/00, C07D 209/20, A23L 33/10

(54) **BENZAMIDE DERIVATIVE, METHOD FOR PREPARING SAME, AND PHARMACEUTICAL COMPOSITION FOR PREVENTION OR TREATMENT OF CANCER CONTAINING SAME AS ACTIVE INGREDIENT**

(30) Priority: 17.05.2021 KR 20210063304
(71) Applicant: HK inno.N Corporation, Seoul 04551 (KR); Korea Research Institute of Chemical Technology, Daejeon 34114 (KR)
(72) Inventor: KANG, Seockyong, Seoul 04551 (KR); YOON, Daseul, Seoul 04551 (KR); KIM, Hyejeong, Seoul 04551 (KR); PARK, Som Yi, Seoul 04551 (KR); KIM, Dongkyu, Seoul 04551 (KR); PARK, Ji-Yeon, Seoul 04551 (KR); BYEON, Yeji, Seoul 04551 (KR); JO, Hye-Im, Seoul 04551 (KR); JUNG, Seung Hee, Seoul 04551 (KR); CHOI, Seong-Il, Seoul 04551 (KR); LEE, Seung Chul, Seoul 04551 (KR); LEE, Kwangho, Daejeon 34114 (KR)
(74) Representative: Abel & Imray LLP
(86) International application number: PCT/KR2022/006895
(87) International publication number: WO 2022/245061

(57) **Abstract**

The present invention relates to a benzamide derivative, a preparation method thereof, and a pharmaceutical composition for preventing or treating cancer containing the same as an active ingredient. The benzamide derivative provided in one aspect of the present invention can be used for the prevention or treatment of cancer by inhibiting EGFR mutation, and can be effectively used as an anticancer agent since it exhibits a significant synergistic effect when co-administered with an EGFR antagonist such as Cetuximab.

## Description

### BACKGROUND OF THE INVENTION

### 1. Field of the Invention

The present invention relates to a benzamide derivative, a preparation method thereof, and a pharmaceutical composition for preventing or treating cancer containing the same as an active ingredient.

### 2. Description of the Related Art

The incidence of cancer is related to various environmental factors including chemicals, radiation, and viruses, as well as changes in oncogenes, tumor suppressor genes, and genes related to apoptosis and DNA repair. Recent understanding of the molecular mechanisms of cancer has enabled targeted anticancer therapy, a new treatment.

Targeted agents are generally made to target the molecules that cancer cells characteristically have to show their effectiveness. Molecular targets are genes related to cancer cell signal transduction pathway, angiogenesis, matrix, cell cycle regulator, and apoptosis. Currently, 'signal transduction pathway inhibitors' including tyrosine kinase inhibitors and 'angiogenesis inhibitors' are used as important targeted agents in cancer treatment.

Protein tyrosine kinase has been known to play an important role in many malignant tumors. In particular, epidermal growth factor receptor (EGFR), a receptor tyrosine kinase of the erbB family, is abnormally activated in many epithelial cell tumors including non-small cell lung carcinoma (NSCLC), breast cancer, glioma, squamous cell carcinoma of the head and neck, colon cancer, rectal carcinoma, head and neck cancer, stomach cancer, and prostate cancer, and it has been known that the activation of the EGFR-tyrosine kinase causes continuous cell proliferation, invasion of surrounding tissues, distant metastasis, blood vessel formation, and increases cell survival.

Particularly, EGFR is one of the ErbB tyrosine kinase receptors family (EGFR, HER-2, ErbB-3, ErbB-4), and is a transmembrane tyrosine kinase having an intracellular domain including an extracellular ligand-binding domain and a tyrosine kinase domain. When a ligand is bound to a receptor that forms a homodimer or heterodimer, the intracellular tyrosine kinase is activated, and the signal stimulated by EGFR activates phosphatidylinositol 3-kinase (PI3K/AKT/mTOR, RAS/RAF/MAPK, JAK/STAT) signaling pathway (Non-patent reference 1, Nat Rev Cancer 2007;7:169-81. Epidermal growth factor receptor mutations in lung cancer).

In particular, EGFR is overexpressed in more than half of non-small cell lung cancer (NSCLC), and many studies have been conducted with EGFR as a target of treatment. EGFR TKI (tyrosine kinase inhibitor), which inhibits EGFR tyrosine kinase activity, has been developed, and the representative drugs include gefitinib (IRESSA^{™}), erlotinib (TARCEVA^{™}), and lapatinib (TYKERB^{™}, TYVERB^{™}).

On the other hand, in 2004, it was reported that the activation mutation of EGFR is correlated with the response to gefitinib therapy in non-small-cell lung cancer (NSCLC). Particularly, the EGFR mutation is largely classified into a sensitizing mutation and a resistant mutation, and the deletion of exon 19 and the L858R point mutation of exon 21 are the most important sensitizing mutations, accounting for about 85-90%, and the exon 19 mutation is known to have better sensitivity to TKI. On the other hand, the T790M point mutation of exon 20 is the most important resistant mutation and is known to be found in more than 50% of acquired resistance patients.

Somatic mutations identified so far include intraframe deletion in exon 19 or insertions in exon 20, as well as point mutations in which a single nucleic acid residue is modified in the expressed protein (e.g., L858R, G719S, G719C, G719A and L861Q).

Despite the initial clinical effects of gefitinib/erlotinib on NSCLC patients with EGFR mutations, advanced cancer eventually develops in most patients during therapy with these agents. Early studies of relapsed specimens identified a secondary EGFR mutation, T790M, which makes zepithinib and erlotinib ineffective inhibitors of EGFR kinase activity. It was demonstrated in subsequent studies that the EGFR T790M mutation was found in approximately 50% (24/48) of tumors of patients who acquired resistance to gefitinib or erlotinib. This secondary genetic modification occurs at a position similar to the 'gatekeeper' residue and the secondary resistance allele associated with it in patients treated with kinase inhibitors (e.g., T315I in ABL in imatinib resistant CML).

It has long been known that the EGFR mutation, EGFR_del19 or EGFR_L858R, is the major cause of non-small cell lung cancer and head and neck cancer, and their therapeutic drugs, Iressa and Taseba, have been developed and are currently used in clinical trials. However, when these drugs were used in patients, acquired resistance was observed, resulting in EGFR secondary mutations based on the structure of the drug, and it was also found that this is the main cause of actual drug resistance. When the first generation EGFR inhibitors are used for an average of 10 months, the acquired resistance, the T790M mutation located in the gatekeeper of the EGFR kinase, occurs, and the first generation EGFR inhibitors are not effective. That is, EGFR_del19_T790M or EGFR_L858R_T790M double mutation occurs, and the conventional therapeutic agents do not show efficacy.

Based on these facts, the need for the development of 2^{nd} and 3^{rd} generation drugs with excellent drug efficacy and new structures emerged.

In the past 10 years, various 3^{rd} generation new drug candidates that have an effect on the double mutation of EGFR T790M have been discovered and clinical studies are in progress, and the most advanced of them is AZD9291 of AstraZeneca, a multinational pharmaceutical company. However, it has been reported that resistance to AZD9291 occurs in about 10 months, resulting in loss of the drug efficacy of AZD9291, and in particular, resistance to triple mutations including C797S has been reported.

Accordingly, there is a need for the development of inhibitors that exhibit relatively low inhibition of WT EGFR and higher inhibition of specific activated or resistant mutant forms of EGFR.

### SUMMARY OF THE INVENTION

It is an object of the present invention to provide a benzamide derivative capable of preventing or treating cancer by inhibiting EGFR mutation.

It is another object of the present invention to provide a pharmaceutical composition for preventing or treating cancer comprising the benzamide derivative as an active ingredient.

It is another object of the present invention to provide a health functional food for preventing or ameliorating cancer comprising the benzamide derivative as an active ingredient.

It is another object of the present invention to provide a combination formulation for preventing or treating cancer comprising the benzamide derivative as an active ingredient.

To achieve the above objects, in one aspect of the present invention, the present invention provides a compound represented by formula 1, an optical isomer thereof, a solvate thereof, a hydrate thereof or a pharmaceutically acceptable salt thereof.

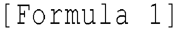

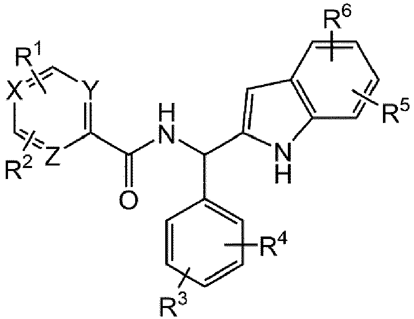

In formula 1,
X, Y and Z are independently carbon or nitrogen atoms;
R¹ is -H, -OH, halogen, C₁₋₁₀ alkyl unsubstituted or substituted with one or more halogens, C₁₋₁₀ alkoxy unsubstituted or substituted with one or more halogens, nitro, C₁₋₁₀ alkylsulfanyl, cyano, amino, C₁₋₁₀ alkylamino, or 5-6 membered heteroaryl;
R² is substituted C₆₋₁₀ aryl, or substituted 5-10 membered heteroaryl,
wherein the substituted C₆₋₁₀ aryl or substituted 5-10 membered heteroaryl is independently substituted aryl or heteroaryl substituted with one or more substituents selected from the group consisting of halogen, unsubstituted or substituted C₁₋₁₀ alkyl and unsubstituted or substituted 4-10 membered fully or partially saturated heterocycloalkyl,
wherein the substituted C₁₋₁₀ alkyl is substituted with 5-6 membered heterocycloalkyl in which 1 or 2 C₁₋₅ alkyls are substituted with substituted amino,
the substituted 4-10 membered fully or partially saturated heterocycloalkyl is 4-10 membered heterocycloalkyl substituted with one or more substituents selected from the group consisting of - OH, halogen, C₁₋₅ alkyl unsubstituted or substituted with one or more halogens, C₁₋₅ alkyl substituted with 5-6 membered heterocycloalkyl, C₁₋₅ alkoxy unsubstituted or substituted with one or more halogens, nitro, C₁₋₅ alkylsulfanyl, cyano, amino unsubstituted or substituted with 1 or 2 C₁₋₅ alkyls, 5-6 membered heterocycloalkyl unsubstituted or substituted with C₁₋₅ alkyl, C₁₋₅ alkoxycarbonyl, 5-6 membered heteroaryl, C₆₋₁₀ arylaminocarbonyl and C₁₋₅ alkoxy-C₁₋₅ alkoxy- C₆₋₁₀ aryl;
   - R³: is halogen;
   - R⁴: is -OH, halogen or C₁₋₁₅ alkoxy;
   - R⁵: is -H, -OH, halogen, C₁₋₄ alkyl or C₁₋₄ alkoxy; and
   - R⁶: is -H, -OH, halogen, C₁₋₄ alkyl or C₁₋₄ alkoxy.

In another aspect of the present invention, the present invention provides a pharmaceutical composition comprising a compound represented by formula 1, an optical isomer thereof, a solvate thereof, a hydrate thereof or a pharmaceutically acceptable salt thereof as an active ingredient for the prevention or treatment of cancer.

In another aspect of the present invention, the present invention provides a health functional food composition comprising a compound represented by formula 1, an optical isomer thereof, a solvate thereof, a hydrate thereof or a pharmaceutically acceptable salt thereof as an active ingredient for the prevention or amelioration of cancer.

In another aspect of the present invention, the present invention provides a combination formulation comprising a compound represented by formula 1, an optical isomer thereof, a solvate thereof, a hydrate thereof or a pharmaceutically acceptable salt thereof as an active ingredient for the prevention or amelioration of cancer.

### ADVANTAGEOUS EFFECT

The benzamide derivative provided in one aspect of the present invention can be used for the prevention or treatment of cancer by inhibiting EGFR mutation, and can be effectively used as an anticancer agent since it exhibits a significant synergistic effect when co-administered with an EGFR antagonist such as cetuximab.

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

The term "alkylene", "alkenyl" or "alkyl" includes straight or branched saturated hydrocarbon residues, unless otherwise specified. For example, "C₁₋₆ alkyl" refers to alkyl having a backbone of 1 to 6 carbons. Specifically, C₁₋₆ alkyl may include methyl, ethyl, n-propyl, i-propyl, n-butyl, i-butyl, t-butyl, n-pentyl, i-pentyl, t-pentyl, sec-pentyl, neopentyl, hexyl, and the like.

The term "cycloalkyl", unless otherwise specified, includes carbocyclic groups containing carbon atoms. For example, "C₃₋₈ cycloalkyl" refers to cycloalkyl with a backbone of 3 to 8 carbons. Specifically, C₃₋₈ cycloalkyl may include cyclopropyl, cyclobutyl, cyclopentyl, cyclohexyl, and the like.

The term "heterocycloalkyl", unless otherwise specified, includes a monovalent saturated residue consisting of 1 to 3 rings containing 1, 2, 3 or 4 heteroatoms selected from N, O or S. Two or three rings may include bridged, fused or spiro heterocycloalkyl.

The term "heteroaryl", unless otherwise specified, may include aromatic radicals with a single ring or two or three fused rings having one or more aromatic rings containing 1, 2, 3 or 4 ring heteroatoms selected from N, O or S.

In one aspect of the present invention, the present invention provides a compound represented by formula 1, an optical isomer thereof, a solvate thereof, a hydrate thereof or a pharmaceutically acceptable salt thereof.

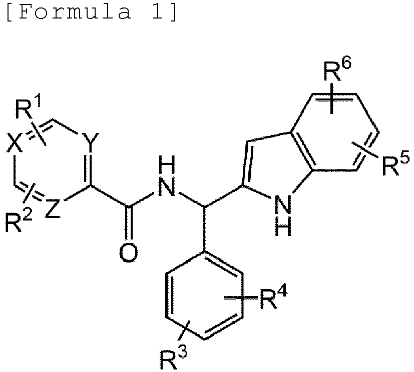

In formula 1,
X, Y and Z are independently carbon or nitrogen atoms;
R¹ is -H, -OH, halogen, C₁₋₁₀ alkyl unsubstituted or substituted with one or more halogens, C₁₋₁₀ alkoxy unsubstituted or substituted with one or more halogens, nitro, C₁₋₁₀ alkylsulfanyl, cyano, amino, C₁₋₁₀ alkylamino, or 5-6 membered heteroaryl;
R² is substituted C₆₋₁₀ aryl, or substituted 5-10 membered heteroaryl,
wherein the substituted C₆₋₁₀ aryl or substituted 5-10 membered heteroaryl is independently substituted aryl or heteroaryl substituted with one or more substituents selected from the group consisting of halogen, unsubstituted or substituted C₁₋₁₀ alkyl and unsubstituted or substituted 4-10 membered fully or partially saturated heterocycloalkyl,
wherein the substituted C₁₋₁₀ alkyl is substituted with 5-6 membered heterocycloalkyl in which 1 or 2 C₁₋₅ alkyls are substituted with substituted amino,
the substituted 4-10 membered fully or partially saturated heterocycloalkyl is 4-10 membered heterocycloalkyl substituted with one or more substituents selected from the group consisting of - OH, halogen, C₁₋₅ alkyl unsubstituted or substituted with one or more halogens, C₁₋₅ alkyl substituted with 5-6 membered heterocycloalkyl, C₁₋₅ alkoxy unsubstituted or substituted with one or more halogens, nitro, C₁₋₅ alkylsulfanyl, cyano, amino unsubstituted or substituted with 1 or 2 C₁₋₅ alkyls, 5-6 membered heterocycloalkyl unsubstituted or substituted with C₁₋₅ alkyl, C₁₋₅ alkoxycarbonyl, 5-6 membered heteroaryl, C₆₋₁₀ arylaminocarbonyl and C₁₋₅ alkoxy-C₁₋₅ alkoxy-C₆₋₁₀ aryl;
R³ is halogen;
R⁴ is -OH, halogen or C₁₋₁₅ alkoxy;
R⁵ is -H, -OH, halogen, C₁₋₄ alkyl or C₁₋₄ alkoxy; and
R⁶ is -H, -OH, halogen, C₁₋₄ alkyl or C₁₋₄ alkoxy.

In another aspect,
X, Y and Z are independently carbon or nitrogen atoms;
R¹ is -H, -OH, halogen, C₁₋₅ alkyl unsubstituted or substituted with one or more halogens, C₁₋₅ alkoxy unsubstituted or substituted with one or more halogens, nitro, C₁₋₅ alkylsulfanyl, cyano, amino, C₁₋₅ alkylamino, or 5-6 membered heteroaryl;
R² is substituted C₆₋₁₀ aryl, or substituted 5-6 membered heteroaryl,
wherein the substituted C₆₋₁₀ aryl or substituted 5-6 membered heteroaryl is independently aryl or heteroaryl substituted with one or more substituents selected from the group consisting of halogen, unsubstituted or substituted C₁₋₅ alkyl and unsubstituted or substituted 4-8 membered fully or partially saturated heterocycloalkyl containing at least one N,
wherein the substituted C₁₋₅ alkyl is substituted with 6 membered heterocycloalkyl in which 2 C₁₋₅ alkyls are substituted with substituted amino,
the substituted 4-8 membered fully or partially saturated heterocycloalkyl is 4-8 membered heterocycloalkyl substituted with one or more substituents selected from the group consisting of - OH, halogen, C₁₋₅ alkyl unsubstituted or substituted with one or more halogens, C₁₋₅ alkyl substituted with 5-6 membered heterocycloalkyl, C₁₋₅ alkoxy unsubstituted or substituted with one or more halogens, nitro, C₁₋₅ alkylsulfanyl, cyano, amino unsubstituted or substituted with 1 or 2 C₁₋₅ alkyls, 5-6 membered heterocycloalkyl unsubstituted or substituted with C₁₋₅ alkyl, C₁₋₅ alkoxycarbonyl, 5-6 membered heteroaryl, C₆₋₁₀ arylaminocarbonyl and C₁₋₅ alkoxy-C₁₋₅ alkoxy-C₆₋₁₀ aryl;
R³ is halogen;
R⁴ is -OH, halogen or C₁₋₁₀ alkoxy;
R⁵ is -H, -OH or halogen; and
R⁶ is -H, halogen, C₁₋₄ alkyl or C₁₋₄ alkoxy.

In another aspect,
X, Y and Z are independently carbon or nitrogen atoms;
R¹ is -H, -OH, halogen, C₁₋₅ alkyl unsubstituted or substituted with one or more halogens, C₁₋₅ alkoxy unsubstituted or substituted with one or more halogens, nitro, C₁₋₅ alkylsulfanyl, cyano, amino, C₁₋₅ alkylamino, or 5 membered heteroaryl containing at least one N;
R² is substituted phenyl, or substituted 6 membered heteroaryl containing at least one N,
wherein the substituted phenyl or substituted 6 membered heteroaryl is independently phenyl or heteroaryl substituted with one or more substituents selected from the group consisting of halogen, unsubstituted or substituted C₁₋₃ alkyl and unsubstituted or substituted 4-8 membered fully or partially saturated heterocycloalkyl containing at least one N,
wherein the substituted C₁₋₃ alkyl is substituted with 6 membered heterocycloalkyl in which 2 C₁₋₃ alkyls are substituted with substituted amino,
the substituted 4-8 membered heterocycloalkyl is 4-8 membered heterocycloalkyl substituted with one or more substituents selected from the group consisting of -OH, C₁₋₃ alkyl unsubstituted or substituted with 5-6 membered heterocycloalkyl, amino unsubstituted or substituted with 1 or 2 C₁₋₃ alkyls, 5-6 membered heterocycloalkyl unsubstituted or substituted with C₁₋₃ alkyl, C₁₋₄ alkoxycarbonyl, 5 membered heteroaryl containing at least one N, phenylaminocarbonyl and C₁₋₃ alkoxy-C₁₋₃ alkoxy-phenyl;
R³ is halogen;
R⁴ is -OH, halogen or C₁₋₅ alkoxy;
R⁵ is -H, -OH or halogen; and
R⁶ is -H, halogen, C₁₋₄ alkyl or C₁₋₄ alkoxy.

In another aspect,
X, Y and Z are independently carbon or nitrogen atoms;
R¹ is -H, -F, -Cl, -OH, -CH₃, -CF₃, -OCH₃, -OCF₃, iso-propyl, tert-butyl, -SCH₃, -NO₂, -CN or imidazole,
R² is 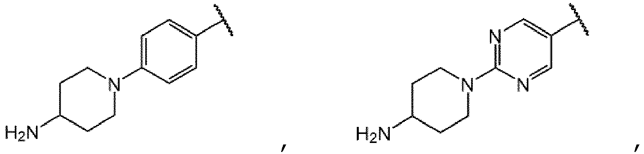 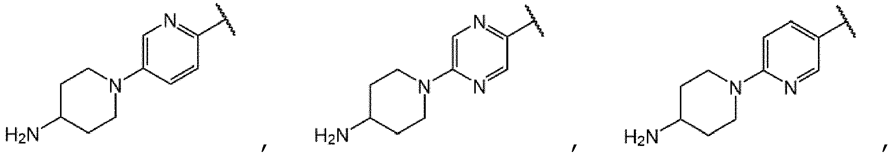 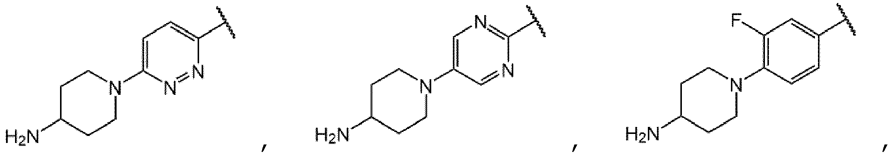 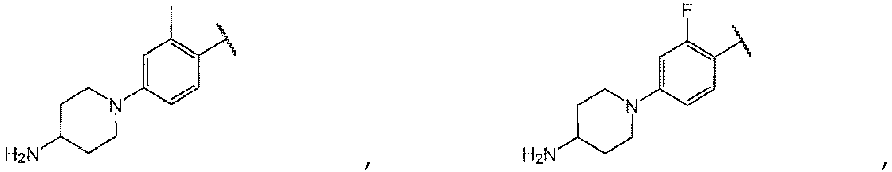 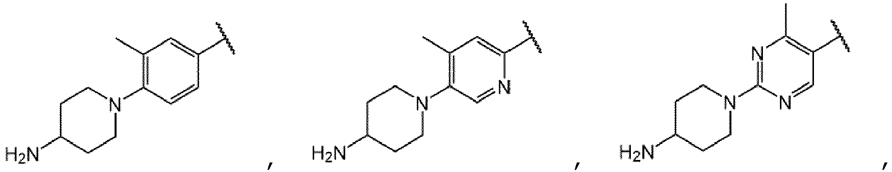 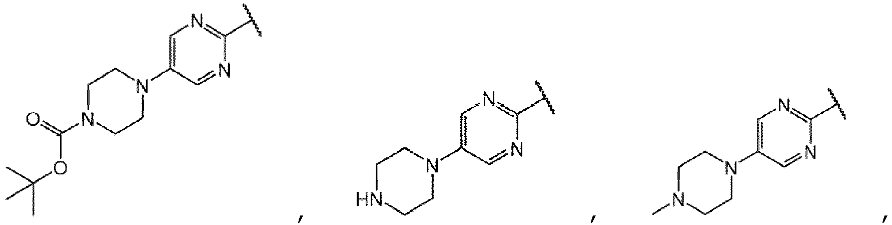 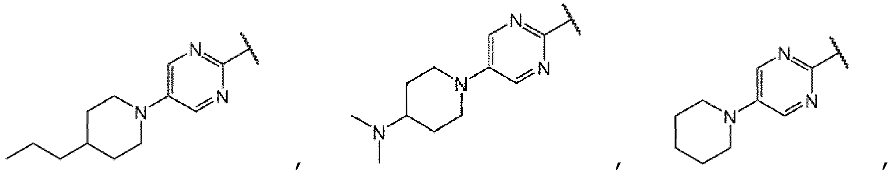 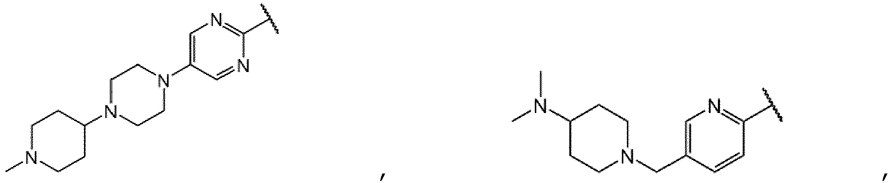 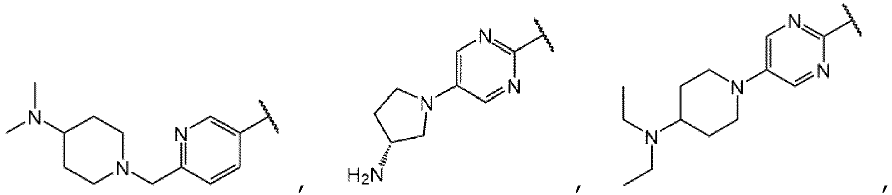 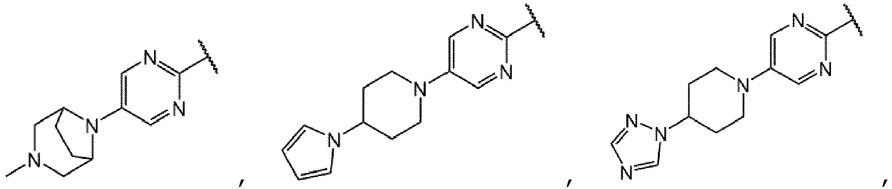 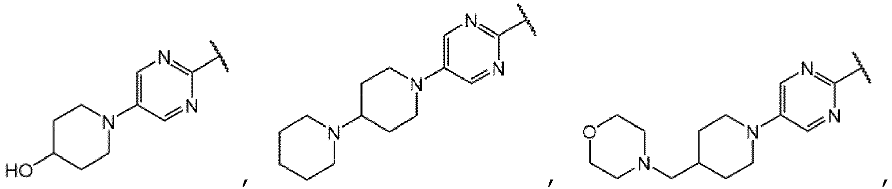 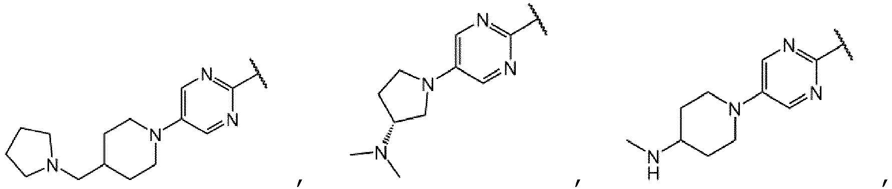 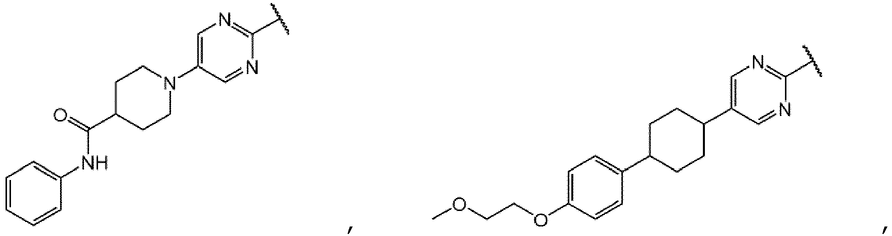 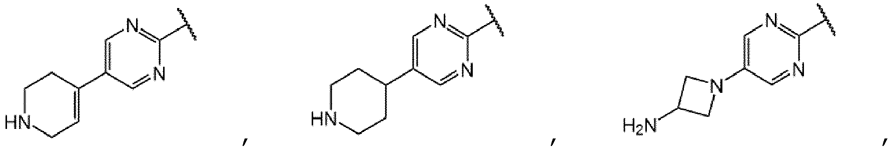 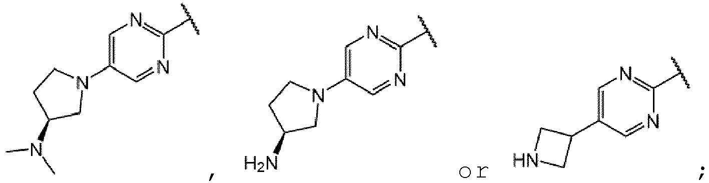
R³ is -F or -Cl;
R⁴ is -OH or -OCH₃;
R⁵ is -H, -OH, -F, -Cl, C₁₋₄ straight or branched alkyl or C₁₋₄ straight or branched alkoxy; and
R⁶ is -H, -OH, -F, -Cl, C₁₋₄ straight or branched alkyl or C₁₋₄ straight or branched alkoxy.

Preferably,
X is carbon or nitrogen atoms;
Y and Z are carbon atoms;
R¹ is -H, -F, -Cl, -OH, -CH₃, -CF₃, -OCH₃, -OCF₃, iso-propyl, tert-butyl, -SCH₃, -NO₂, -CN or imidazole,
R² is 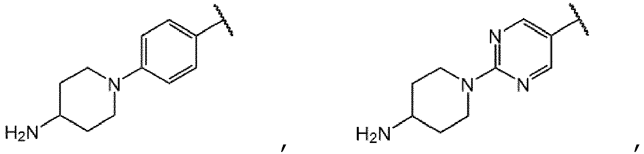 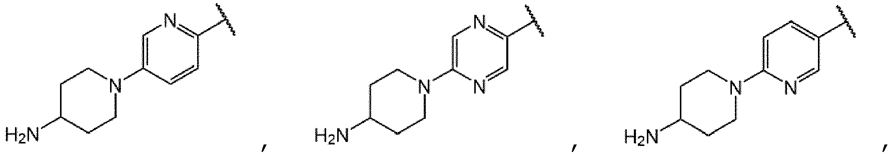 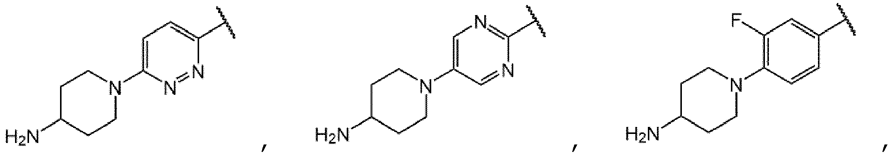 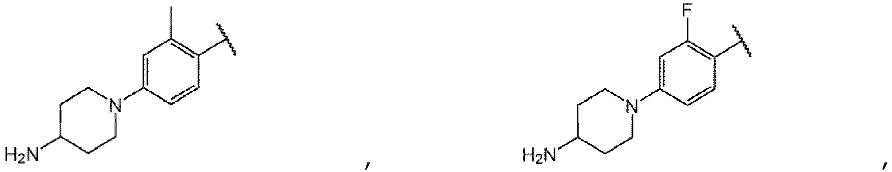 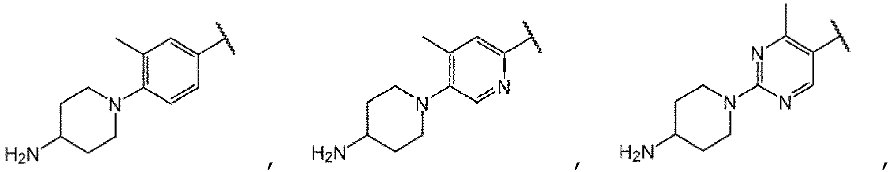 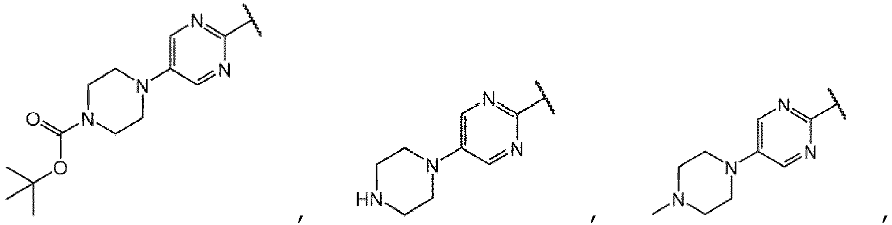 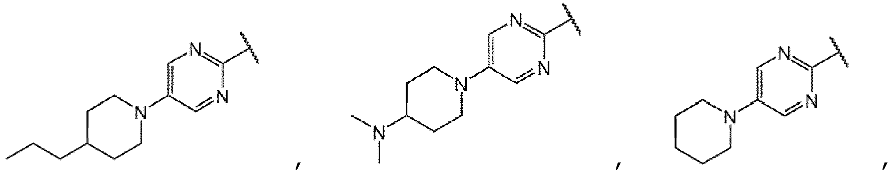 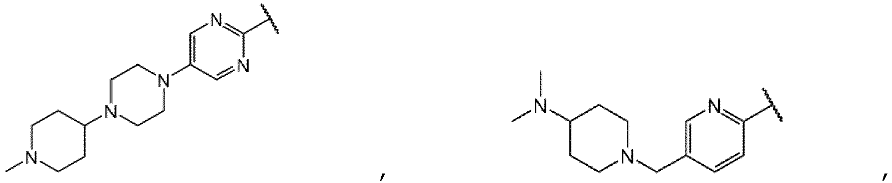 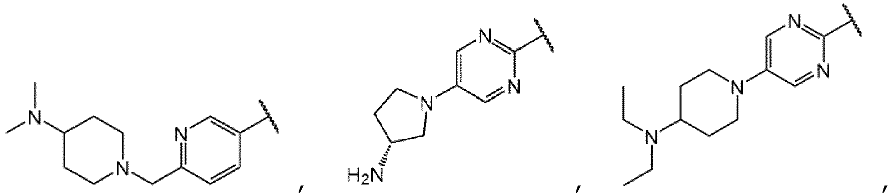 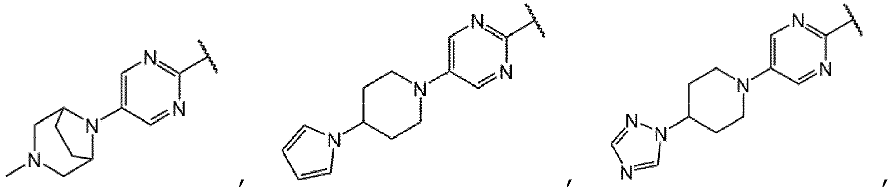 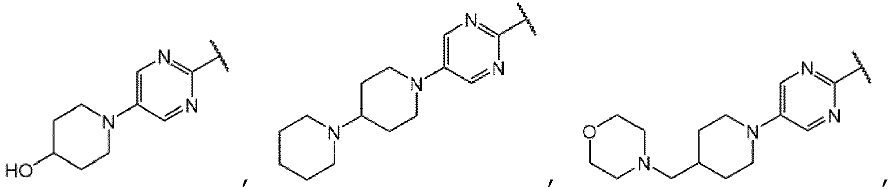 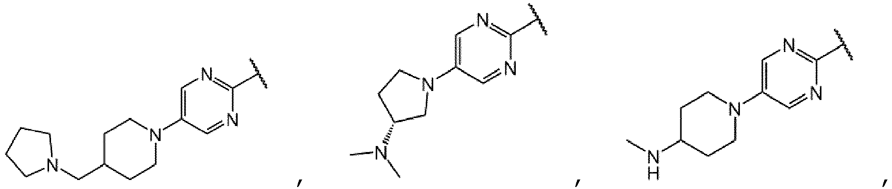 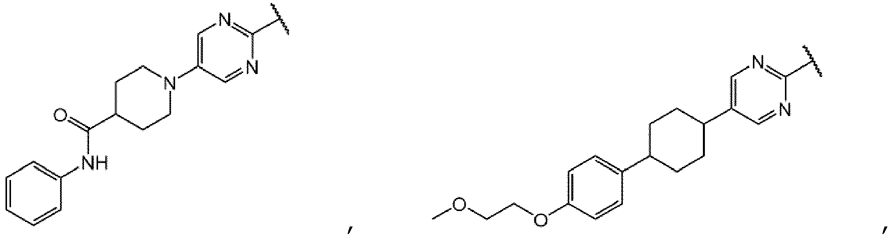 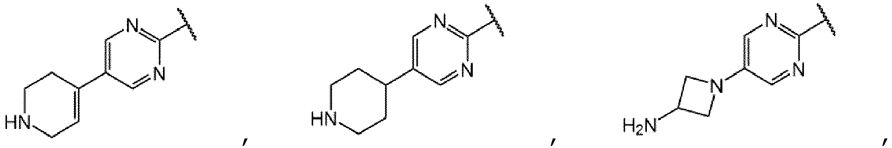 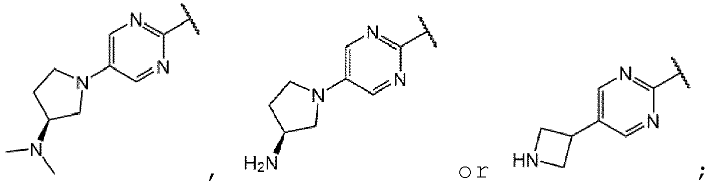
R³ is -F;
R⁴ is -OH or -OCH₃;
R⁵ is -H; and
R⁶ is -H, -F or -Cl.

Preferably, the compound represented by formula 1 can be a compound represented by formula 2 below.

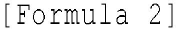

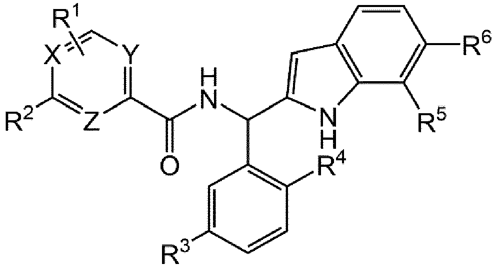

In formula 2, X, Y, Z, R¹, R², R³, R⁴, R⁵ and R⁶ are independently as defined in formula 1 above.

In another aspect,
X is carbon or nitrogen atoms;
Y and Z are carbon atoms;
R¹ is -H, -F, -Cl, -OH, -CH₃, -CF₃, -OCH₃, -OCF₃, iso-propyl, tert-butyl, -SCH₃, -NO₂, -CN or imidazole;
R² is 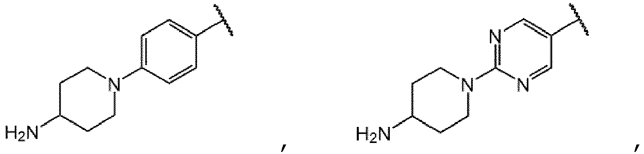 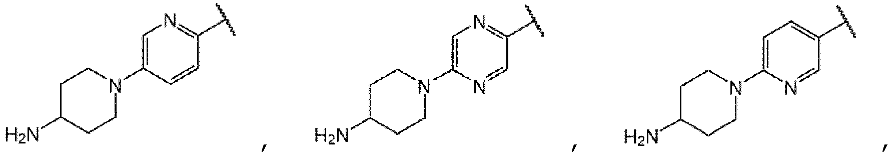 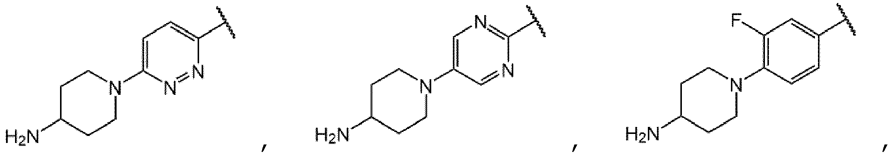 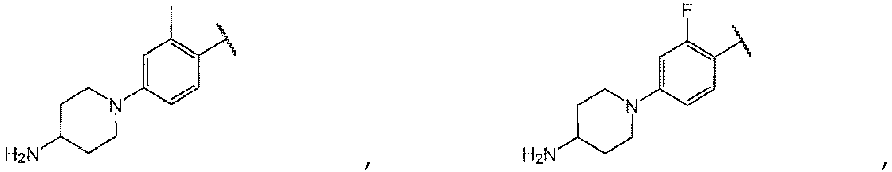 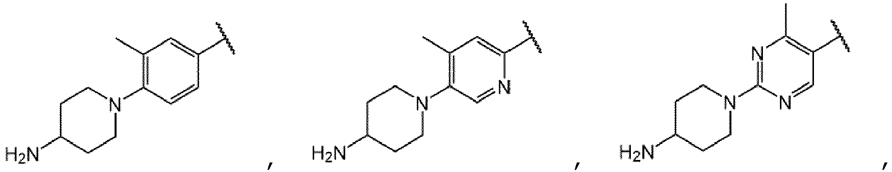 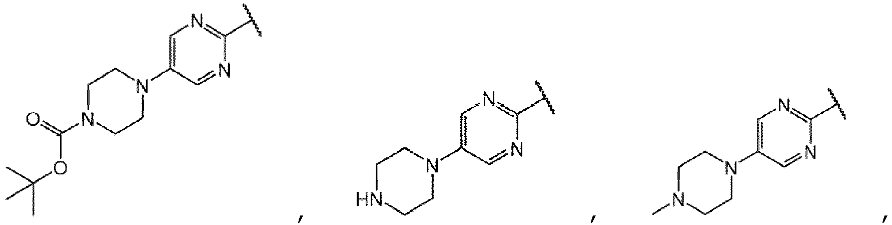 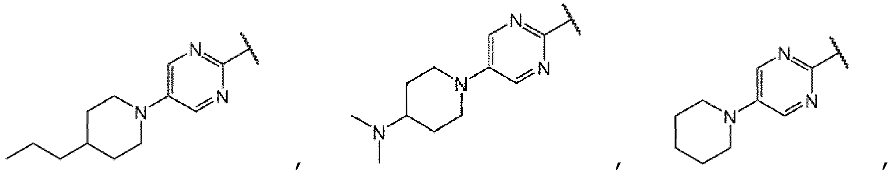 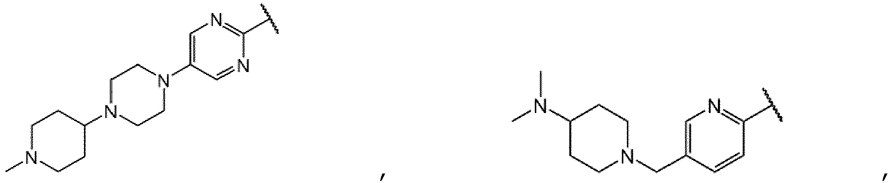 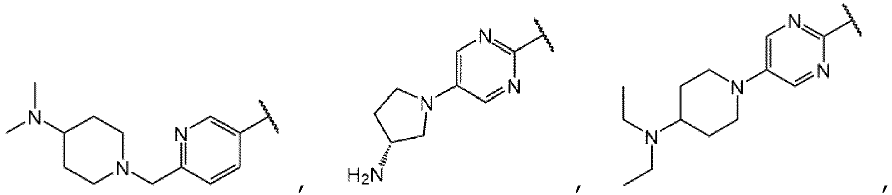 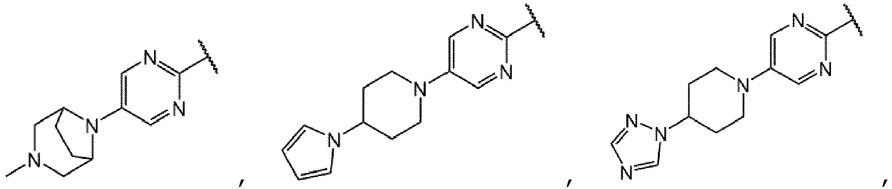 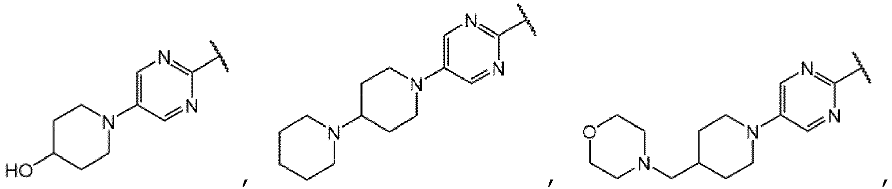 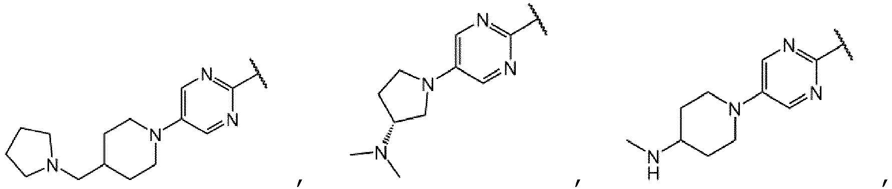 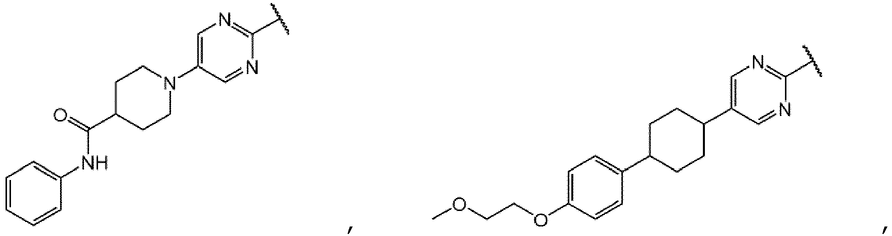 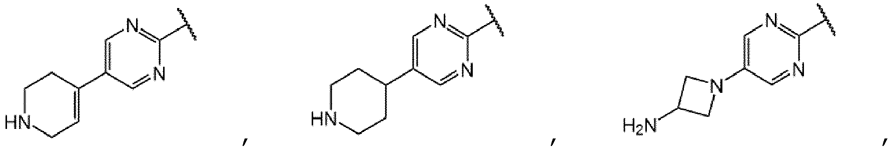 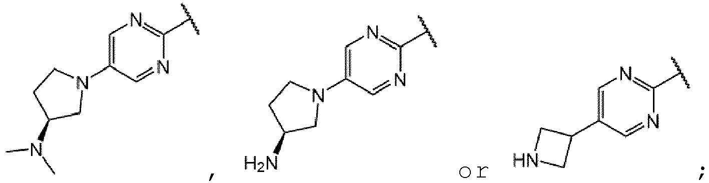
R³ is -F;
R⁴ is -OH or -OCH₃;
R⁵ is -H; and
R⁶ is -H, -F or -Cl.

Most preferably, the compound represented by formula 1 can be any one compound selected from the group consisting of the following compounds.
(1) (R)-4'-(4-aminopiperidine-1-yl)-N-((5-fluoro-2-hydroxyphenyl)(1H-indole-2-yl)methyl)-5-methyl-[1,1'-biphenyl]-3-carboxamide;
(2) (R)-4'-(4-aminopiperidine-1-yl)-N-((5-fluoro-2-hydroxyphenyl) (1H-indole-2-yl)methyl)-5-isopropyl-[1,1'-biphenyl]-3-carboxamide;
(3) (R)-4'-(4-aminopiperidine-1-yl)-N-((5-fluoro-2-hydroxyphenyl)(1H-indole-2-yl)methyl)-5-methoxy-[1,1'-biphenyl]-3-carboxamide;
(4) (R)-4'-(4-aminopiperidine-1-yl)-5-(tert-butyl)-N-((5-fluoro-2-hydroxyphenyl)(1H-indole-2-yl)methyl)-[1,1'-biphenyl]-3-carboxamide;
(5) (R)-4'-(4-aminopiperidine-1-yl)-5-fluoro-N-((5-fluoro-2-hydroxyphenyl)(1H-indole-2-yl)methyl)-[1,1'-biphenyl]-3-carboxamide;
(6) (R)-4'-(4-aminopiperidine-1-yl)-N-((5-fluoro-2-hydroxyphenyl)(1H-indole-2-yl)methyl)-5-(trifluoromethyl)-[1,1'-biphenyl]-3-carboxamide;
(7) (R)-4'-(4-aminopiperidine-1-yl)-N-((5-fluoro-2-hydroxyphenyl)(1H-indole-2-yl)methyl)-5-nitro-[1,1'-biphenyl]-3-carboxamide;
(8) (R)-4'-(4-aminopiperidine-1-yl)-N-((5-fluoro-2-hydroxyphenyl)(1H-indole-2-yl)methyl)-5-(methylthio)-[1,1'-biphenyl]-3-carboxamide;
(9) (R)-4'-(4-aminopiperidine-1-yl)-5-cyano-N-((5-fluoro-2-hydroxyphenyl)(1H-indole-2-yl)methyl)-[1,1'-biphenyl]-3-carboxamide;
(10) (R)-4'-(4-aminopiperidine-1-yl)-5-chloro-N-((5-fluoro-2-hydroxyphenyl)(1H-indole-2-yl)methyl)-[1,1'-biphenyl]-3-carboxamide;
(11) (R)-4'-(4-aminopiperidine-1-yl)-N-((5-fluoro-2-hydroxyphenyl)(1H-indole-2-yl)methyl)-5-(trifluoromethoxy)-[1,1'-biphenyl]-3-carboxamide;
(12) (R)-4'-(4-aminopiperidine-1-yl)-N-((5-fluoro-2-methoxyphenyl)(1H-indole-2-yl)methyl)-5-(trifluoromethoxy)-[1,1'-biphenyl]-3-carboxamide;
(13) (R)-4'-(4-aminopiperidine-1-yl)-N-((5-fluoro-2-hydroxyphenyl)(1H-indole-2-yl)methyl)-5-(1H-imidazole-1-yl)-[1,1'-biphenyl]-3-carboxamide;
(14) (R)-2-(4-(4-aminopiperidine-1-yl)phenyl)-N-((5-fluoro-2-hydroxyphenyl)(1H-indole-2-yl)methyl)isonicotinamide;
(15) (R)-2-(4-(4-aminopiperidine-1-yl)phenyl)-N-((5-fluoro-2-methoxyphenyl)(1H-indole-2-yl)methyl)isonicotinamide;
(16) (R)-3-(2-(4-aminopiperidine-1-yl)pyrimidine-5-yl)-N-((5-fluoro-2-hydroxyphenyl)(1H-indole-2-yl)methyl)-5-methylbenzamide;
(17) (R)-3-(5-(4-aminopiperidine-1-yl)pyridine-2-yl)-N-((5-fluoro-2-hydroxyphenyl)(1H-indole-2-yl)methyl)-5-methylbenzamide;
(18) (R)-3-(5-(4-aminopiperidine-1-yl)pyrazine-2-yl)-N-((5-fluoro-2-hydroxyphenyl)(1H-indole-2-yl)methyl)-5-methylbenzamide;
(19) (R)-3-(6-(4-aminopiperidine-1-yl)pyridine-3-yl)-N-((5-fluoro-2-hydroxyphenyl)(1H-indole-2-yl)methyl)-5-methylbenzamide;
(20) (R)-3-(6-(4-aminopiperidine-1-yl)pyridazine-3-yl)-N-((5-fluoro-2-hydroxyphenyl)(1H-indole-2-yl)methyl)-5-methylbenzamide;
(21) (R)-3-(5-(4-aminopiperidine-1-yl)pyrimidine-2-yl)-N-((5-fluoro-2-hydroxyphenyl)(1H-indole-2-yl)methyl)-5-methylbenzamide;
(22) (R)-4'-(4-aminopiperidine-1-yl)-3'-fluoro-N-((5-fluoro-2-hydroxyphenyl)(1H-indole-2-yl)methyl)-5-methyl-[1,1'-biphenyl]-3-carboxamide;
(23) (R)-4'-(4-aminopiperidine-1-yl)-N-((5-fluoro-2-hydroxyphenyl)(1H-indole-2-yl)methyl)-2',5-dimethyl-[1,1'-biphenyl]-3-carboxamide;
(24) (R)-4'-(4-aminopiperidine-1-yl)-2'-fluoro-N-((5-fluoro-2-hydroxyphenyl)(1H-indole-2-yl)methyl)-5-methyl-[1,1'-biphenyl]-3-carboxamide;
(25) (R)-4'-(4-aminopiperidine-1-yl)-N-((5-fluoro-2-hydroxyphenyl)(1H-indole-2-yl)methyl)-3',5-dimethyl-[1,1'-biphenyl]-3-carboxamide;
(26) (R)-4'-(4-aminopiperidine-1-yl)-N-((6-fluoro-1H-indole-2-yl)(5-fluoro-2-hydroxyphenyl)methyl)-5-methyl-[1,1'-biphenyl]-3-carboxamide;
(27) (R)-4'-(4-aminopiperidine-1-yl)-N-((6-chloro-1H-indole-2-yl)(5-fluoro-2-hydroxyphenyl)methyl)-5-methyl-[1,1'-biphenyl]-3-carboxamide;
(28) (R)-4'-(4-aminopiperidine-1-yl)-N-((5-fluoro-2-hydroxyphenyl)(1H-indole-2-yl)methyl)-6-methyl-[1,1'-biphenyl]-3-carboxamide;
(29) (R)-4'-(4-aminopiperidine-1-yl)-N-((5-fluoro-2-hydroxyphenyl)(1H-indole-2-yl)methyl)-4-methyl-[1,1'-biphenyl]-3-carboxamide;
(30) (R)-4'-(4-aminopiperidine-1-yl)-N-((5-fluoro-2-hydroxyphenyl)(1H-indole-2-yl)methyl)-2-methyl-[1,1'-biphenyl]-3-carboxamide;
(31) (S)-4'-(4-aminopiperidine-1-yl)-N-((5-fluoro-2-hydroxyphenyl)(1H-indole-2-yl)methyl)-5-methyl-[1,1'-biphenyl]-3-carboxamide;
(32) 4'-(4-aminopiperidine-1-yl)-N-((5-fluoro-2-hydroxyphenyl)(1H-indole-2-yl)methyl)-5-methyl-[1,1'-biphenyl]-3-carboxamide;
(33) (R)-3-(5-(4-aminopiperidine-1-yl)-4-methylpyridine-2-yl)-N-((5-fluoro-2-hydroxyphenyl)(1H-indole-2-yl)methyl)-5-methylbenzamide;
(34) (R)-3-(2-(4-aminopiperidine-1-yl)-4-methylpyrimidine-5-yl)-N-((5-fluoro-2-hydroxyphenyl)(1H-indole-2-yl)methyl)-5-methylbenzamide;
(35) tert-butyl (R)-4-(2-(3-(((5-fluoro-2-hydroxyphenyl)(1H-indole-2-yl)methyl)carbamoyl)-5-methylphenyl)pyrimidine-5-yl)piperazine-1-carboxylate;
(36) (R)-N-((5-fluoro-2-hydroxyphenyl)(1H-indole-2-yl)methyl)-3-methyl-5-(5-(piperazine-1-yl)pyrimidine-2-yl)benzamide;
(37) (R)-N-((5-fluoro-2-hydroxyphenyl)(1H-indole-2-yl)methyl)-3-methyl-5-(5-(4-methylpiperazine-1-yl)pyrimidine-2-yl)benzamide;
(38) N-[(R)-(5-fluoro-2-hydroxy-phenyl)-(1H-indole-2-yl)methyl]-3-methyl-5-[5-(4-propyl-1-piperidyl)pyrimidine-2-yl]benzamide;
(39) 3-[5-[4-(dimethylamino)-1-piperidyl]pyrimidine-2-yl]-N-[(R)-(5-fluoro-2-hydroxyphenyl)-(1H-indole-2-yl)methyl]-5-methyl-benzamide;
(40) N-[(R)-(5-fluoro-2-hydroxy-phenyl)-(1H-indole-2-yl)methyl]-3-methyl-5-[5-(1-piperidyl)pyrimidine-2-yl]benzamide;
(41) N-[(R)-(5-fluoro-2-hydroxy-phenyl)-(1H-indole-2-yl)methyl]-3-methyl-5-[5-[4-(1-methyl-4-piperidyl)piperazine-1-yl]pyrimidine-2-yl]benzamide;
(42) (R)-3-(5-((4-(dimethylamino)piperidine-1-yl)methyl)pyridine-2-yl)-N-((5-fluoro-2-hydroxyphenyl)(1H-indole-2-yl))methyl)-5-methylbenzamide;
(43) (R)-3-(6-((4-(dimethylamino)piperidine-1-yl)methyl)pyridine-3-yl)-N-((5-fluoro-2-hydroxyphenyl)(1H-indole-2-yl))methyl)-5-methylbenzamide;
(44) 3-[5-[(3R)-3-aminopyrrolidine-1-yl]pyrimidine-2-yl]-N-[(R)-(5-fluoro-2-hydroxyphenyl)-(1H-indole-2-yl)methyl]-5-methyl-benzamide;
(45) (R)-3-(5-(4-(diethylamino)piperidine-1-yl)pyrimidine-2-yl)-N-((5-fluoro-2-hydroxyphenyl)(1H-indole-2-yl)methyl)-5-methylbenzamide;
(46) N-((R)-(5-fluoro-2-hydroxyphenyl)(1H-indole-2-yl)methyl)-3-methyl-5-(5-(3-methyl-3,8-diazabicyclo[3.2.1]octane-8-yl)pyrimidine-2-yl)benzamide;
(47) (R)-3-(5-(4-(1H-pyrrole-1-yl)piperidine-1-yl)pyrimidine-2-yl)-N-((5-fluoro-2-hydroxyphenyl)(1H-indole-2-yl)methyl)-5-methylbenzamide;
(48) (R)-3-(5-(4-(1H-1,2,4-triazole-1-yl)piperidine-1-yl)pyrimidine-2-yl)-N-((5-fluoro-2-hydroxyphenyl)(1H-indole-2-yl)methyl)-5-methylbenzamide;
(49) (R)-N-((5-fluoro-2-hydroxyphenyl)(1H-indole-2-yl)methyl)-3-(5-(4-hydroxypiperidine-1-yl)pyrimidine-2-yl)-5-methylbenzamide;
(50) (R)-3-(5-([1,4'-bipiperidine]-1'-yl)pyrimidine-2-yl)-N-((5-fluoro-2-hydroxyphenyl)(1H-indole-2-yl)methyl)-5-methylbenzamide;
(51) (R)-N-((5-fluoro-2-hydroxyphenyl)(1H-indole-2-yl)methyl)-3-methyl-5-(5-(4-(morpholinomethyl)piperidine-1-yl)pyrimidine-2-yl)benzamide;
(52) (R)-N-((5-fluoro-2-hydroxyphenyl)(1H-indole-2-yl)methyl)-3-methyl-5-(5-(4-(pyrrolidine-1-ylmethyl)piperidine-1-yl)pyrimidine-2-yl)benzamide;
(53) 3-[5-[(3R)-3-(dimethylamino)pyrrolidine-1-yl]pyrimidine-2-yl]-N-[(R)-(5-fluoro-2-hydroxyphenyl)-(1H-indole-2-yl)methyl]-5-methyl-benzamide;
(54) (R)-N-((5-fluoro-2-hydroxyphenyl)(1H-indole-2-yl)methyl)-3-methyl-5-(5-(4-(methylamino)piperidine-1-yl)pyrimidine-2-yl)benzamide;
(55) (R)-1-(2-(3-(((5-fluoro-2-hydroxyphenyl)(1H-indole-2-yl)methyl)carbamoyl)-5-methylphenyl)pyrimidine-5-yl)-N-phenylpiperidine-4-carboxamide;
(56) (R)-N-((5-fluoro-2-hydroxyphenyl)(1H-indole-2-yl)methyl)-3-(5-(4-(4-(2-methoxyethoxy)phenyl)piperazine-1-yl)pyrimidine-2-yl)-5-methylbenzamide;
(57) (R)-N-((5-fluoro-2-hydroxyphenyl)(1H-indole-2-yl)methyl)-3-methyl-5-(5-(1,2,3,6-tetrahydropyridine-4-yl)pyrimidine-2-yl)benzamide;
(58) (R)-N-((5-fluoro-2-hydroxyphenyl)(1H-indole-2-yl)methyl)-3-methyl-5-(5-(piperidine-4-yl)pyrimidine-2-yl)benzamide;
(59) (R)-3-(5-(3-aminoazetidine-1-yl)pyrimidine-2-yl)-N-((5-fluoro-2-hydroxyphenyl)(1H-indole-2-yl)methyl)-5-methylbenzamide;
(60) 3-(5-((S)-3-(dimethylamino)pyrrolidine-1-yl)pyrimidine-2-yl)-N-((R)-(5-fluoro-2-hydroxyphenyl)(1H-indole-2-yl)methyl)-5-methylbenzamide;
(61) 3-(5-((S)-3-aminopyrrolidine-1-yl)pyrimidine-2-yl)-N-((R)-(5-fluoro-2-hydroxyphenyl)(1H-indole-2-yl) methyl)-5-methylbenzamide; and
(62) (R)-3-(5-(azetidine-3-yl)pyrimidine-2-yl)-N-((5-fluoro-2-hydroxyphenyl)(1H-indole-2-yl)methyl)-5-methylbenzamide.

The compound represented by formula 1 of the present invention can be used as a form of a pharmaceutically acceptable salt, in which the salt is preferably acid addition salt formed by pharmaceutically acceptable free acids. The acid addition salt herein can be obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono/dicarboxylate, phenyl-substituted alkanoate, hydroxy alkanoate, alkandioate, aromatic acids, and aliphatic/aromatic sulfonic acids; or organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate, and mandelate.

The acid addition salt according to the present invention can be prepared by the conventional method known to those in the art. For example, the derivative represented by formula 1 is dissolved in an organic solvent such as methanol, ethanol, acetone, methylenechloride, and acetonitrile, to which organic acid or inorganic acid is added to induce precipitation. Then, the precipitate is filtered and dried to give the salt. Or the solvent and the excessive acid are distillated under reduced pressure, and dried to give the salt. Or the precipitate is crystallized in an organic solvent to give the same.

A pharmaceutically acceptable metal salt can be prepared by using a base. Alkali metal or alkali earth metal salt is obtained by the following processes: dissolving the compound in excessive alkali metal hydroxide or alkali earth metal hydroxide solution; filtering non-soluble compound salt; evaporating the remaining solution and drying thereof. At this time, the metal salt is preferably prepared in the pharmaceutically suitable form of sodium, potassium, or calcium salt. And the corresponding silver salt is prepared by the reaction of alkali metal or alkali earth metal salt with proper silver salt (ex; silver nitrate).

In addition, the present invention includes not only the compound represented by formula 1 but also a pharmaceutically acceptable salt thereof, and a solvate, an optical isomer, or a hydrate possibly produced from the same.

The term "hydrate" refers to a compound or a salt thereof of the present invention containing a stoichiometric or non-stoichiometric amount of water bound by a non-covalent intermolecular force. The hydrate of the compound represented by formula 1 of the present invention can contain a stoichiometric or non-stoichiometric amount of water bound by a non-covalent intermolecular force. The hydrate can contain 1 equivalent or more of water, preferably 1 to 5 equivalents of water. The hydrate can be prepared by crystallizing the compound represented by formula 1, the isomer thereof, or the pharmaceutically acceptable salt thereof from water or the solvent containing water.

The term "solvate" refers to a compound or a salt thereof of the present invention containing a stoichiometric or non-stoichiometric amount of solvent bound by a non-covalent intermolecular force. Preferred solvents therefor include solvents that are volatile, non-toxic, and/or suitable for administration to humans.

The term "isomer" refers to a compound or a salt thereof of the present invention having the same chemical formula or molecular formula, but structurally or sterically different. Such isomers include structural isomers such as tautomers, R or S isomers having an asymmetric carbon center, stereoisomers such as geometric isomers (trans, cis), and optical isomers (enantiomers). All these isomers and mixtures thereof are also included in the scope of the present invention.

The compound represented by formula 1 can be prepared through the following general formulas, and the general formulas 1 and 2 corresponding to the preparation process of the compound of Example 1 are shown below.

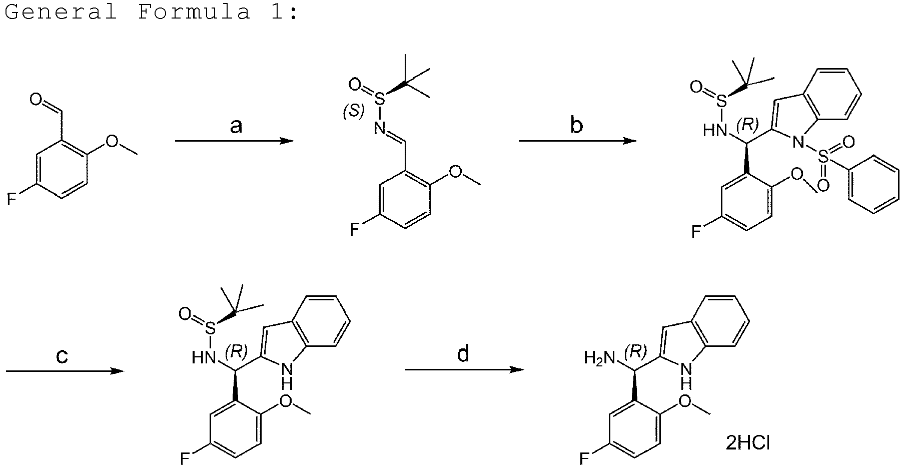

Scheme 1. Reagents and Conditions: (a) (S)-2-methylpropane-2-sulfinamide, titanium(IV) ethoxide, THF, rt, 17 h; (b) 1-(phenylsulfonyl)-1H-indole, n-BuLi, THF, -78°C to rt, 3 h; (c) 5 N NaOH, MeOH, reflux, overnight; (d) 4 N HCl dissolved in dioxane, MeOH, rt, 1h.

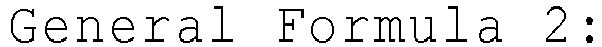

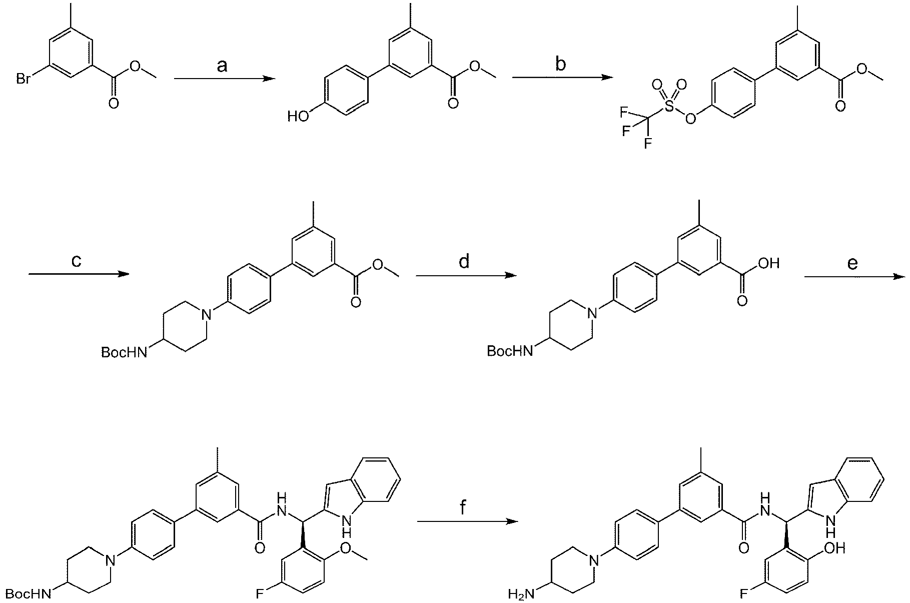

Scheme 2. Reagents and Conditions: (a) methyl 3-bromo-5-methylbenzoate, 4-hydroxyphenylboronic acid, [1,1'-bis(diphenylphosphino)ferrocene]palladium(II) dichloride, potassium carbonate, dioxane, water, - 78°C, 1.5 h (b) trifluoromethanesulfonic acid anhydride, pyridine, dichloromethane, rt, 3 h; (c) tert-butyl piperidine-4-yl carbamate, Ruphos Pd G1, Cs₂CO₃, dioxane, 110°C, overnight; (d) NaOH, dioxane, 110°C, overnight; (e) 1-ethyl-3-(3-dimethylaminopropyl)carbodiimide, hydroxybenzotriazole, triethylamine, CH₂Cl₂, rt, overnight; (f) 1 M BBr₃ dissolved in CH₂Cl₂, 0°C to rt, 30 min.

In another aspect of the present invention, the present invention provides a pharmaceutical composition comprising a compound represented by formula 1, an optical isomer thereof, a solvate thereof, a hydrate thereof or a pharmaceutically acceptable salt thereof as an active ingredient for the prevention or treatment of cancer.

Wherein, the compound can inhibit EGFR (epidermal growth factor receptor) mutation to prevent or treat cancer. At this time, the EGFR mutation is at least one selected from the group consisting of EGFR L858R/T790M and EGFR L858R/T790M/C797S.

The compound represented by formula 1 of the present invention or the pharmaceutically acceptable salt thereof can be administered orally or parenterally and be used in general forms of pharmaceutical formulation. That is, the compound or the pharmaceutically acceptable salt thereof can be prepared for oral or parenteral administration by mixing with generally used diluents or excipients such as fillers, extenders, binders, wetting agents, disintegrating agents and surfactants. Solid formulations for oral administration are tablets, pills, powders, granules and capsules. These solid formulations are prepared by mixing one or more compounds with one or more suitable excipients such as starch, calcium carbonate, sucrose or lactose, gelatin, etc. Except for the simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions and emulsions. Water insoluble excipients and suspensions can contain, in addition to the active compound or compounds, propylene glycol, polyethylene glycol, vegetable oil like olive oil, injectable ester like ethylolate, etc.

The pharmaceutical composition comprising the compound represented by formula 1 or the pharmaceutically acceptable salt thereof as an active ingredient can be administered by parenterally and the parenteral administration includes subcutaneous injection, intravenous injection, intramuscular injection, or intrathoracic injection.

At this time, to prepare the compound represented by formula 1 or the pharmaceutically acceptable salt thereof as a formulation for parenteral administration, the compound represented by formula 1 or the pharmaceutically acceptable salt thereof is mixed with a stabilizer or a buffering agent in water to produce a solution or suspension, which is then formulated as ampoules or vials. The composition herein can be sterilized and additionally contains preservatives, stabilizers, wettable powders or emulsifiers, salts and/or buffers for the regulation of osmotic pressure, and other therapeutically useful materials, and the composition can be formulated by the conventional mixing, granulating or coating method.

The formulations for oral administration are exemplified by tablets, pills, hard/soft capsules, solutions, suspensions, emulsions, syrups, granules, elixirs, and troches, etc. These formulations can include diluents (for example, lactose, dextrose, sucrose, mannitol, sorbitol, cellulose, and/or glycine) and lubricants (for example, silica, talc, stearate and its magnesium or calcium salt, and/or polyethylene glycol) in addition to the active ingredient. Tablets can include binding agents such as magnesium aluminum silicate, starch paste, gelatin, methylcellulose, sodium carboxymethylcellulose and/or polyvinylpyrolidone, and if necessary disintegrating agents such as starch, agarose, alginic acid or its sodium salt or azeotropic mixtures and/or absorbents, coloring agents, flavours, and sweeteners can be additionally included thereto.

The pharmaceutical composition of the present invention is administered in a pharmaceutically effective dose.

The term "pharmaceutically effective dose" means an amount sufficient to treat a disease with a reasonable benefit/risk ratio applicable to medical treatment or improvement. The effective dose level depends on the factors including subject type and severity, age, gender, drug activity, sensitivity to drug, time of administration, administration route and excretion rate, duration of treatment, concomitant drugs, and other factors well known in the medical field. For example, 0.001 mg/kg to 100 mg/kg, 0.01 mg/kg to 10 mg/kg, or 0.1 mg/kg to 1 mg/kg is included in the effective dose. The upper limit of the dose of the pharmaceutical composition of the present invention can be selected by those skilled in the art within an appropriate range.

In another aspect of the present invention, the present invention provides a health functional food composition comprising a compound represented by formula 1, an optical isomer thereof, a solvate thereof, a hydrate thereof, or a pharmaceutically acceptable salt thereof as an active ingredient for the prevention or amelioration of cancer.

The compound represented by formula 1 of the present invention can be added as it is or as mixed with other food components according to the conventional method. The mixing ratio of active ingredients can be regulated according to the purpose of use (prevention or amelioration). In general, the compound of the present invention is preferably added to food or beverages by 0.1 ~ 90 weight part for the total weight of the food. However, if long term administration is required for health and hygiene or regulating health condition, the content can be lower than the above but higher content can be accepted as well since the compound of the present invention has been proved to be very safe.

In addition, the health beverage composition of the present invention can additionally include various flavors or natural carbohydrates, etc, like other beverages. The natural carbohydrates above can be one of monosaccharides such as glucose and fructose; disaccharides such as maltose and sucrose; polysaccharides such as dextrin and cyclodextrin, and sugar alcohols such as xilytole, sorbitol and erythritol. Besides, natural sweetening agents (thaumatin, stevia extract, for example rebaudioside A, glycyrrhizin, etc.) and synthetic sweetening agents (saccharin, aspartame, etc.) can be included as a sweetening agent. The content of the natural carbohydrate is preferably 1 ~ 20 g and more preferably 5 ~ 12 g in 100 g of the composition of the present invention.

In addition to the ingredients mentioned above, the compound represented by formula 1 of the present invention can include in variety of nutrients, vitamins, minerals (electrolytes), flavors including natural flavors and synthetic flavors, coloring agents and extenders (cheese, chocolate, etc.), pectic acid and its salts, alginic acid and its salts, organic acid, protective colloidal viscosifiers, pH regulators, stabilizers, antiseptics, glycerin, alcohols, carbonators which used to be added to soda, etc. In addition, the compound represented by formula 1 of the present invention can contain natural fruit juice and fruit flesh for the production of fruit juice beverages and vegetable beverages.

In another aspect of the present invention, the present invention provides a combination preparation comprising a compound represented by formula 1, an optical isomer thereof, a solvate thereof, a hydrate thereof or a pharmaceutically acceptable salt thereof as an active ingredient for the prevention or treatment of cancer.

At this time, the combination preparation is a preparation in which at least one selected from the group consisting of the components described below is administered in combination with the compound represented by formula 1.

In addition, the anticancer treatment described herein can be applied as a monotherapy, or can include conventional surgery, radiotherapy, chemotherapy or immunotherapy in addition to the compound of the present invention. Such chemotherapy can be performed concurrently, sequentially, or separately with the treatment using the compound of the present invention, wherein one or more of the following categories of anticancer agents can be used for the chemotherapy.
(i) Anti-proliferative/anti-neoplastic drugs and combinations thereof, as used in medical oncology, for example, alkylating agents (for example, cis-platin, oxaliplatin, carboplatin, cyclophosphamide, nitrogen mustard, melphalan, chlorambucil, busulfan, temozolamide and nitrosourea); antimetabolites (for example, gemcitabine and antifolates such as fluoropyrimidine such as 5-fluorouracil and tegafur, raltitrexed, methotrexate, cytosine arabinoside and hydroxyurea); anti-tumor antibiotics (for example, anthracyclines such as adriamycin, bleomycin, doxorubicin, daunomycin, epirubicin, idarubicin, mitomycin C, dactinomycin and mithramycin); mitotic inhibitors (for example, vinca alkaloids such as vincristine, vinblastine, vindesine and vinorelbine, taxoids such as taxol and taxotere, and polokinase inhibitors); and topoisomerase inhibitors (for example, epipodophyllotoxins such as etoposide and teniposide, amsacrine, topotecan and camptothecin);
(ii) Cell proliferation inhibitors, for example, antiestrogens (for example, tamoxifen, fulvestrant, toremifene, raloxifene, droloxifene and iodoxifene), antiandrogens (for example, bicalutamide, flutamide, nilutamide and cyproterone acetate), LHRH antagonists or LHRH agonists (for example, goserelin, leuprorelin and buserellin), progesterones (for example, megestrol acetate), aromatase inhibitors (for example, anastrozole, letrozole, vorazole and eximestane) and 5α-reductase inhibitors such as finasterid;
(iii) Invasion inhibitors [for example, c-Src kinase inhibitors such as 4-(6-chloro-2,3-methylenedioxyanilino)-7-[2-(4-methylpiperazine-1-yl)ethoxy]-5-tetrahydropyran-4-yloxyquinazoline [AZD0530 (saracatinib); WO 01/94341], N-(2-chloro-6-methylphenyl)-2-{6-[4-(2-hydroxyethyl)piperazine-1-yl]-2-methylpyrimidine-4-ylamino}thiazole-5-carboxamide (dasatinib, BMS-354825; J. Med. Chem., 2004, 47, 6658-6661) and bosutinib (SKI-606), and metalloproteinase inhibitors such as marimastat, urokinase plasminogen activator receptor inhibitors or antibodies to heparana];
(iv) Growth factor inhibitors (for example, growth factor antibodies and growth factor receptor antibodies such as anti-erbB2 antibody trastuzumab [Herceptin^{™}], anti-EGFR antibody panitumumab, anti-erbB1 antibody cetuximab [Erbitux, C225] and any growth factor or growth factor receptor antibody disclosed in the literature [Stern et al. Critical reviews in oncology/haematology, 2005, Vol. 54, pp11-29]; tyrosine kinase inhibitors such as epidermal growth factor inhibitors (for example, EGFR tyrosine kinase inhibitors such as N-(3-chloro-4-fluorophenyl)-7-methoxy-6-(3-morpholinopropoxy)-quinazoline-4-amine (gefitinib, ZD1839), N-(3-ethynylphenyl)-6,7-bis(2-methoxyethoxy)quinazoline-4-amine (erlotinib, OSI-774) and 6-acrylamido-N-(3-chloro-4-fluorophenyl)-7-(3-morpholinopropoxy)-quinazoline-4-amine (CI 1033), N-(2-[2-dimethylaminoethyl-methylamino]-5-{[4-(1H-indole-3-yl)pyrimidine-2-yl]amino}-4-methoxyphenyl)prop-2-eneamide (osimertinib, AZD9291), erbB2 tyrosine kinase inhibitors such as lapatinib); hepatocyte growth factor inhibitors; insulin growth factor inhibitors; platelet-derived growth factor inhibitors such as imatinib and/or nilotinib (AMN107); serine/threonine kinase inhibitors (for example, Ras/Raf signaling inhibitors such as farnesyl transferase inhibitors such as sorafenib (BAY 43-9006), tipifarnib (R115777) and lonafarnib (SCH66336)), inhibitors of cell signaling via MEK and/or AKT kinase, c-kit inhibitors, abl kinase inhibitors, PI3 kinase inhibitors, Plt3 kinase inhibitors, CSF-1R kinase inhibitors, IGF receptor (insulin-like growth factor) kinase inhibitors; aurora Kinase Inhibitors (for example, AZD1152, PH739358, VX-680, MLN8054, R763, MP235, MP529, VX-528 and AX39459) and cyclin dependent kinase inhibitors such as CDK2 and/or CDK4 inhibitors);
(v) Angiogenesis inhibitors such as those that inhibit the effect of vascular endothelial growth factor [for example, anti-vascular endothelial cell growth factor antibody bevacizumab (Avastin^{™}), and VEGF receptor tyrosine kinase inhibitors such as vandetanib (ZD6474), vatalanib (PTK787), sunitinib (SU11248), axitinib (AG-013736), pazopanib (GW 786034) and 4-(4-fluoro-2-methylindole-5-yloxy)-6-methoxy-7-(3-pyrrolidine-1-ylpropoxy)quinazoline (AZD2171; Example 240 of WO 00/47212) such as the compounds disclosed in WO 97/22596, WO 97/30035, WO 97/32856 and WO 98/13354, and the compounds acting by other mechanisms (for example, linomid, integrin αvβ3 inhibitor and angiostatin)];
(vi) Vascular damaging agents such as Combretastatin A4 and the compounds disclosed in WO 99/02166, WO 00/40529, WO 00/41669, WO 01/92224, WO 02/04434 and WO 02/08213;
(vii) Endoterin receptor antagonists such as zivotentan (ZD4054) or atrasentan;
(viii) Antisense therapies, for example those against the targets listed above, such as ISIS 2503 and anti-ras antisense;
(ix) Gene therapy approaches (for example, approaches to replace aberrant genes such as aberrant p53 or aberrant BRCA1 or BRCA2, GDEPT (gene directed enzyme pro drug therapy) approaches such as approaches using cytosine deaminase, thymidine kinase or bacterial nitroreductase, and approaches to increase a patient's resistance to chemotherapy or radiotherapy, such as multiple drug resistance gene therapy); and
(x) Immunotherapy approaches (for example, in vitro and in vivo approaches to increase the immunogenicity of patient tumor cells, such as those using transfection with cytokines such as interleukin 2, interleukin 4 or granulocyte macrophage colony stimulating factor, approaches to reduce T cell anergy, approaches using transfected immune cells, such as dendritic cells transfected with cytokines, approaches using tumor cell lines transfected with cytokines, approaches using anti-idiotypic antibodies, approaches to reduce the action of immunosuppressive cells such as regulatory T cells, myeloid derived suppressor cells or IDO (indole amine 2,3-deoxygenase) expressing dendritic cells, and approaches using cancer vaccines consisting of proteins or peptides derived from tumor-associated antigens such as NY-ESO-1, MAGE-3, WT1 or Her2/neu).

Therefore, in a further aspect of the present invention, there is provided a pharmaceutical product for the combination treatment of cancer comprising a compound of formula (I) as defined above, and a further anti-tumor agent as defined above.

In this aspect of the present invention, a pharmaceutical product for the combination treatment of cancer comprising a compound of formula (I) as defined above or a pharmaceutically acceptable salt thereof, and a further anti-tumor agent as defined above is provided.

In this specification, when the term "combination treatment" is used in reference to combination therapy, it should be understood that it may mean simultaneous, separate or sequential administration. The meaning of "combination administration" should be interpreted similarly to "combination treatment". In one aspect of the present invention, "combination treatment" refers to simultaneous administration. In another aspect of the present invention, "combination treatment" refers to separate administration. In a further aspect of the present invention, "combination treatment" refers to sequential administration. If the administration is sequential or separate, the delay in administration of the second component should be such that the benefit of the effect resulting from the use of the combination is not lost. Thus, in one embodiment, sequential treatment comprises administering each component of the combination within a period of 11 days. In another embodiment, the said period of time is 10 days. In another embodiment, the said period of time is 9 days. In another embodiment, the said period of time is 8 days. In another embodiment, the said period of time is 7 days. In another embodiment, the said period of time is within 6 days. In another embodiment, the said period of time is within 5 days. In another embodiment, the said period of time is within 4 days. In another embodiment, the said period of time is within 3 days. In another embodiment, the said period of time is within 2 days. In another embodiment, the said period of time is within 24 hours. In another embodiment, the said period of time is within 12 days.

In another aspect of the present invention, the present invention provides a method for treating cancer, which comprises a step of administering a pharmaceutical composition or a health functional food composition comprising a compound represented by formula 1, an optical isomer thereof, a solvate thereof, a hydrate thereof, or a pharmaceutically acceptable salt thereof as an active ingredient to a subject in need.

In another aspect of the present invention, the present invention provides a compound represented by formula 1, an optical isomer thereof, a solvate thereof, a hydrate thereof, or a pharmaceutically acceptable salt thereof for use in the prevention or treatment of cancer.

In another aspect of the present invention, the present invention provides a use of a compound represented by formula 1, an optical isomer thereof, a solvate thereof, a hydrate thereof, or a pharmaceutically acceptable salt thereof for the manufacture of a medicament for the prevention or treatment of cancer.

The benzamide derivative provided in an aspect of the present invention can be used for preventing or treating cancer by suppressing EGFR mutation, and exhibits a remarkable synergy effect on anticancer activity when administered in combination with an EGFR antagonist such as Cetuximab, so that it can be effectively used as an anticancer agent. The above is supported by the Examples and Experimental Examples described later.

In the method or use, the detailed description of the pharmaceutical composition described above may be applied.

Hereinafter, the present invention will be described in detail by the following examples and experimental examples. However, the following examples and experimental examples are only for illustrating the present invention, and the contents of the present invention are not limited thereto.

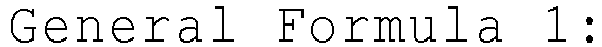

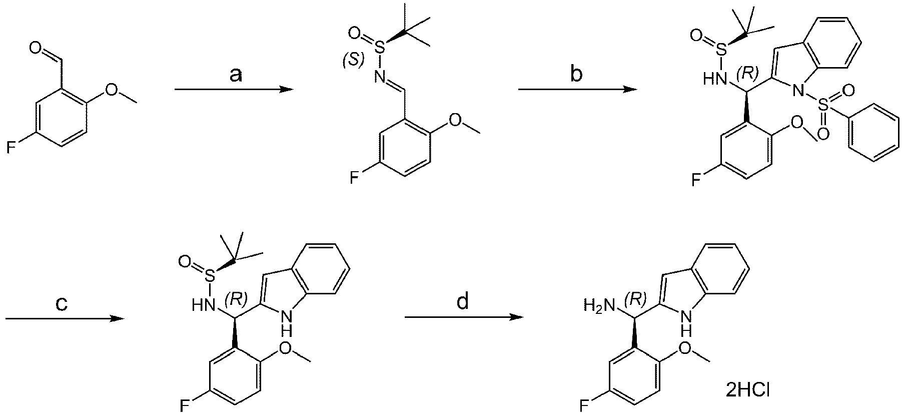

Scheme 1. Reagents and Conditions: (a) (S)-2-methylpropane-2-sulfinamide, titanium(IV) ethoxide, THF, rt, 17 h; (b) 1-(phenylsulfonyl)-1H-indole, n-BuLi, THF, -78 °C to rt, 3 h; (c) 5 N NaOH, MeOH, reflux, overnight; (d) 4 N HCl dissolved in dioxane, MeOH, rt, 1h.

### [Preparative Example 1]

### (S,E)-N-(5-fluoro-2-methoxybenzylidene)-2-methylpropane-2-sulfinamide

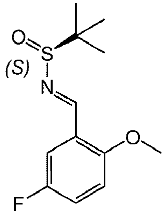

A mixture of 5-fluoro-2-methoxybenzaldehyde (26.7 g, 173 mmol) and (S)-2-methylpropane-2-sulfinamide (20.0 g, 165 mmol) was dissolved in tetrahydrofuran (4 mL) with titanium(IV) ethoxide (75.3 g, 330 mmol). The reaction flask was sealed with a septum, and the reaction mixture was stirred at room temperature for 24 hours. The reaction mixture was extracted with ethyl acetate and water. The ethyl acetate layer was collected, dried over anhydrous magnesium sulfate and filtered, and the solvent was removed by evaporation under reduced pressure. The residue was purified by flash column chromatography (Hex/EtOAc, 10:1) filled with silica gel to give (S,E)-N-(5-fluoro-2-methoxybenzylidene)-2-methylpropane-2-sulfinamide (40.6 g).

95% yield: ¹H NMR (500 MHz, chloroform-*d*) δ 9.03 (d, J = 2.4 Hz, 1H), 7.69 (dd, J = 8.8, 3.2 Hz, 1H), 7.19 (ddd, J = 9.1, 7.7, 3.2 Hz, 1H), 6.94 (dd, J = 9.1, 4.1 Hz, 1H), 3.90 (s, 3H), 1.29 (s, 9H); LC-MS: 274.3 [M+H⁺].

### [Preparative Example 2]

### (S)-N-((R)-(5-fluoro-2-methoxyphenyl)(1-(phenylsulfonyl)-1H-indole-2-yl)methyl)-2-methylpropane-2-sulfinamide

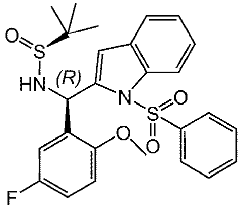

At -78 °C, n-butyl lithium was added dropwise to a solution of 1-(phenylsulfonyl)-1H-indole (56.0 g, 218 mmol) dissolved in tetrahydrofuran (200 mL). After the reaction was carried out at -78°C for 1 hour, a solution of (S,E)-N-(5-fluoro-2-methoxybenzylidene)-2-methylpropane-2-sulfinamide [Preparative Example 1] (40.0 g, 155 mmol) dissolved in THF (200 mL) was added thereto, and the mixture was stirred at -78°C for 2 hours. The reaction was terminated by adding a saturated aqueous NH₄Cl solution to the mixture, followed by extraction with ethyl acetate. The ethyl acetate layer was collected, dried over anhydrous magnesium sulfate and filtered. The solvent was removed by evaporation under reduced pressure. The residue was purified by flash column chromatography (CH₂Cl₂/EtOAc, 10:1) filled with silica gel to give (S)-N-((R)-(5-fluoro-2-methoxyphenyl)(1-(phenylsulfonyl)-1H-indole-2-yl)methyl)-2-methylpropane-2-sulfinamide [Preparative Example 2] as a white solid (79.0 g, 98%).

¹H NMR (300 MHz, chloroform-*d*) δ 8.15 (dd, J = 8.3, 1.1 Hz, 1H), 7.76 - 7.69 (m, 2H), 7.53 - 7.47 (m, 2H), 7.42 - 7.30 (m, 3H), 6.98 (ddd, J = 8.9, 7.7, 3.0 Hz, 1H), 6.90 (dd, J = 9. 0, 4.5 Hz, 1H), 6.80 (d, J = 5.8 Hz, 1H), 6.76 (d, J = 0.9 Hz, 1H), 6.72 (dd, J = 9.1, 3.0 Hz, 1H), 3.92 (s, 3H), 1.26 (s, 9H); LC-MS: 531.6 [M+H⁺].

### [Preparative Example 3]

### (S)-N-((R)-(5-fluoro-2-methoxyphenyl)(1H-indole-2-yl)methyl)-2-methylpropane-2-sulfinamide

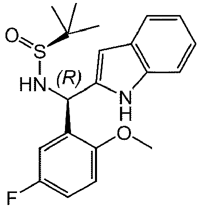

NaOH dissolved in water (8 N, 69.6 g, 768 mmol, 96.0 mL) was added to a solution of (S)-N-((R)-(5-fluoro-2-methoxyphenyl)(1-(phenylsulfonyl)-1H-indole-2-yl)methyl)-2-methylpropane-2-sulfinamide (79.0 g, 154 mmol) dissolved in methanol (700 mL), and the mixture was refluxed overnight. The mixture was stirred at 80°C for 24 hours and then cooled at room temperature. An aqueous solution was added thereto. The mixture was concentrated under reduced pressure. The residue was washed with water and MeOH and dried to give (S)-N-((R)-(5-fluoro-2-methoxyphenyl) (1H-indole-2-yl)methyl)-2-methylpropane-2-sulfinamide [Preparative Example 3] as a white solid (50.5 g, 87%) .

¹H NMR (400 MHz, DMSO-*d6*) δ 8.83 (d, J = 2.50 Hz, 1H), 7.62 (dd, J = 3.31, 8.94 Hz, 1H), 7.45 (dt, J = 3.38, 8.63 Hz, 1H), 7.24 (dd, J = 4.25, 9.26 Hz, 1H), 3.91 (s, 3H), 1.17 (s, 9H).

### [Preparative Example 4]

### (R)-(5-fluoro-2-methoxyphenyl) (1H-indole-2-yl)methaneamine

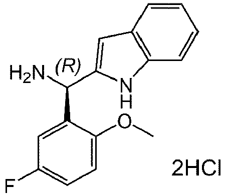

At room temperature, 4 N HCl dissolved in dioxane (0.16 mL, 0.32 mmol) was added to a solution of (S)-N-((R)-(5-fluoro-2-methoxyphenyl)(1H-indole-2-yl)methyl)-2-methylpropane-2-sulfinamide [Preparative Example 3] (51.0 g, 135 mmol) dissolved in methanol (500 mL). The mixture was concentrated under reduced pressure. The residue was solidified with diethyl ether and filtered to give (R)-(5-fluoro-2-methoxyphenyl)(1H-indole-2-yl)methaneamine [Preparative Example 4] (yellow solid) in a yield of 97% .

¹H NMR (300 MHz, methanol-d4) δ 7.59 (dt, J = 7.8, 1.1 Hz, 1H), 7.40 - 7.35 (m, 1H), 7.26 - 7.13 (m, 3H), 7.07 (ddd, J = 8.1, 7.1, 1.1 Hz, 1H), 6.99 (dd, J = 8.9, 2.7 Hz, 1H), 6.62 (t, J = 0.9 Hz, 1H), 6.01 (s, 1H), 3.97 (s, 3H), 3.68 (s, 3H);LC-MS: 391.4 [M+H⁺].

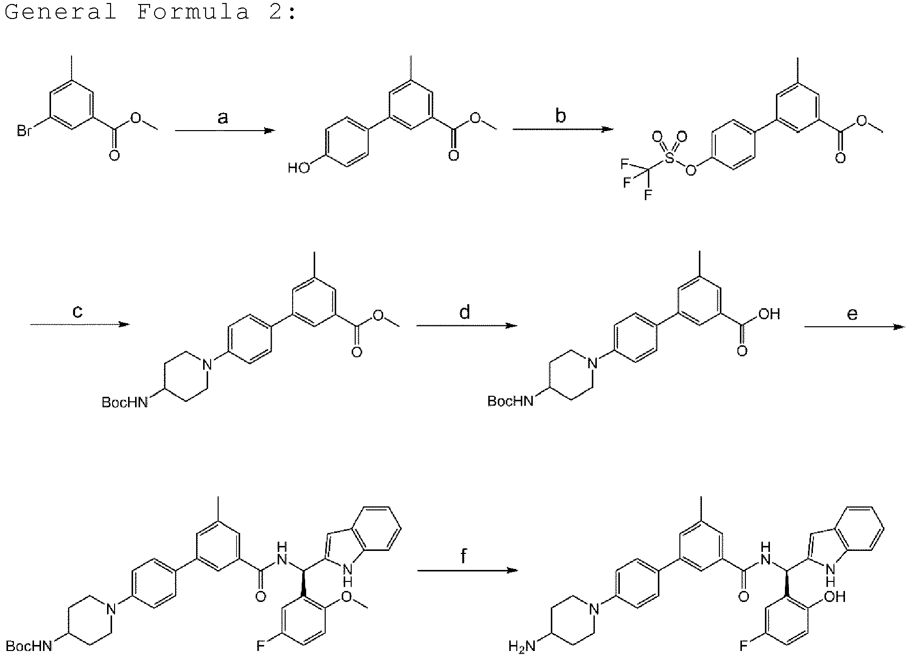

Scheme 2. Reagents and Conditions:: (a) methyl 3-bromo-5-methylbenzoate, 4-hydroxyphenylboronic acid, [1,1'-bis(diphenylphosphino)ferrocene]palladium(II) dichloride, potassium carbonate, dioxane, water, - 78 °C , 1.5 h (b) trifluoromethanesulfonic acid anhydride, pyridine, dichloromethane, rt, 3 h; (c) tert-butyl piperidine-4-yl carbamate, Ruphos Pd G1, Cs₂CO₃, dioxane, 110°C, overnight; (d) NaOH, dioxane, 110°C, overnight; (e) 1-ethyl-3-(3-dimethylaminopropyl)carbodiimide, hydroxybenzotriazole, triethylamine, CH₂Cl₂, rt, overnight; (f) 1 M BBr₃ dissolved in CH₂Cl₂, 0°C to rt, 30 min.

### [Preparative Example 5]

### methyl 4'-hydroxy-5-methyl-[1,1'-biphenyl]-3-carboxylate

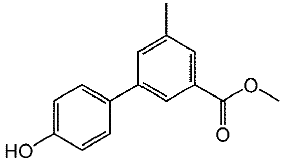

4-Hydroxyphenylboronic acid (108 mg, 0.79 mmol), methyl 3-bromo-5-methylbenzoate (150 mg, 0.65 mmol), [1,1'-bis(diphenylphosphino)ferrocene]palladium(II) dichloride (48 mg, 0.066mmol) and potassium carbonate (181 mg, 1.3 mmol) were degassed in dioxane/H₂O (2.5/0.5 mL) for 10 minutes. The stirred suspension was heated to 100 °C for 1.5 hr, diluted with ethyl acetate, and washed with water and brine. The organic layer was dried over magnesium sulfate and then concentrated. The residue was purified by MPLC to give 4'-hydroxy-5-methyl-[1,1'-biphenyl]-3-carboxylate [Preparative Example 5] as a yellow solid (120 mg, 75%).

¹H NMR (400 MHz, chloroform-d) δ 8.07 (s, 1H), 7.82 (s, 1H), 7.57 (s, 1H), 7.52 - 7.50 (m, 2H), 6.99 - 6.96 (m, 2H), 5.94 (br s, 1H), 3.98 (s, 3H), 2.47 (s, 3H); LC-MS: 243.2 [M+H⁺].

### [Preparative Example 6]

### methyl 5-methyl-4'-(((trifluoromethyl)sulfonyl)oxy)-[1,1'-biphenyl]-3-carboxylate

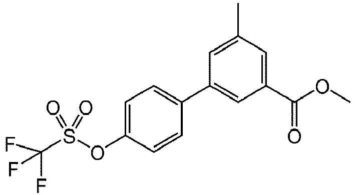

At room temperature, pyridine (0.052 ml, 0.64 mmol) was added to a solution of methyl 4'-hydroxy-5-methyl-[1,1'-biphenyl]-3-carboxylate (100 mg, 0.41 mmol) and trifluoromethanesulfonic acid anhydride (128 mg, 0.45 mmol) dissolved in anhydrous dichloromethane. The mixture was stirred for 3 hours. The residue was extracted with water and sodium bicarbonate, and washed with brine. The organic layer was dried over magnesium sulfate, filtered and concentrated. The crude compound was purified by MPLC to give methyl 5-methyl-4'-(((trifluoromethyl)sulfonyl)oxy)-[1,1'-biphenyl]-3-carboxylate [Preparative Example 6] as a yellow solid (102 mg, 66%).

¹H NMR (400 MHz, chloroform-*d*) δ 8.06 (s, 1H), 7.90 (s, 1H), 7.69 - 7.67 (m, 2H), 7.57 (s, 1H), 7.38 - 7.36 (m, 2H), 3.96 (s, 3H), 2.49 (s, 3H); LC-MS: 375.3 [M+H⁺].

### [Preparative Example 7]

### methyl 4'-(4-((tert-butoxycarbonyl)amino)piperidine-1-yl)-5-methyl-[1,1'-biphenyl]-3-carboxylate

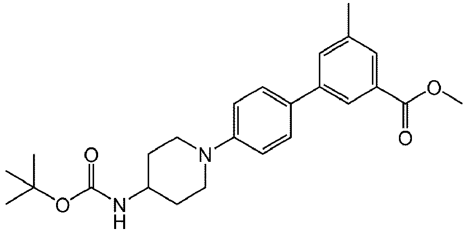

4-(Tert-butoxycarbonylamino)piperidine (61.1 mg, 0.31mmol), methyl 5-methyl-4'-(((trifluoromethyl)sulfonyl)oxy)-[1,1'-biphenyl]-3-carboxylate [Preparative Example 6] (104 mg, 0.28 mmol), Ruphos Pd G1 (22.7 mg, 0.028 mmol) and cesium carbonate (81.4 mg, 0.025 mmol) were degassed in anhydrous 1,4-dioxane (2.5 mL) for 10 minutes. The stirred suspension was heated at 110 °C overnight, diluted with ethyl acetate, and washed with water and brine. The organic layer was dried over magnesium sulfate and concentrated to give methyl 4'-(4-((tert-butoxycarbonyl)amino)piperidine-1-yl)-5-methyl-[1,1'-biphenyl]-3-carboxylate [Preparative Example 7] (yellow solid), which was used in the next step without further purification. (65 mg, 55%)

¹H NMR (400 MHz, chloroform-*d*) δ 8.06 (s, 1H), 7.90 (s, 1H), 7.58 (s, 1H), 7.56 - 7.53 (m, 2H), 7.03 - 7.00 (m, 2H), 4.57 - 4.47 (m, 1H), 3.95 (s, 3H), 3.77 - 3.67 (m, 2H), 2.96 - 2.89 (m, 2H), 2.47 (s, 3H), 2.11 - 2.07 (m, 2H), 1.63 - 1.53 (m, 2H), 1.49 (s, 9H); LC-MS: 425.5 [M+H⁺].

### [Preparative Example 8]

### 4'-(4-((tert-butoxycarbonyl)amino)piperidine-1-yl)-5-methyl-[1,1'-biphenyl]-3-carboxylic acid

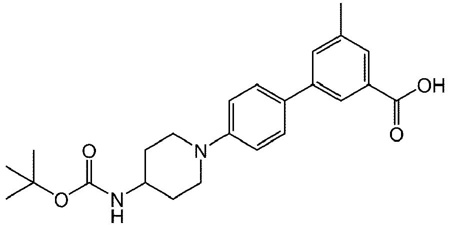

At room temperature, NaOH (5.6 mg, 0.14 mmol) was added to a solution of methyl 4'-(4-((tert-butoxycarbonyl)amino)piperidine-1-yl)-S-methyl-[1,1'-biphenyl]-3-carboxylate (58.9 mg, 0.14 mmol) dissolved in dioxane. The mixture was heated at 110 °C and stirred for 12 hours. 1 N hydrochloric acid was added until the pH of the mixture was 2. The resulting solid was filtered and dried to give 4'-(4-((tert-butoxycarbonyl)amino)piperidine-1-yl)-5-methyl-[1,1'-biphenyl]-3-carboxylic acid [Preparative Example 8] as a yellow solid (45 mg, 79%).

### [Preparative Example 9]

### tert-butyl (R)-(1-(3'-(((5-fluoro-2-methoxyphenyl)(1H-indole-2-yl)methyl)carbamoyl)-5'-methyl-[1,1'-biphenyl]-4-yl)piperidine-4-yl)carbamate

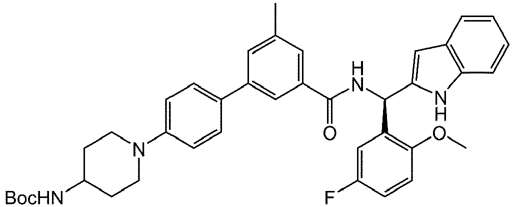

Triethylamine (0.017 ml, 0.12 mmol) was added to a solution of (R)-(5-fluoro-2-methoxyphenyl)(1H-indole-2-yl)methaneamine [Preparative Example 4] (30 mg, 0.11 mmol), 4'-(4-((tert-butoxycarbonyl)amino)piperidine-1-yl)-5-methyl-[1,1'-biphenyl]-3-carboxylic acid [Preparative Example 8] (45 mg, 0.11 mmol), 1-ethyl-3-(3-dimethylaminopropyl)carbodiimide (17 mg, 0.11 mmol), and hydroxybenzotriazole (15 mg, 0.11 mmol) dissolved in CH₂Cl₂ at room temperature. The mixture was stirred overnight. The residue was extracted with water and sodium bicarbonate, and washed with brine. The organic layer was dried over magnesium sulfate and then concentrated. The crude compound was purified by MPLC to give tert-butyl (R)-(1-(3'-(((5-fluoro-2-methoxyphenyl)(1H-indole-2-yl)methyl)carbamoyl)-5'-methyl-[1,1'-biphenyl]-4-yl)piperidine-4-yl)carbamate [Preparative Example 9] as a yellow solid. (48 mg, 66%)

¹H NMR (400 MHz, chloroform-*d*) δ 8.87 (s, 1H), 7.79 (s, 1H), 7.73 (d, J = 8.4 Hz, 1H), 7.54 - 7.36 (m, 5H), 7.34 (d, J = 8.0 Hz, 1H), 7.20 - 6.99 (m, 7H), 6.72 (d, J = 8.0 Hz, 1H), 6.06 (s, 1H), 4.50 (m, 1H), 3.90 (s, 3H), 3.72 - 3.66 (m, 2H), 2.95 - 2.89 (m, 2H), 2.47 (s, 3H), 2.11 - 2.08 (m, 2H), 1.49 (s, 9H); LC-MS: 663.8 [M+H⁺].

### [Example 1]

### (R)-4'-(4-aminopiperidine-1-yl)-N-((5-fluoro-2-hydroxyphenyl)(1H-indole-2-yl)methyl)-5-methyl-[1,1'-biphenyl]-3-carboxamide

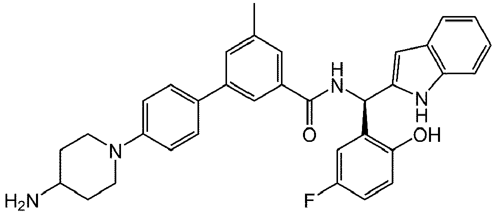

1 M boron tribromide dissolved in dichloromethane (0.20 ml) was slowly added to tert-butyl (R)-(1-(3'-(((5-fluoro-2-methoxyphenyl)(1H-indole-2-yl)methyl)carbamoyl)-5'-methyl-[1,1'-biphenyl]-4-yl)piperidine-4-yl)carbamate [Preparative Example 9] (45 mg, 0.068 mmol) at 0 °C. The reaction mixture was stirred at room temperature overnight, to which sodium bicarbonate was added at 0°C to terminate the reaction. Tetrahydrofuran was added thereto along with brine. The collected organic layer was dried over MgSO_{4,} filtered and concentrated. The residue was purified by PTLC to give (R)-4'-(4-aminopiperidine-1-yl)-N-((5-fluoro-2-hydroxyphenyl)(1H-indole-2-yl)methyl)-5-methyl-[1,1'-biphenyl]-3-carboxamide [Example 1] (11 mg, 30%) as a white solid.

¹H NMR (400 MHz, DMSO-*d6*) δ 11.12 (br s, 1H), 9.27 (br d, J=8.38 Hz, 1H), 7.97 (s, 1H), 7.66 (s, 1H), 7.55-7.62 (m, 3H), 7.43 (d, J=7.88 Hz, 1H), 7.33 (d, J=8.00 Hz, 1H), 7.21 (dd, J=3.13, 9.76 Hz, 1H), 6.90-7.06 (m, 5H), 6.87-6.87 (m, 1H), 6.78-6.88 (m, 1H), 5.99 (s, 1H), 3.67-3.73 (m, 2H), 2.74-2.81 (m, 2H), 2.65-2.71 (m, 1H), 2.46 (br s, 1H), 2.41 (s, 3H), 1.71-1.83 (m, 2H), 1.23-1.37 (m, 2H); LC-MS: 549.6 [M+H⁺].

### [Example 2]

### (R)-4'-(4-aminopiperidine-1-yl)-N-((5-fluoro-2-hydroxyphenyl)(1H-indole-2-yl)methyl)-5-isopropyl-[1,1'-biphenyl]-3-carboxamide

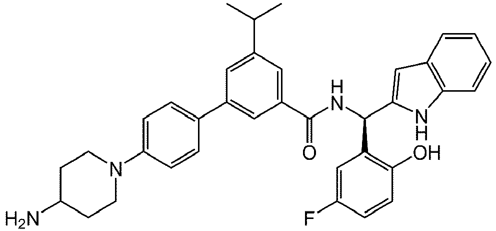

(R)-4'-(4-aminopiperidine-1-yl)-N-((5-fluoro-2-hydroxyphenyl)(1H-indole-2-yl)methyl)-5-isopropyl-[1,1'-biphenyl]-3-carboxamide [Example 2] (34%) was prepared by a method similar to the method described in [Example 1]; LC-MS: 577.7 [M+H⁺].

### [Example 3]

### (R)-4'-(4-aminopiperidine-1-yl)-N-((5-fluoro-2-hydroxyphenyl)(1H-indole-2-yl)methyl)-5-methoxy-[1,1'-biphenyl]-3-carboxamide

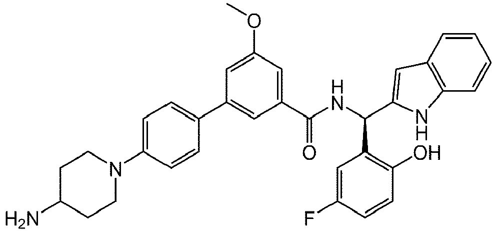

(R)-4'-(4-aminopiperidine-1-yl)-N-((5-fluoro-2-hydroxyphenyl)(1H-indole-2-yl)methyl)-5-methoxy-[1,1'-biphenyl]-3-carboxamide [Example 3] (53%) was prepared by a method similar to the method described in [Example 1].

¹H NMR (400 MHz, DMSO-*d₆*) δ 11.16 (s, 1H), 9.28 (d, J = 8.4 Hz, 1H), 7.63 (s, 1H), 7.53 (d, J = 8.8 Hz, 2H), 7.41 (d, J = 8.0 Hz, 1H), 7.36-7.31 (m, 2H), 7.24 (s, 1H), 7.18-6.99 (m, 6H), 6.96 -6.92 (m, 1H), 6.85 (d, J = 8.4 Hz, 1H), 5.92 (s, 1H), 3.80 (s, 3H), 3.72-3.70 (m, 2H), 2.80-2.74 (m, 3H), 1.81-1.78 (m, 2H), 1.38-1.30 (m, 2H); LC-MS: 565.6 [M+H⁺].

### [Example 4]

### (R)-4'-(4-aminopiperidine-1-yl)-5-(tert-butyl)-N-((5-fluoro-2-hydroxyphenyl)(1H-indole-2-yl)methyl)-[1,1'-biphenyl]-3-carboxamide

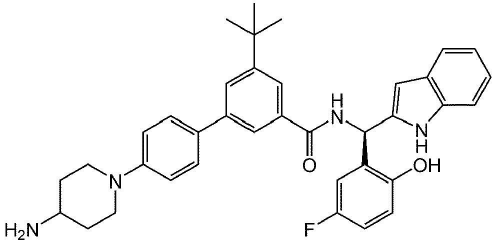

(R)-4'-(4-aminopiperidine-1-yl)-5-(tert-butyl)-N-((5-fluoro-2-hydroxyphenyl)(1H-indole-2-yl)methyl)-[1,1'-biphenyl]-3-carboxamide [Example 4] (16%) was prepared by a method similar to the method described in [Example 1]; LC-MS: 591.7 [M+H⁺].

### [Example 5]

### (R)-4'-(4-aminopiperidine-1-yl)-5-fluoro-N-((5-fluoro-2-hydroxyphenyl)(1H-indole-2-yl)methyl)-[1,1'-biphenyl]-3-carboxamide

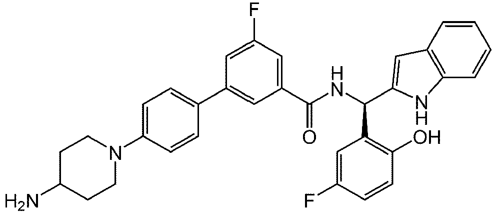

(R)-4'-(4-aminopiperidine-1-yl)-5-fluoro-N-((5-fluoro-2-hydroxyphenyl)(1H-indole-2-yl)methyl)-[1,1'-biphenyl]-3-carboxamide [Example 5] (22%) was prepared by a method similar to the method described in [Example 1].

¹H NMR (400 MHz, DMSO-*d₆*) δ 11.16 (s, 1H), 9.41 (d, J = 8.0 Hz, 1H), 8.07 (s, 1H), 7.66-7.60 (m, 5H), 7.43 (d, J = 7.6 Hz, 1H), 7.32 (d, J = 8.0 Hz, 1H), 7.20 (dd, J = 3.2, 9.6 Hz, 1H), 7.06-6.92 (m, 6H), 6.88-6.81 (m, 3H), 5.97 (s, 1H), 3.77-3.74 (m, 2H), 2.83-2.77 (m, 3H), 1.82-1.79 (m, 2H), 1.36-1.30 (m, 2H);LC-MS: 553.6 [M+H⁺].

### [Example 6]

### 4'-(4-aminopiperidine-1-yl)-N-((5-fluoro-2-hydroxyphenyl)(1H-indole-2-yl)methyl)-5-(trifluoromethyl)-[1,1'-biphenyl]-3-carboxamide

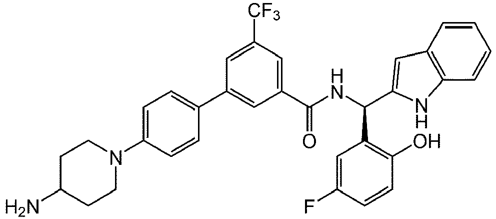

4'-(4-Aminopiperidine-1-yl)-N-((5-fluoro-2-hydroxyphenyl)(1H-indole-2-yl)methyl)-5-(trifluoromethyl)-[1,1'-biphenyl]-3-carboxamide [Example 6] (2%) was prepared by a method similar to the method described in [Example 1].

¹H NMR (400 MHz, DMSO-*d₆*) δ 11.07-11.21 (m, 1H), 9.51-9.62 (m, 1H), 8.33-8.45 (m, 1H), 8.10-8.15 (m, 1H), 8.00-8.06 (m, 1H), 7.64-7.70 (m, 2H), 7.40-7.44 (m, 1H), 7.30-7.34 (m, 2H), 7.12-7.16 (m, 1H), 7.04-7.08 (m, 1H), 7.05 (s, 2H), 6.94-6.97 (m, 1H), 6.83-6.88 (m, 1H), 6.78-6.82 (m, 1H), 6.62-6.69 (m, 1H), 5.96-6.00 (m, 1H), 5.29-5.32 (m, 2H), 1.99-2.04 (m, 1H), 1.93-1.98 (m, 2H), 1.82-1.91 (m, 2H), 1.42-1.46 (m, 4H), 0.83 (s, 2H); LC-MS: 603.6 [M+H⁺].

### [Example 7]

### 4'-(4-aminopiperidine-1-yl)-N-((5-fluoro-2-hydroxyphenyl)(1H-indole-2-yl)methyl)-5-nitro-[1,1'-biphenyl]-3-carboxamide

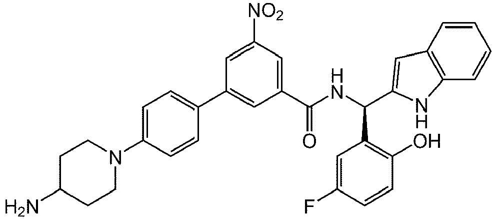

4'-(4-Aminopiperidine-1-yl)-N-((5-fluoro-2-hydroxyphenyl)(1H-indole-2-yl)methyl)-5-nitro-[1,1'-biphenyl]-3-carboxamide [Example 7] (3%) was prepared by a method similar to the method described in [Example 1].

¹H NMR (400 MHz, DMSO-d6) δ 11.08-11.25 (m, 1H), 9.60-9.75 (m, 1H), 8.58-8.65 (m, 2H), 8.48-8.56 (m, 1H), 7.70-7.75 (m, 1H), 7.73 (d, J = 9.01 Hz, 1H), 7.41-7.46 (m, 1H), 7.30-7.37 (m, 1H), 7.15-7.22 (m, 1H), 6.92-7.10 (m, 5H), 6.82-6.90 (m, 2H), 5.91-6.01 (m, 1H), 3.75-3.82 (m, 2H), 2.76-2.91 (m, 3H), 2.41-2.47 (m, 1H), 1.75-1.88 (m, 2H), 1.32-1.43 (m, 2H), 1.24 (s, 2H); LC-MS: 580.6 [M+H⁺].

### [Example 8]

### 4'-(4-aminopiperidine-1-yl)-N-((5-fluoro-2-hydroxyphenyl)(1H-indole-2-yl)methyl)-5-(methylthio)-[1,1'-biphenyl]-3-carboxamide

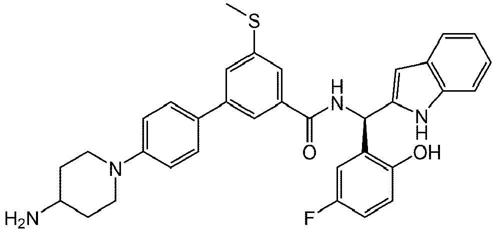

4'-(4-Aminopiperidine-1-yl)-N-((5-fluoro-2-hydroxyphenyl)(1H-indole-2-yl)methyl)-5-(methylthio)-[1,1'-biphenyl]-3-carboxamide [Example 8] (2%) was prepared by a method similar to the method described in [Example 1].

¹H NMR (400 MHz, DMSO-d6) δ 11.04-11.16 (m, 1H), 9.22-9.39 (m, 1H), 7.92 (t, J = 1.44 Hz, 1H), 7.69 (d, J = 1.50 Hz, 1H), 7.56-7.64 (m, 3H), 7.42 (d, J = 7.88 Hz, 1H), 7.33 (dd, J = 0.69, 8.07 Hz, 1H), 7.19 (dd, J = 3.13, 9.76 Hz, 1H), 7.00-7.08 (m, 3H), 6.91-6.99 (m, 2H), 6.79-6.88 (m, 2H), 5.96 (s, 1H), 3.74 (br d, J = 12.76 Hz, 2H), 3.70-3.71 (m, 1H), 2.75-2.86 (m, 3H), 2.75-2.76 (m, 1H), 2.57 (s, 3H), 2.44 (s, 1H), 1.74-1.86 (m, 2H), 1.33-1.40 (m, 2H), 1.24 (s, 2H);LC-MS: 581.7 [M+H⁺].

### [Example 9]

### 4'-(4-aminopiperidine-1-yl)-5-cyano-N-((5-fluoro-2-hydroxyphenyl)(1H-indole-2-yl)methyl)-[1,1'-biphenyl]-3-carboxamide

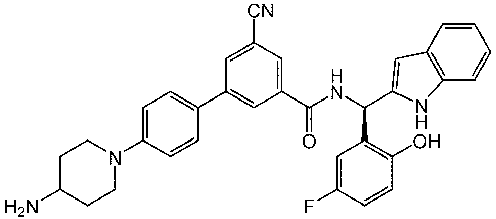

4'-(4-aminopiperidine-1-yl)-5-cyano-N-((5-fluoro-2-hydroxyphenyl)(1H-indole-2-yl)methyl)-[1,1'-biphenyl]-3-carboxamide [Example 9] (2%) was prepared by a method similar to the method described in [Example 1].

¹H NMR (400 MHz, DMSO-d6) δ 11.04-11.24 (m, 1H), 9.39-9.61 (m, 1H), 8.43-8.46 (m, 1H), 8.21-8.30 (m, 2H), 7.65-7.73 (m, 2H), 7.40-7.46 (m, 1H), 7.29-7.36 (m, 1H), 7.14-7.21 (m, 1H), 6.92-7.09 (m, 5H), 6.84-6.89 (m, 1H), 6.81 (d, J = 8.25 Hz, 1H), 5.98 (s, 1H), 3.80-3.85 (m, 2H), 2.96-3.07 (m, 1H), 2.79-2.89 (m, 2H), 1.96-2.03 (m, 1H), 1.81-1.89 (m, 2H), 1.41-1.51 (m, 2H), 0.81-0.90 (m, 2H); LC-MS: 660.6 [M+H⁺].

### [Example 10]

### (R)-4'-(4-aminopiperidine-1-yl)-5-chloro-N-((5-fluoro-2-hydroxyphenyl)(1H-indole-2-yl)methyl)-[1,1'-biphenyl]-3-carboxamide

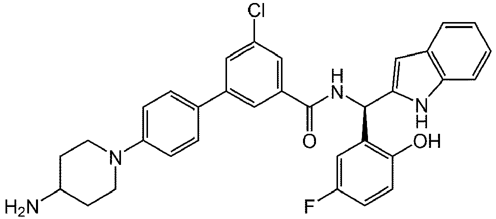

(R)-4'-(4-aminopiperidine-1-yl)-5-chloro-N-((5-fluoro-2-hydroxyphenyl)(1H-indole-2-yl)methyl)-[1,1'-biphenyl]-3-carboxamide [Example 10] (15%) was prepared by a method similar to the method described in [Example 1].

¹H NMR (400 MHz, DMSO-d6) δ 11.16 (br s, 1H), 9.46 (br d, J = 8.00 Hz, 1H), 8.13 (t, J = 1.50 Hz, 1H), 7.81-7.86 (m, 2H), 7.63 (d, J = 9.01 Hz, 2H), 7.40-7.47 (m, 1H), 7.33 (t, J = 1.00 Hz, 1H), 7.18 (dd, J = 3.19, 9.69 Hz, 1H), 7.00-7.06 (m, 3H), 6.92-6.99 (m, 2H), 6.83-6.88 (m, 1H), 6.80 (d, J = 1.00 Hz, 1H), 5.97 (s, 1H), 3.73 (br d, J = 12.76 Hz, 2H), 2.70-2.83 (m, 3H), 1.77 (br dd, J = 2.94, 12.57 Hz, 2H), 1.24-1.36 (m, 2H); LC-MS: 570.0 [M+H⁺].

### [Example 11]

### (R)-4'-(4-aminopiperidine-1-yl)-N-((5-fluoro-2-hydroxyphenyl)(1H-indole-2-yl)methyl)-5-(trifluoromethoxy)-[1,1'-biphenyl]-3-carboxamide

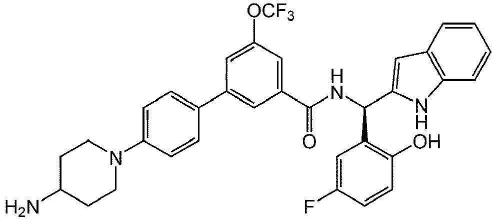

(R)-4'-(4-aminopiperidine-1-yl)-N-((5-fluoro-2-hydroxyphenyl)(1H-indole-2-yl)methyl)-5-(trifluoromethoxy)-[1,1'-biphenyl]-3-carboxamide [Example 11] (8%) was prepared by a method similar to the method described in [Example 1].

¹H NMR (400 MHz, DMSO-*d6*) δ 11.18 (br s, 1H), 9.53 (br d, J = 8.00 Hz, 1H), 8.26 (t, J = 1.00 Hz, 1H), 7.74 (d, J = 1.00 Hz, 2H), 7.66 (d, J = 8.88 Hz, 2H), 7.43 (d, J = 7.88 Hz, 1H), 7.32 (t, J = 7.74 Hz, 1H), 7.18 (dd, J = 3.19, 9.69 Hz, 1H), 7.00-7.07 (m, 3H), 6.92-6.99 (m, 2H), 6.80-6.89 (m, 2H), 5.96 (s, 1H), 3.73 (br q, J = 13.01 Hz, 1H), 2.72-2.84 (m, 3H), 1.77 (br d, J = 9.88 Hz, 2H), 1.27-1.36 (m, 2H); LC-MS: 619.6 [M+H⁺].

### [Example 12]

### (R)-4'-(4-aminopiperidine-1-yl)-N-((5-fluoro-2-methoxyphenyl)(1H-indole-2-yl)methyl)-5-(trifluoromethoxy)-[1,1'-biphenyl]-3-carboxamide

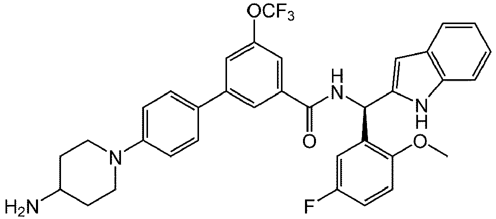

After preparing an intermediate by a method similar to the method described in [Example 1], (R)-4'-(4-aminopiperidine-1-yl)-N-((5-fluoro-2-methoxyphenyl)(1H-indole-2-yl)methyl)-5-(trifluoromethoxy)-[1,1'-biphenyl]-3-carboxamide [Example 12] (8%) was obtained as a by-product of [Example 11].

¹H NMR (400 MHz, DMSO-*d6*) δ 11.22 (s, 1H), 9.58 (d, J = 8.26 Hz, 1H), 8.26 (t, J = 1.44 Hz, 1H), 7.75 (s, 2H), 7.66 (d, J= 9.01 Hz, 2H), 7.43 (d, J = 7.75 Hz, 1H), 7.28-7.34 (m, 2H), 7.17 (q, J = 1.00 Hz, 1H), 7.08-7.12 (m, 1H), 7.02-7.08 (m, 3H), 6.92-6.97 (m, 1H), 6.86 (d, J = 8.13 Hz, 1H), 5.90-5.92 (m, 1H), 3.80 (s, 3H), 3.70-3.76 (m, J = 12.76 Hz, 2H), 2.75-2.84 (m, 3H), 1.74-1.81 (m, 2H), 1.27-1.35 (m, 2H); LC-MS: 633.6 [M+H⁺].

### [Example 13]

### (R)-4'-(4-aminopiperidine-1-yl)-N-((5-fluoro-2-hydroxyphenyl)(1H-indole-2-yl)methyl)-5-(1H-imidazole-1-yl)-[1,1'-biphenyl]-3-carboxamide

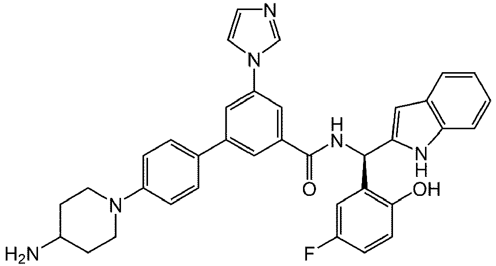

(R)-4'-(4-aminopiperidine-1-yl)-N-((5-fluoro-2-hydroxyphenyl)(1H-indole-2-yl)methyl)-5-(1H-imidazole-1-yl)-[1,1'-biphenyl]-3-carboxamide [Example 13] (44%) was prepared by a method similar to the method described in [Example 1].

¹H NMR (400 MHz, DMSO-d6) δ 11.17 (br s, 1H), 9.44 (br d, J = 7.38 Hz, 1H), 8.42 (t, J = 1.00 Hz, 1H), 8.14 (s, 1H), 8.02 (t, J = 1.38 Hz, 1H), 8.00 (t, J = 1.63 Hz, 1H), 7.91 (t, J=1.25 Hz, 1H), 7.72 (d, J = 8.88 Hz, 2H), 7.43 (d, J = 7.75 Hz, 1H), 7.33 (t, J = 1.00 Hz, 1H), 7.18 (dd, J = 3.13, 9.63 Hz, 1H), 7.14 (t, J = 1.00 Hz, 1H), 7.02-7.07 (m, 3H), 6.92-7.02 (m, 2H), 6.81-6.89 (m, 2H), 6.00 (s, 1H), 3.71-3.77 (m, 2H), 2.69-2.88 (m, 3H), 1.74-1.82 (m, 2H), 1.25-1.39 (m, 2H); LC-MS: 601.6 [M+H⁺].

### [Example 14]

### (R)-2-(4-(4-aminopiperidine-1-yl)phenyl)-N-((5-fluoro-2-hydroxyphenyl)(1H-indole-2-yl)methyl)isonicotinamide

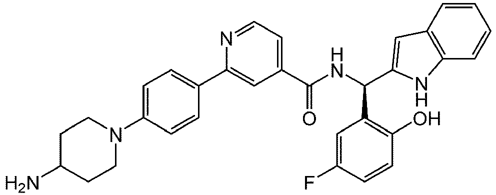

(R)-2-(4-(4-aminopiperidine-1-yl)phenyl)-N-((5-fluoro-2-hydroxyphenyl)(1H-indole-2-yl)methyl)isonicotinamide [Example 14] (12%) was prepared by a method similar to the method described in [Example 1].

¹H NMR (400 MHz, DMSO-*d6*) δ 11.19 (br s, 1H), 9.60 (br d, J = 7.75 Hz, 1H), 8.71 (d, J = 5.00 Hz, 1H), 8.30 (s, 1H), 8.01 (d, J = 9.00 Hz, 2H), 7.65 (dd, J = 1.50, 5.13 Hz, 1H), 7.44 (d, J = 7.75 Hz, 1H), 7.38 (br d, J = 4.75 Hz, 1H), 7.30-7.36 (m, 1H), 7.19 (dd, J = 3.13, 9.63 Hz, 1H), 7.00-7.07 (m, 4H), 6.92-7.00 (m, 2H), 6.80-6.89 (m, 2H), 5.99 (s, 1H), 3.76 (br d, J = 12.76 Hz, 2H), 2.71-2.86 (m, 3H), 1.78 (br d, J = 10.26 Hz, 2H), 1.26-1.37 (m, 2H); LC-MS: 536.6 [M+H⁺].

### [Example 15]

### (R)-2-(4-(4-aminopiperidine-1-yl)phenyl)-N-((5-fluoro-2-methoxyphenyl) (1H-indole-2-yl)methyl)isonicotinamide

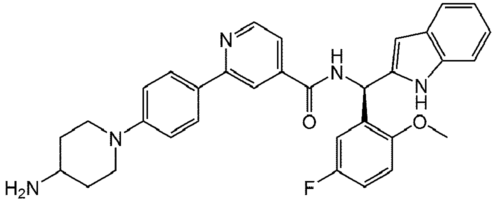

(R)-2-(4-(4-aminopiperidine-1-yl)phenyl)-N-((5-fluoro-2-methoxyphenyl)(1H-indole-2-yl)methyl)isonicotinamide [Example 15] (16%) was prepared by a method similar to the method described in [Example 1].

¹H NMR (400 MHz, DMSO-*d6*) δ 11.22 (s, 1H), 9.64 (d, J = 8.26 Hz, 1H), 8.71 (d, J = 5.12 Hz, 1H), 8.30 (s, 1H), 8.01 (d, J = 9.01 Hz, 2H), 7.62-7.68 (m, 1H), 7.43 (d, J = 7.88 Hz, 1H), 7.28-7.36 (m, 2H), 7.14-7.20 (m, 1H), 7.02-7.12 (m, 4H), 6.93-6.98 (m, 1H), 6.86 (d, J = 8.13 Hz, 1H), 5.92-5.95 (m, 1H), 3.81 (s, 3H), 3.76 (br d, J = 12.88 Hz, 2H), 2.70-2.86 (m, 3H), 1.74-1.82 (m, 2H), 1.27-1.36 (m, 2H); LC-MS: 550.6 [M+H⁺].

### [Example 16]

### (R)-3-(2-(4-aminopiperidine-1-yl)pyrimidine-5-yl)-N-((5-fluoro-2-hydroxyphenyl) (1H-indole-2-yl)methyl)-5-methylbenzamide

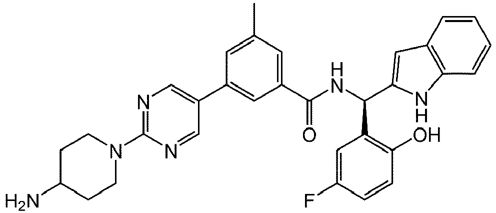

(R)-3-(2-(4-aminopiperidine-1-yl)pyrimidine-5-yl)-N-((5-fluoro-2-hydroxyphenyl)(1H-indole-2-yl)methyl)-5-methylbenzamide [Example 16] (31%) was prepared by a method similar to the method described in [Example 1].

¹H NMR (400 MHz, DMSO-d6) δ 11.13 (br s, 1H), 9.21 (br d, J = 8.50 Hz, 1H), 8.72-8.79 (m, 2H), 8.01 (s, 1H), 7.72 (s, 1H), 7.64 (s, 1H), 7.43 (d, J = 7.75 Hz, 1H), 7.33 (dd, J = 0.75, 8.00 Hz, 1H), 7.20 (dd, J = 3.19, 9.69 Hz, 1H), 7.03 (dt, J = 1.25, 7.57 Hz, 1H), 6.91-7.01 (m, 2H), 6.85 (dd, J = 4.88, 8.88 Hz, 1H), 6.81 (d, J = 8.13 Hz, 1H), 5.99 (s, 1H), 4.50-4.61 (m, 2H), 3.01-3.10 (m, 2H), 2.81-2.91 (m, 1H), 2.42 (s, 3H), 1.74-1.81 (m, 2H), 1.12-1.22 (m, 2H); LC-MS: 550.6 [M+H⁺].

### [Example 17]

### (R)-3-(5-(4-aminopiperidine-1-yl)pyridine-2-yl)-N-((5-fluoro-2-hydroxyphenyl)(1H-indole-2-yl)methyl)-5-methylbenzamide

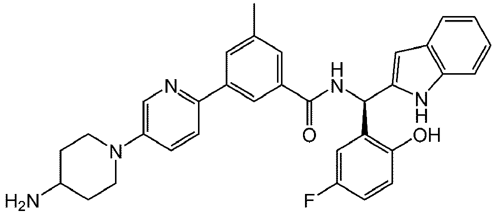

(R)-3-(5-(4-aminopiperidine-1-yl)pyridine-2-yl)-N-((5-fluoro-2-hydroxyphenyl)(1H-indole-2-yl)methyl)-5-methylbenzamide [Example 17] (16%) was prepared by a method similar to the method described in [Example 1].

¹H NMR (400 MHz, DMSO-d6) δ 11.13 (br s, 1H), 9.27 (br d, J = 8.38 Hz, 1H), 8.38 (d, J = 2.75 Hz, 1H), 8.32 (s, 1H), 8.30 (br s, 1H), 7.99 (s, 1H), 7.84 (d, J = 8.88 Hz, 1H), 7.71 (s, 1H), 7.39-7.44 (m, 2H), 7.33 (dd, J = 0.69, 8.07 Hz, 1H), 7.23 (dd, J = 3.25, 9.76 Hz, 1H), 7.04 (dt, J = 1.19, 7.60 Hz, 1H), 6.91-7.00 (m, 2H), 6.80-6.87 (m, 2H), 6.00 (s, 1H), 3.76 (br d, J = 12.63 Hz, 2H), 2.80-2.87 (m, 2H), 2.71-2.79 (m, 1H), 2.43 (s, 3H), 1.76-1.83 (m, 2H), 1.30-1.38 (m, 2H) ; LC-MS: 550.6 [M+H⁺].

### [Example 18]

### (R)-3-(5-(4-aminopiperidine-1-yl)pyrazine-2-yl)-N-((5-fluoro-2-hydroxyphenyl)(1H-indole-2-yl)methyl)-5-methylbenzamide

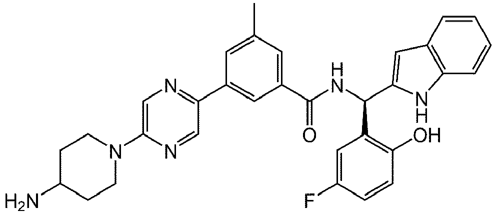

(R)-3-(5-(4-aminopiperidine-1-yl)pyrazine-2-yl)-N-((5-fluoro-2-hydroxyphenyl)(1H-indole-2-yl)methyl)-5-methylbenzamide [Example 18] (46%) was prepared by a method similar to the method described in [Example 1].

¹H NMR (400 MHz, DMSO-d6) δ 11.12 (br s, 1H), 9.24 (br d, J = 8.38 Hz, 1H), 8.70-8.75 (m, 1H), 8.41 (t, J = 1.00 Hz, 1H), 8.27-8.34 (m, 1H), 7.92-7.97 (m, 1H), 7.73 (s, 1H), 7.43 (d, J = 7.75 Hz, 1H), 7.33 (t, J = 8.13 Hz, 1H), 7.23 (dd, J = 3.25, 9.76 Hz, 1H), 7.04 (dt, J = 1.25, 7.57 Hz, 1H), 6.91-7.00 (m, 2H), 6.85 (dd, J = 4.82, 8.82 Hz, 1H), 6.81 (d, J = 8.38 Hz, 1H), 6.00 (s, 1H), 4.20-4.35 (m, 2H), 2.95-3.09 (m, 2H), 2.79-2.88 (m, 1H), 2.43 (s, 3H), 1.62-1.85 (m, 2H), 1.17-1.33 (m, 2H); LC-MS: 551.6 [M+H⁺].

### [Example 19]

### (R)-3-(6-(4-aminopiperidine-1-yl)pyridine-3-yl)-N-((5-fluoro-2-hydroxyphenyl)(1H-indole-2-yl)methyl)-5-methylbenzamide

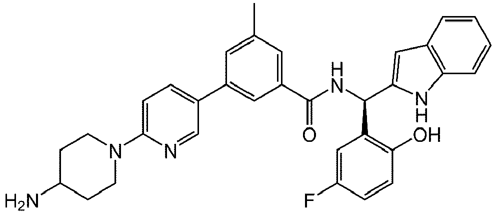

(R)-3-(6-(4-aminopiperidine-1-yl)pyridine-3-yl)-N-((5-fluoro-2-hydroxyphenyl)(1H-indole-2-yl)methyl)-5-methylbenzamide [Example 19] (83%) was prepared by a method similar to the method described in [Example 1].

¹H NMR (400 MHz, DMSO-d6) δ 11.14 (br s, 1H), 9.25 (br s, 1H), 8.50 (d, J = 2.50 Hz, 1H), 7.98 (s, 1H), 7.88 (dd, J = 2.56, 8.94 Hz, 1H), 7.68 (s, 1H), 7.61 (s, 1H), 7.42 (d, J = 7.75 Hz, 1H), 7.33 (t, J = 8.00 Hz, 1H), 7.20 (dd, J = 3.06, 9.69 Hz, 1H), 7.00-7.06 (m, 1H), 6.90-7.00 (m, 3H), 6.78-6.87 (m, 2H), 5.99 (s, 1H), 4.23 (br d, J = 12.76 Hz, 2H), 2.88-2.98 (m, 2H), 2.75-2.85 (m, 1H), 2.41 (s, 3H), 1.75 (br d, J = 12.38 Hz, 2H), 1.15-1.24 (m, 2H); LC-MS: 550.6 [M+H⁺].

### [Example 20]

### (R)-3-(6-(4-aminopiperidine-1-yl)pyridazine-3-yl)-N-((5-fluoro-2-hydroxyphenyl) (1H-indole-2-yl)methyl)-5-methylbenzamide

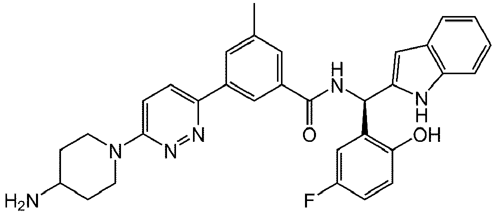

(R)-3-(6-(4-aminopiperidine-1-yl)pyridazine-3-yl)-N-((5-fluoro-2-hydroxyphenyl)(1H-indole-2-yl)methyl)-5-methylbenzamide [Example 20] (72%) was prepared by a method similar to the method described in [Example 1].

¹H NMR (400 MHz, DMSO-d6) δ ppm 11.08 - 11.21 (m, 1H),, 9.25 - 9.45 (m, 1H) 8.00 - 8.11 (m, 1H), 8.37 (s, 1 H), 7.96 (d, J = 9.76 Hz, 1H), 7.80 (s, 1 H), 7.30 - 7.45 (m, 3 H), 7.18 - 7.26 (m, 1 H), 6.95 (br d, J = 6.88 Hz, 3 H), 6.83 (s, 2 H), 6.00 (s, 1 H), 5.97 - 5.97 (m, 1 H), 4.24 - 4.40 (m, 2 H), 3.00 - 3.13 (m, 2 H),, 2.80 - 2.89 (m, 1 H), 2.42 - 2.46 (m, 3 H), 1.74 - 1.84 (m, 2 H), 1.20 - 1.29 (m, 2 H); LC-MS: 551.6 [M+H⁺].

### [Example 21]

### (R)-3-(5-(4-aminopiperidine-1-yl)pyrimidine-2-yl)-N-((5-fluoro-2-hydroxyphenyl) (1H-indole-2-yl)methyl)-5-methylbenzamide

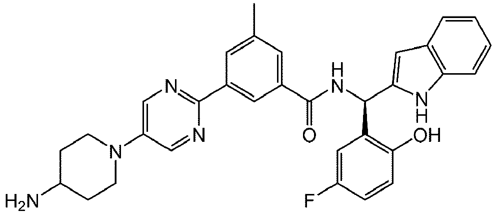

(R)-3-(5-(4-aminopiperidine-1-yl)pyrimidine-2-yl)-N-((5-fluoro-2-hydroxyphenyl)(1H-indole-2-yl)methyl)-5-methylbenzamide [Example 21] (24%) was prepared by a method similar to the method described in [Example 1].

¹H NMR (400 MHz, DMSO-d6) δ 11.11 (br s, 1H), 9.29 (br d, J = 8.63 Hz, 1H), 8.57-8.61 (m, 3H), 8.24 (s, 1H), 7.80 (s, 1H), 7.43 (d, J = 7.88 Hz, 1H), 7.33 (d, J = 7.96 Hz, 1H), 7.25 (dd, J = 3.19, 9.82 Hz, 1H), 7.04 (dt, J = 1.25, 7.57 Hz, 1H), 6.92-7.00 (m, 2H), 6.79-6.87 (m, 2H), 6.00 (s, 1H), 3.82 (br d, J = 13.01 Hz, 2H), 2.86-2.92 (m, 2H), 2.75-2.81 (m, 1H), 2.44 (s, 3H), 1.76-1.85 (m, 2H), 1.28-1.36 (m, 2H); LC-MS: 551.6 [M+H⁺].

### [Example 22]

### (R)-4'-(4-aminopiperidine-1-yl)-3'-fluoro-N-((5-fluoro-2-hydroxyphenyl) (1H-indole-2-yl)methyl)-5-methyl-[1,1'-biphenyl]-3-carboxamide

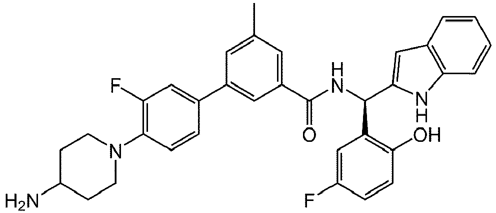

(R)-4'-(4-aminopiperidine-1-yl)-3'-fluoro-N-((5-fluoro-2-hydroxyphenyl)(1H-indole-2-yl)methyl)-5-methyl-[1,1'-biphenyl]-3-carboxamide [Example 22] (59%) was prepared by a method similar to the method described in [Example 1].

¹H NMR (400 MHz, DMSO-d6) δ 11.13 (br s, 1H), 9.27 (br d, J = 8.50 Hz, 1H), 8.00 (s, 1H), 7.71 (s, 1H), 7.65 (s, 1H), 7.47-7.58 (m, 2H), 7.43 (d, J = 7.75 Hz, 1H), 7.32 (t, J=7.72 Hz, 1H), 7.19 (dd, J = 3.13, 9.63 Hz, 1H), 7.11 (t, J = 8.94 Hz, 1H), 7.04 (dt, J = 1.13, 7.57 Hz, 1H), 6.91-7.01 (m, 2H), 6.79-6.89 (m, 2H), 5.98 (s, 1H), 3.37-3.41 (m, 2H), 2.79-2.90 (m, 1H), 2.69-2.76 (m, 2H), 2.42 (s, 3H), 1.77-1.84 (m, 2H), 1.34-1.49 (m, 2H); LC-MS: 567.6 [M+H⁺].

### [Example 23]

### (R)-4'-(4-aminopiperidine-1-yl)-N-((5-fluoro-2-hydroxyphenyl)(1H-indole-2-yl)methyl)-2',5-dimethyl-[1,1'-biphenyl]-3-carboxamide

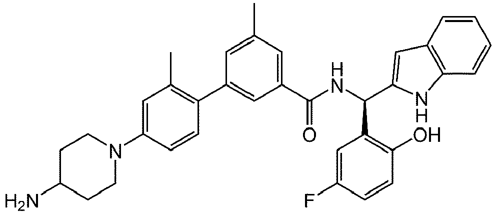

(R)-4'-(4-aminopiperidine-1-yl)-N-((5-fluoro-2-hydroxyphenyl)(1H-indole-2-yl)methyl)-2',5-dimethyl-[1,1'-biphenyl]-3-carboxamide [Example 23] (56%) was prepared by a method similar to the method described in [Example 1].

¹H NMR (400 MHz, DMSO-d6) δ 11.09 (br s, 1H), 9.18 (br d, J = 8.50 Hz, 1H), 7.71 (s, 1H), 7.68 (s, 1H), 7.41 (d, J = 7.63 Hz, 1H), 7.31 (dd, J = 0.75, 8.13 Hz, 1H), 7.28 (s, 1H), 7.20 (dd, J = 3.25, 9.76 Hz, 1H), 7.07 (d, J = 8.38 Hz, 1H), 7.02 (dt, J = 1.19, 7.54 Hz, 1H), 6.91-6.99 (m, 2H), 6.78-6.87 (m, 4H), 5.97 (s, 1H), 3.66 (br d, J = 12.76 Hz, 2H), 2.72-2.77 (m, 2H), 2.67-2.71 (m, 1H), 2.40 (s, 3H), 2.20 (s, 3H), 1.72-1.80 (m, 2H), 1.26-1.36 (m, 1H); LC-MS: 563.6 [M+H⁺].

### [Example 24]

### (R)-4'-(4-aminopiperidine-1-yl)-2'-fluoro-N-((5-fluoro-2-hydroxyphenyl) (1H-indole-2-yl)methyl)-5-methyl-[1,1'-biphenyl]-3-carboxamide

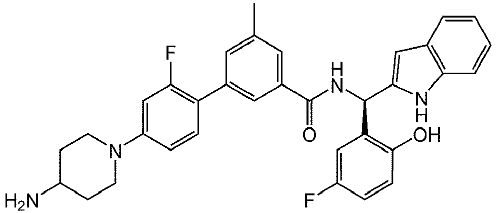

(R)-4'-(4-aminopiperidine-1-yl)-2'-fluoro-N-((5-fluoro-2-hydroxyphenyl)(1H-indole-2-yl)methyl)-5-methyl-[1,1'-biphenyl]-3-carboxamide [Example 24] (55%) was prepared by a method similar to the method described in [Example 1].

¹H NMR (400 MHz, DMSO-d6) δ 11.11 (br s, 1H), 9.22 (br d, J = 8.00 Hz, 1H), 7.85 (s, 1H), 7.71 (s, 1H), 7.47 (s, 1H), 7.37-7.44 (m, 2H), 7.32 (t, J = 8.01 Hz, 1H), 7.20 (dd, J = 3.19, 9.69 Hz, 1H), 7.03 (dt, J = 1.13, 7.57 Hz, 1H), 6.91-7.00 (m, 2H), 6.78-6.87 (m, 4H), 5.98 (s, 1H), 3.73 (br d, J = 12.88 Hz, 2H), 2.77-2.86 (m, 2H), 2.71-2.77 (m, 1H), 2.41 (s, 3H), 1.76 (br d, J = 13.13 Hz, 2H), 1.23-1.33 (m, 2H); LC-MS: 567.6 [M+H⁺].

### [Example 25]

### (R)-4'-(4-aminopiperidine-1-yl)-N-((5-fluoro-2-hydroxyphenyl)(1H-indole-2-yl)methyl)-3',5-dimethyl-[1,1'-biphenyl]-3-carboxamide

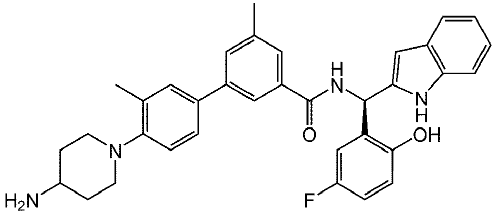

(R)-4'-(4-aminopiperidine-1-yl)-N-((5-fluoro-2-hydroxyphenyl)(1H-indole-2-yl)methyl)-3',5-dimethyl-[1,1'-biphenyl]-3-carboxamide [Example 25] (50%) was prepared by a method similar to the method described in [Example 1].

¹H NMR (400 MHz, DMSO-d6) δ 11.12 (br s, 1H), 9.25 (br d, J = 8.13 Hz, 1H), 7.96 (s, 1H), 7.70 (s, 1H), 7.60 (s, 1H), 7.40-7.53 (m, 3H), 7.32 (t, J = 7.73 Hz, 1H), 7.21 (dd, J = 3.19, 9.69 Hz, 1H), 7. 09 (d, J = 8.25 Hz, 1H), 7.03 (dt, J = 1.13, 7.57 Hz, 1H), 6.91-7.00 (m, 2H), 6.79-6.88 (m, 2H), 5.98 (s, 1H), 3.05 (br d, J = 12.13 Hz, 2H), 2.59-2.69 (m, 3H), 2.42 (s, 3H), 2.30 (s, 3H), 1.77-1.84 (m, 2H), 1.36-1.48 (m, 2H); LC-MS: 563.6 [M+H⁺].

### [Example 26]

### (R)-4'-(4-aminopiperidine-1-yl)-N-((6-fluoro-1H-indole-2-yl) (5-fluoro-2-hydroxyphenyl)methyl)-5-methyl-[1,1'-biphenyl]-3-carboxamide

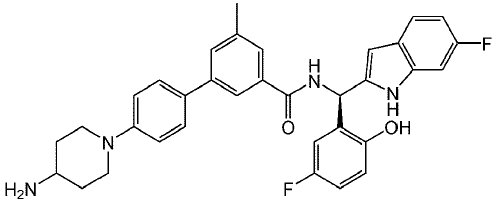

(R)-4'-(4-aminopiperidine-1-yl)-N-((6-fluoro-1H-indole-2-yl)(5-fluoro-2-hydroxyphenyl)methyl)-5-methyl-[1,1'-biphenyl]-3-carboxamide [Example 26] (47%) was prepared by a method similar to the method described in [Example 1].

¹H NMR (400 MHz, DMSO-d6) δ 11.21 (br s, 1H), 9.27 (br d, J = 8.38 Hz, 1H), 7.96 (s, 1H), 7.65 (s, 1H), 7.56-7.60 (m, 3H), 7.42 (dd, J = 5.50, 8.63 Hz, 1H), 7.22 (dd, J = 3.13, 9.76 Hz, 1H), 7.08 (dd, J = 2.31, 10.07 Hz, 1H), 7.01 (d, J = 9.01 Hz, 2H), 6.93-6.98 (m, 1H), 6.77-6.88 (m, 3H), 5.98 (s, 1H), 3.70 (br d, J = 12.76 Hz, 2H), 2.67-2.81 (m, 3H), 2.41 (s, 3H), 1.74-1.82 (m, 2H), 1.23-1.37 (m, 2H); LC-MS: 567.6 [M+H⁺].

### [Example 27]

### (R)-4'-(4-aminopiperidine-1-yl)-N-((6-chloro-1H-indole-2-yl) (5-fluoro-2-hydroxyphenyl)methyl)-5-methyl-[1,1'-biphenyl]-3-carboxamide

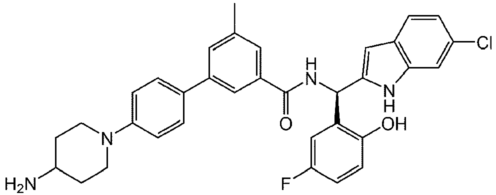

(R)-4'-(4-aminopiperidine-1-yl)-N-((6-chloro-1H-indole-2-yl)(5-fluoro-2-hydroxyphenyl)methyl)-5-methyl-[1,1'-biphenyl]-3-carboxamide [Example 27] (45%) was prepared by a method similar to the method described in [Example 1].

¹H NMR (400 MHz, DMSO-d6) δ 11.31 (br s, 1H), 9.29 (br d, J = 8.63 Hz, 1H), 7.96 (s, 1H), 7.66 (s, 1H), 7.59 (br d, J = 8.50 Hz, 3H), 7.44 (d, J = 8.38 Hz, 1H), 7.32-7.37 (m, 1H), 7.22 (br dd, J = 3.13, 9.76 Hz, 1H), 6.92-7.07 (m, 5H), 6.78-6.92 (m, 2H), 6.01 (s, 1H), 3.78-3.80 (m, 2H), 2.78-2.81 (m, 2H), 2.41 (s, 3H), 1.85-1.91 (m, 2H), 1.44-1.51 (m, 2H); LC-MS: 584.0 [M+H⁺].

### [Example 28]

### (R)-4'-(4-aminopiperidine-1-yl)-N-((5-fluoro-2-hydroxyphenyl)(1H-indole-2-yl)methyl)-6-methyl-[1,1'-biphenyl]-3-carboxamide

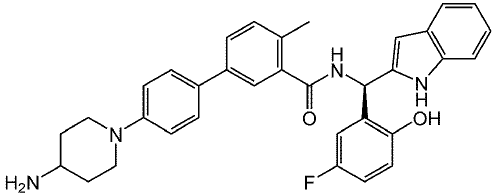

(R)-4'-(4-aminopiperidine-1-yl)-N-((5-fluoro-2-hydroxyphenyl)(1H-indole-2-yl)methyl)-6-methyl-[1,1'-biphenyl]-3-carboxamide [Example 28] (3%) was prepared by a method similar to the method described in [Example 1].

¹H NMR (400 MHz, DMSO-d6) δ 10.50-11.24 (m, 1H), 8.93-9.33 (m, 1H), 7.42 (d, J = 7.75 Hz, 1H), 7.24-7.35 (m, 3H), 7.19-7.24 (m, 1H), 7.09-7.16 (m, 3H), 7.00-7.06 (m, 1H), 6.91-7.00 (m, 4H), 6.78-6.86 (m, 1H), 6.68-6.76 (m, 1H), 5.96-6.14 (m, 1H), 3.93-4.08 (m, 2H), 3.40 (s, 1H), 2.62-2.77 (m, 3H), 2.15 (s, 3H), 1.72-1.86 (m, 2H), 1.25-1.43 (m, 2H); LC-MS: 549.6 [M+H⁺].

### [Example 29]

### (R)-4'-(4-aminopiperidine-1-yl)-N-((5-fluoro-2-hydroxyphenyl)(1H-indole-2-yl)methyl)-4-methyl-[1,1'-biphenyl]-3-carboxamide

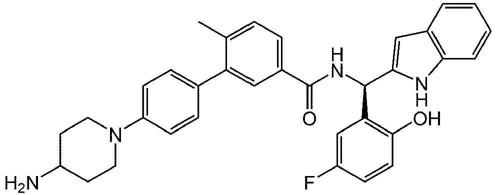

(R)-4'-(4-aminopiperidine-1-yl)-N-((5-fluoro-2-hydroxyphenyl)(1H-indole-2-yl)methyl)-4-methyl-[1,1'-biphenyl]-3-carboxamide [Example 29] (4%) was prepared by a method similar to the method described in [Example 1].

¹H NMR (400 MHz, DMSO-d6) δ 10.77-11.21 (m, 1H), 9.07-9.28 (m, 1H), 7.61 (d, J=1.88 Hz, 1H), 7.51 (d, J=8.75 Hz, 3H), 7.39-7.45 (m, 1H), 7.32-7.37 (m, 1H), 7.27 (s, 1H), 7.10-7.18 (m, 1H), 7.04 (s, 1H), 7.00 (d, J=9.01 Hz, 2H), 6.95 (br d, J=8.00 Hz, 2H), 6.79-6.88 (m, 1H), 6.67-6.77 (m, 1H), 6.03 (s, 1H), 3.87-4.06 (m, 2H), 3.38-3.41 (m, 1H), 2.65-2.75 (m, 3H), 2.30 (s, 3H), 1.69-1.85 (m, 2H), 1.22-1.39 (m, 2H); LC-MS: 549.6 [M+H⁺].

### [Example 30]

### (R)-4'-(4-aminopiperidine-1-yl)-N-((5-fluoro-2-hydroxyphenyl) (1H-indole-2-yl)methyl)-2-methyl-[1,1'-biphenyl]-3-carboxamide

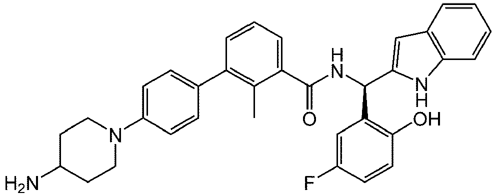

(R)-4'-(4-aminopiperidine-1-yl)-N-((5-fluoro-2-hydroxyphenyl) (1H-indole-2-yl)methyl)-2-methyl-[1,1'-biphenyl]-3-carboxamide [Example 30] (9%) was prepared by a method similar to the method described in [Example 1].

¹H NMR (400 MHz, DMSO-d6) δ 10.83-11.27 (m, 1H), 9.04-9.32 (m, 1H), 7.76-7.83 (m, 2H), 7.29-7.43 (m, 3H), 7.23 (d, J = 8.63 Hz, 3H), 7.01 (s, 5H), 6.76-6.87 (m, 2H), 5.97 (s, 1H), 5.76 (s, 1H), 3.68 (br d, J = 12.63 Hz, 2H), 3.37-3.42 (m, 1H), 2.64-2.81 (m, 3H), 2.30 (s, 3H), 1.73-1.81 (m, 2H), 1.26-1.39 (m, 2H); LC-MS: 549.6 [M+H⁺].

### [Example 31]

### (S)-4'-(4-aminopiperidine-1-yl)-N-((5-fluoro-2-hydroxyphenyl)(1H-indole-2-yl)methyl)-5-methyl-[1,1'-biphenyl]-3-carboxamide

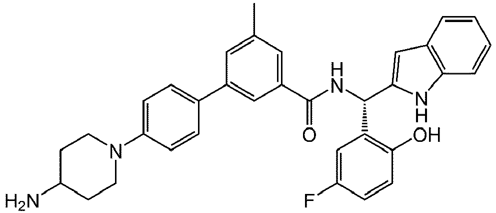

(S)-4'-(4-aminopiperidine-1-yl)-N-((5-fluoro-2-hydroxyphenyl)(1H-indole-2-yl)methyl)-5-methyl-[1,1'-biphenyl]-3-carboxamide [Example 31] (53%) was prepared by a method similar to the method described in [Example 1].

¹H NMR (400 MHz, DMSO-d6) δ 11.11 (br s, 1H), 9.25 (br d, J = 8.51 Hz, 1H), 7.97 (s, 1H), 7.66 (s, 1H), 7.54-7.62 (m, 3H), 7.42 (d, J = 7.75 Hz, 1H), 7.33 (dd, J = 0.81, 8.07 Hz, 1H), 7.21 (dd, J = 3.13, 9.76 Hz, 1H), 6.91-7.06 (m, 6H), 6.79-6.87 (m, 2H), 5.98 (s, 1H), 3.65-3.74 (m, 2H), 2.69-2.81 (m, 3H), 2.41 (s, 3H), 1.73-1.83 (m, 2H), 1.25-1.37 (m, 2H); LC-MS: 549.6 [M+H⁺].

### [Example 32]

### 4'-(4-aminopiperidine-1-yl)-N-((5-fluoro-2-hydroxyphenyl)(1H-indole-2-yl)methyl)-5-methyl-[1,1'-biphenyl]-3-carboxamide

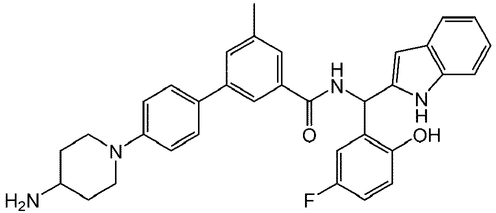

4'-(4-Aminopiperidine-1-yl)-N-((5-fluoro-2-hydroxyphenyl)(1H-indole-2-yl)methyl)-5-methyl-[1,1'-biphenyl]-3-carboxamide [Example 32] (31%) was prepared by a method similar to the method described in [Example 1].

¹H NMR (400 MHz, DMSO-d6) δ 11.12 (br s, 1H), 9.26 (br d, J = 8.50 Hz, 1H), 7.96 (s, 1H), 7.65 (s, 1H), 7.54-7.63 (m, 3H), 7.42 (d, J = 7.88 Hz, 1H), 7.32 (d, J = 8.76 Hz, 1H), 7.21 (dd, J = 3.19, 9.69 Hz, 1H), 6.91-7.05 (m, 5H), 6.79-6.87 (m, 2H), 5.98 (s, 1H), 3.69 (br d, J = 12.63 Hz, 2H), 2.70-2.80 (m, 3H), 2.41 (s, 3H), 1.74-1.82 (m, 2H), 1.24-1.37 (m, 2H); LC-MS: 549.6 [M+H⁺].

### [Example 33]

### (R)-3-(5-(4-aminopiperidine-1-yl)-4-methylpyridine-2-yl)-N-((5-fluoro-2-hydroxyphenyl)(1H-indole-2-yl)methyl)-5-methylbenzamide;

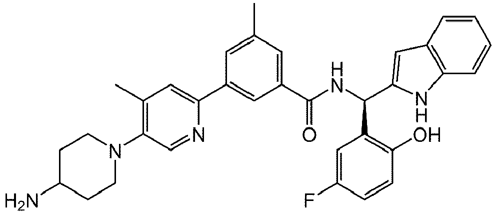

(R)-3-(5-(4-aminopiperidine-1-yl)- 4-methylpyridine-2-yl)-N-((5-fluoro-2-hydroxyphenyl)(1H-indole-2-yl)methyl)-5-methylbenzamide [Example 33] (23%) was prepared by a method similar to the method described in [Example 1].

¹H NMR (400 MHz, DMSO-d6) δ 11.12 (br s, 1H), 9.26 (br d, J = 8.38 Hz, 1H), 8.35 (s, 1H), 8.30 (s, 1H), 8.00 (s, 1H), 7.80 (s, 1H), 7.77 (s, 1H), 7 .43 (d, J = 7.75 Hz, 1H), 7.33 (d, J=8.13 Hz, 1H), 7.23 (dd, J = 3.13, 9.76 Hz, 1H), 7.01-7.08 (m, 1H), 6.90-6.99 (m, 2H), 6.80-6.88 (m, 2H), 6.00 (s, 1H), 3.14 (br d, J = 12.01 Hz, 2H), 2.69-2.79 (m, 3H), 2.44 (s, 3H), 2.32 (s, 3H), 1.83 (br d, J = 10.88 Hz, 2H), 1.35-1.48 (m, 2H); LC-MS: 564.6 [M+H⁺].

### [Example 34]

### (R)-3-(2-(4-aminopiperidine-1-yl)-4-methylpyrimidine-5-yl)-N-((5-fluoro-2-hydroxyphenyl)(1H-indole-2-yl)methyl)-5-methylbenzamide

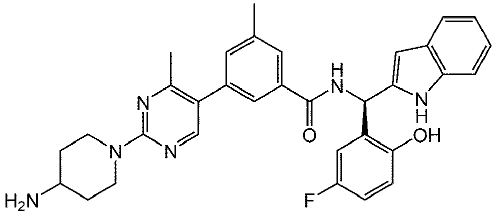

(R)-3-(2-(4-aminopiperidine-1-yl)-4-methylpyrimidine-5-yl)-N-((5-fluoro-2-hydroxyphenyl)(1H-indole-2-yl)methyl)-5-methylbenzamide [Example 34] (13%) was prepared by a method similar to the method described in [Example 1].

¹H NMR (400 MHz, DMSO-*d6*) δ 10.91-11.23 (m, 1H), 9.05-9.28 (m, 1H), 8.20 (s, 1H), 7.65-7.89 (m, 2H), 7.39-7.45 (m, 1H), 7.36 (s, 1H), 7.31 (br d, J = 0.75 Hz, 1H), 7.18 (dd, J = 3.13, 9.76 Hz, 1H), 7.03 (br d, J = 1.00 Hz, 1H), 6.91-6.99 (m, 2H), 6.85 (br d, J = 4.88 Hz, 1H), 6.80 (s, 1H), 5.95-6.04 (m, 1H), 4.51-4.60 (m, 2H), 2.96-3.03 (m, 2H), 2.78-2.88 (m, 1H), 2.37-2.43 (m, 3H), 2.29 (s, 3H), 1.70-1.80 (m, 2H), 1.10-1.20 (m, 2H); LC-MS: 565.6 [M+H⁺].

### [Example 35]

### tert-butyl (R)-4-(2-(3-(((5-fluoro-2-hydroxyphenyl)(1H-indole-2-yl)methyl)carbamoyl)-5-methylphenyl)pyrimidine-5-yl)piperazine-1-carboxylate

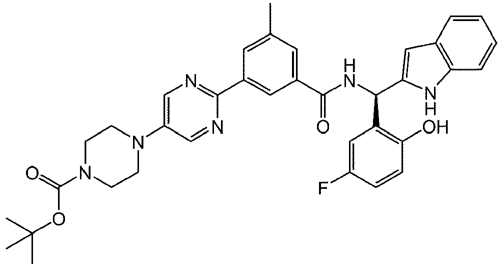

Tert-butyl (R)-4-(2-(3-(((5-fluoro-2-hydroxyphenyl)(1H-indole-2-yl)methyl)carbamoyl)-5-methylphenyl)pyrimidine-5-yl)piperazine-1-carboxylate[Example 35] (82%) was prepared by a method similar to the method described in [Preparative Example 9].

¹H NMR (400 MHz, DMSO-d6) δ 11.08-11.13 (m, 1H), 9.65 (s, 1H), 9.29 (d, J=8.63 Hz, 1H), 8.62 (s, 3H), 8.25 (s, 1H), 7.82 (s, 1H), 7.43 (d, J=8.00 Hz, 1H), 7.33 (d, J=7.67 Hz, 1H), 7.25 (dd, J=3.19, 9.82 Hz, 1H), 7.04 (dt, J=1.13, 7.57 Hz, 1H), 6.92-7.01 (m, 2H), 6.80-6.88 (m, 2H), 6.00 (t, J=1.00 Hz, 1H), 3.46-3.53 (m, 4H), 3.28-3.32 (m, 4H), 2.44 (s, 3H), 1.43 (s, 9H); LC-MS: 637.7 [M+H⁺].

### [Example 36]

### (R)-N-((5-fluoro-2-hydroxyphenyl)(1H-indole-2-yl)methyl)-3-methyl-5-(5-(piperazine-1-yl)pyrimidine-2-yl)benzamide

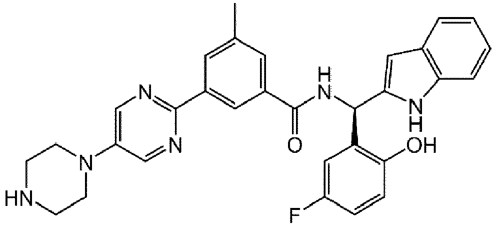

(R)-N-((5-fluoro-2-hydroxyphenyl)(1H-indole-2-yl)methyl)-3-methyl-5-(5-(piperazine-1-yl)pyrimidine-2-yl)benzamide [Example 36] (11%) was prepared by a method similar to the method described in [Example 1].

¹H NMR (400 MHz, DMSO-d6) δ 11.10 (s, 1H), 9.65 (br s, 1H), 9.28 (d, J=8.63 Hz, 1H), 8.55-8.63 (m, 3H), 8.23-8.26 (m, 1H), 7.80 (s, 1H), 7.43 (d, J=7.88 Hz, 1H), 7.33 (dd, J=0.75, 8.13 Hz, 1H), 7.25 (dd, J=3.13, 9.76 Hz, 1H), 7.04 (dt, J=1.19, 7.60 Hz, 1H), 6.91-7.01 (m, 2H), 6.79-6.88 (m, 2H), 6.00 (t, J=1.00 Hz, 1H), 3.19-3.25 (m, 4H), 2.81-2.89 (m, 4H), 2.43-2.46 (m, 3H); LC-MS: 537.7 [M+H⁺].

### [Example 37]

### (R)-N-((5-fluoro-2-hydroxyphenyl)(1H-indole-2-yl)methyl)-3-methyl-5-(5-(4-methylpiperazine-1-yl)pyrimidine-2-yl)benzamide

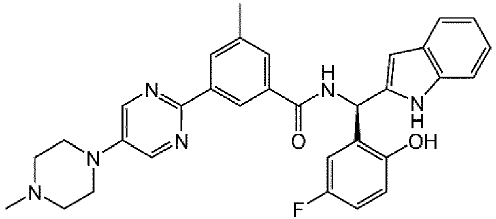

(R)-N-((5-fluoro-2-hydroxyphenyl)(1H-indole-2-yl)methyl)-3-methyl-5-(5-(4-methylpiperazine-1-yl)pyrimidine-2-yl)benzamide [Example 37] (54%) was prepared by a method similar to the method described in [Example 1].

¹H NMR (400 MHz, DMSO-d6) δ 11.11 (s, 1H), 9.67 (s, 1H), 9.28 (d, J=8.63 Hz, 1H), 8.58-8.66 (m, 3H), 8.22-8.26 (m, 1H), 7.82 (s, 1H), 7.43 (d, J=7.88 Hz, 1H), 7.31-7.36 (m, 1H), 7.26 (dd, J=3.13, 9.76 Hz, 1H), 7.02-7.08 (m, 1H), 6.92-7.01 (m, 2H), 6.80-6.89 (m, 2H), 5.99-6.02 (m, 1H), 4.02 (br d, J=12.26 Hz, 2H), 2.78-2.94 (m, 5H), 2.44 (s, 3H), 1.92 (br s, 2H), 1.64 (br d, J=10.13 Hz, 2H), 1.05-1.16 (m, 6H); LC-MS: 551.6 [M+H⁺].

### [Example 38]

### N-[(R)-(5-fluoro-2-hydroxy-phenyl)-(1H-indole-2-yl)methyl]-3-methyl-5-[5-(4-propyl-1-piperidyl)pyrimidine-2-yl]benzamide

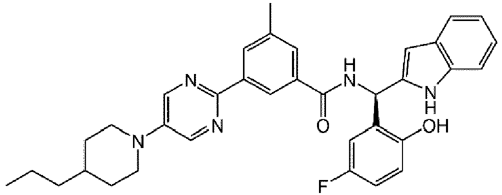

N-[(R)-(5-fluoro-2-hydroxy-phenyl)-(1H-indole-2-yl)methyl]-3-methyl-5-[5-(4-propyl-1-piperidyl)pyrimidine-2-yl]benzamide [Example 38] (18%) was prepared by a method similar to the method described in [Preparative Example 9].

¹H NMR (400 MHz, DMSO-d6) δ 11.07-11.15 (m, 1H), 9.59-9.76 (m, 1H), 9.22-9.31 (m, 1H), 8.60 (s, 1H),8.58 (s, 2H), 8.24 (s, 1H), 7.96 (s, 1H), 7.80 (s, 1H), 7.43 (d, J=7.75 Hz, 1H), 7.32 (s, 1H), 7.25 (dd, J=3.13, 9.76 Hz, 1H), 6.91-7.07 (m, 4H), 6.84-6.88 (m, 1H), 6.83 (s, 1H), 5.98-6.03 (m, 1H), 2.75-2.83 (m,4H), 2.42-2.45 (m, 3H), 1.72-1.80 (m, 2H), 1.39-1.47 (m, 1H), 1.33 (br s, 2H), 1.23 (br t, J=7.44 Hz, 4H),0.91-0.92 (m, 1H), 0.89 (t, J=7.13 Hz, 3H)); LC-MS: 557.7 [M+H⁺].

### [Example 39]

### 3-[5-[4-(dimethylamino)-1-piperidyl]pyrimidine-2-yl]-N-[(R)-(5-fluoro-2-hydroxy-phenyl)-(1H-indole-2-yl)methyl]-5-methyl-benzamide

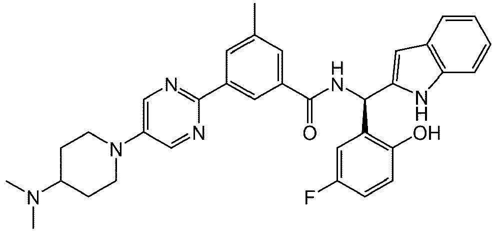

3-[5-[4-(Dimethylamino)-1-piperidyl]pyrimidine-2-yl]-N-[(R)-(5-fluoro-2-hydroxy-phenyl)-(1H-indole-2-yl)methyl]-5-methyl-benzamide[Example 39] (7%) was prepared by a method similar to the method described in [Preparative Example 9].

¹H NMR (400 MHz, CHLOROFORM-d) δ 9.38 (br s, 1H), 8.61 (br d, J=8.00 Hz, 1H), 8.44 (s, 1H),8.12-8.19 (m, 3H), 7.66 (s, 1H), 7.52 (d, J=7.75 Hz, 1H), 7.26-7.31 (m, 2H), 7.12 (t, J=7.16 Hz, 1H),7.04-7.10 (m, 1H), 6.97 (dd, J=2.81, 8.82 Hz, 1H), 6.81-6.92 (m, 2H), 6.70 (br d, J=7.13 Hz, 1H), 6.34 (s,1H), 3.62 (br d, J=12.63 Hz, 1H), 3.45-3.54 (m, 2H), 2.60-2.74 (m, 2H), 2.35 (s, 3H), 2.33 (s, 6H), 2.26(ddd, J=4.00, 7.72, 14.66 Hz, 1H), 1.88 (br d, J=12.13 Hz, 1H), 1.78 (br d, J=12.63 Hz, 1H), 1.52-1.65 (m,1H), 1.39-1.50 (m, 1H), 1.28 (br s, 1H) ; LC-MS: 578.7 [M+H⁺].

### [Example 40]

### N-[(R)-(5-fluoro-2-hydroxy-phenyl)-(1H-indole-2-yl)methyl]-3-methyl-5-[5-(1-piperidyl)pyrimidine-2-yl]benzamide

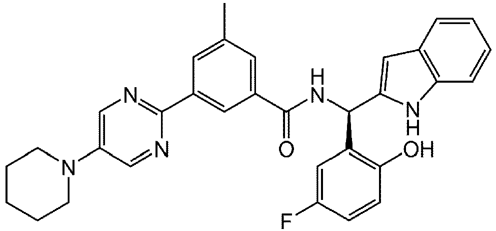

N-[(R)-(5-fluoro-2-hydroxy-phenyl)-(1H-indole-2-yl)methyl]-3-methyl-5-[5-(1-piperidyl)pyrimidine-2-yl]benzamide [Example 40] (24%) was prepared by a method similar to the method described in [Preparative Example 9].

¹H NMR (400 MHz, DMSO-d6) δ 11.10 (s, 1H), 9.65 (s, 1H), 9.28 (d, J=8.63 Hz, 1H), 8.56-8.63 (m, 3H), 8.24 (s, 1H), 7.80 (s, 1H), 7.43 (d, J=7.75 Hz, 1H), 7.34 (d, J=8.00 Hz, 1H), 7.26 (dd, J=2.94, 9.69 Hz, 1H), 7.01-7.08 (m, 1H), 6.90-7.00 (m, 2H), 6.80-6.89 (m, 2H), 6.00 (s, 1H), 2.44 (s, 3H), 1.40-1.81 (m, 7H); LC-MS: 536.61 [M+H⁺].

### [Example 41]

### N-[(R)-(5-fluoro-2-hydroxy-phenyl)-(1H-indole-2-yl)methyl]-3-methyl-5-[5-[4-(1-methyl-4-piperidyl)piperazine-1-yl]pyrimidine-2-yl]benzamide

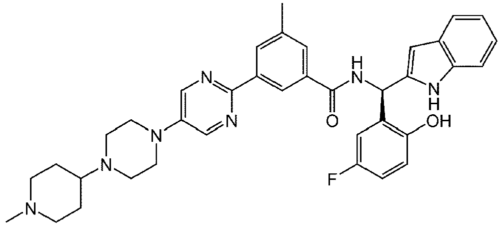

N-[(R)-(5-fluoro-2-hydroxy-phenyl)-(1H-indole-2-yl)methyl]-3-methyl-5-[5-[4-(1-methyl-4-piperidyl)piperazine-1-yl]pyrimidine-2-yl]benzamide[Example 41] (10%) was prepared by a method similar to the method described in [Preparative Example 9].

¹H NMR (400 MHz, DMSO-d6) δ 11.11 (s, 1H), 9.68 (br s, 1H), 9.28 (d, J=8.51 Hz, 1H), 8.56-8.64 (m, 3H), 8.24 (s, 1H), 7.81 (s, 1H), 7.43 (d, J=7.75 Hz, 1H), 7.33 (d, J=8.13 Hz, 1H), 7.25 (dd, J=3.13, 9.76 Hz, 1H), 7.01-7.07 (m, 1H), 6.91-7.00 (m, 2H), 6.79-6.88 (m, 2H), 6.00 (s, 1H), 3.29 (br s, 2H), 3.08 (s, 2H), 2.79 (br d, J=11.51 Hz, 2H), 2.67 (br dd, J=1.88, 3.63 Hz, 1H), 2.62-2.66 (m, 3H), 2.44 (s, 3H), 2.17-2.23 (m, 1H), 2.14 (s, 3H), 1.80-1.90 (m, 2H), 1.74 (br d, J=11.51 Hz, 2H), 1.37-1.50 (m, 2H); LC-MS: 634.76 [M+H⁺].

### [Example 42]

### (R)-3-(5-((4-(dimethylamino)piperidine-1-yl)methyl)pyridine-2-yl)-N-((5-fluoro-2-hydroxyphenyl)(1H-indole-2-yl))methyl)-5-methylbenzamide

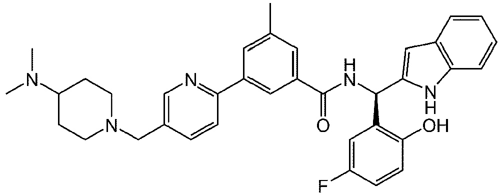

(R)-3-(5-((4-(dimethylamino)piperidine-1-yl)methyl)pyridine-2-yl)-N-((5-fluoro-2-hydroxyphenyl)(1H-indole-2-yl))methyl)-5-methylbenzamide [Example 42] (84%) was prepared by a method similar to the method described in [Preparative Example 9].

¹H NMR (400 MHz, DMSO-d6) δ 11.12 (s, 1H), 9.66 (br s, 1H), 9.29 (d, J=8.51 Hz, 1H), 8.54-8.60 (m, 1H), 8.42 (s, 1H), 8.07 (s, 1H), 7.94-8.05 (m, 1H), 7.78-7.86 (m, 2H), 7.43 (d, J=7.75 Hz, 1H), 7.33 (d, J=8.00 Hz, 1H), 7.23 (dd, J=3.13, 9.63 Hz, 1H), 7.01-7.08 (m, 1H), 6.91-7.01 (m, 2H), 6.81-6.91 (m,2H), 6.00 (s, 1H), 3.47-3.54 (m, 2H), 2.83 (br d, J=11.38 Hz, 2H), 2.43-2.48 (m, 3H), 2.16 (s, 6H), 2.00-2.10 (m, 1H), 1.96 (br t, J=10.82 Hz, 2H), 1.71 (br d, J=12.01 Hz, 2H), 1.30-1.47 (m, 2H); LC-MS: 592.7 [M+H+].

### [Example 43]

### (R)-3-(6-((4-(dimethylamino)piperidine-1-yl)methyl)pyridine-3-yl)-N-((5-fluoro-2-hydroxyphenyl)(1H-indole-2-yl))methyl)-5-methylbenzamide

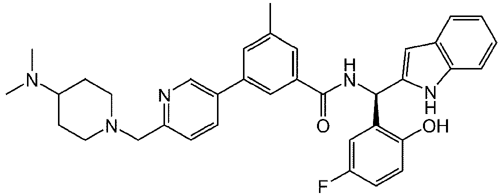

(R)-3-(6-((4-(dimethylamino)piperidine-1-yl)methyl)pyridine-3-yl)-N-((5-fluoro-2-hydroxyphenyl)(1H-indole-2-yl))methyl)-5-methylbenzamide [Example 43] (90%) was prepared by a method similar to the method described in [Preparative Example 9].

¹H NMR (400 MHz, DMSO-d6) δ 11.12 (s, 1H), 9.66 (br s, 1H), 9.29 (d, J=8.51 Hz, 1H), 8.54-8.60 (m, 1H), 8.42 (s, 1H), 8.07 (s, 1H), 7.94-8.05 (m, 1H), 7.78-7.86 (m, 2H), 7.43 (d, J=7.75 Hz, 1H), 7.33 (d, J=8.00 Hz, 1H), 7.23 (dd, J=3.13, 9.63 Hz, 1H), 7.01-7.08 (m, 1H), 6.91-7.01 (m, 2H), 6.81-6.91 (m,2H), 6.00 (s, 1H), 3.47-3.54 (m, 2H), 2.83 (br d, J=11.38 Hz, 2H), 2.43-2.48 (m, 3H), 2.16 (s, 6H), 2.00-2.10 (m, 1H), 1.96 (br t, J=10.82 Hz, 2H), 1.71 (br d, J=12.01 Hz, 2H), 1.30-1.47 (m, 2H); LC-MS: 592.7 [M+H+].

### [Example 44]

### 3-[5-[(3R)-3-aminopyrrolidine-1-yl]pyrimidine-2-yl]-N-[(R)-(5-fluoro-2-hydroxy-phenyl)-(1H-indole-2-yl)methyl]-5-methyl-benzamide

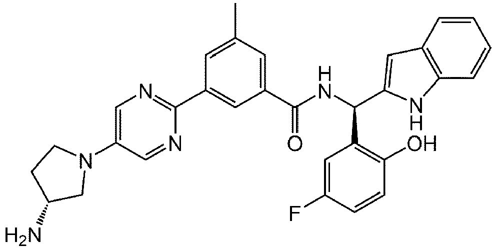

3-[5-[(3R)-3-aminopyrrolidine-1-yl]pyrimidine-2-yl]-N-[(R)-(5-fluoro-2-hydroxy-phenyl)-(1H-indole-2-yl)methyl]-5-methyl-benzamide [Example 44] (86%) was prepared by a method similar to the method described in [Example 1].

¹H NMR (400 MHz, DMSO-d6) δ 11.14 (br s, 1H), 9.31 (br d, J=8.13 Hz, 1H), 8.58 (s, 1H), 8.17-8.24 (m, 3H), 7.76 (s, 1H), 7.43 (d, J=7.75 Hz, 1H), 7.34 (d, J=8.00 Hz, 1H), 7.25 (dd, J=2.69, 9.69 Hz, 1H), 7.01-7.08 (m, 1H), 6.91-7.00 (m, 2H), 6.78-6.87 (m, 2H), 6.02 (s, 1H), 3.58-3.64 (m, 1H), 3.45-3.53 (m, 3H), 3.00 (br dd, J=4.25, 9.63 Hz, 1H), 2.43 (s, 3H), 2.03-2.13 (m, 1H), 1.74 (qd, J=6.05, 12.12 Hz, 1H); LC-MS: 537.6 [M+H+].

### [Example 45]

### (R)-3-(5-(4-(diethylamino)piperidine-1-yl)pyrimidine-2-yl)-N-((5-fluoro-2-hydroxyphenyl)(1H-indole-2-yl)methyl)-5-methylbenzamide

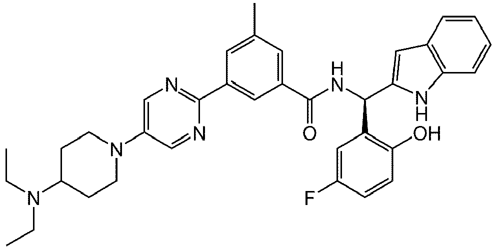

(R)-3-(5-(4-(diethylamino)piperidine-1-yl)pyrimidine-2-yl)-N-((5-fluoro-2-hydroxyphenyl)(1H-indole-2-yl)methyl)-5-methylbenzamide [Example 45] (96%) was prepared by a method similar to the method described in [Preparative Example 9].

¹H NMR (400 MHz, DMSO-d6) δ 11.11 (s, 1H), 9.67 (s, 1H), 9.28 (d, J=8.63 Hz, 1H), 8.58-8.66 (m, 3H), 8.22-8.26 (m, 1H), 7.82 (s, 1H), 7.43 (d, J=7.88 Hz, 1H), 7.31-7.36 (m, 1H), 7.26 (dd, J=3.13, 9.76 Hz, 1H), 7.02-7.08 (m, 1H), 6.92-7.01 (m, 2H), 6.80-6.89 (m, 2H), 5.99-6.02 (m, 1H), 4.02 (br d, J=12.26 Hz, 2H), 2.78-2.94 (m, 5H), 2.44 (s, 3H), 1.92 (br s, 2H), 1.64 (br d, J=10.13 Hz, 2H), 1.05-1.16 (m, 6H); LC-MS: 607.7 [M+H+].

### [Example 46]

### N-((R)-(5-fluoro-2-hydroxyphenyl)(1H-indole-2-yl)methyl)-3-methyl-5-(5-(3-methyl-3,8-diazabicyclo[3.2.1]octane-8-yl)pyrimidine-2-yl)benzamide

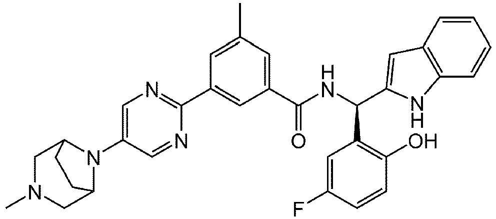

N-((R)-(5-fluoro-2-hydroxyphenyl)(1H-indole-2-yl)methyl)-3-methyl-5-(5-(3-methyl-3,8-diazabicyclo[3.2.1]octane-8-yl)pyrimidine-2-yl)benzamide [Example 46] (91%) was prepared by a method similar to the method described in [Preparative Example 9].

¹H NMR (400 MHz, DMSO-d6) δ 11.10 (s, 1H), 9.65 (s, 1H), 9.26 (d, J=8.63 Hz, 1H), 8.66-8.67 (m, 1H), 8.65-8.66 (m, 1H), 8.58 (s, 1H), 8.44-8.45 (m, 1H), 8.42-8.43 (m, 1H), 8.38 (s, 2H), 8.19-8.24 (m, 1H), 7.78 (s, 1H), 7.41-7.45 (m, 1H), 7.33 (dd, J=0.75, 8.00 Hz, 1H), 7.26 (dd, J=3.19, 9.82 Hz, 1H), 7.04 (dt, J=1.13, 7.57 Hz, 1H), 6.92-7.01 (m, 2H), 6.79-6.88 (m, 2H), 5.99-6.02 (m, 1H), 3.35-3.43 (m, 3H), 3.07 (br s, 2H), 2.53 (br s, 6H), 2.44 (s, 3H), 2.09 (br s, 1H), 1.90 (br d, J=11.63 Hz, 1H), 1.75-1.86 (m, 1H), 1.72 (br d, J=11.51 Hz, 1H); LC-MS: 577.6 [M+H+].

### [Example 47]

### (R)-3-(5-(4-(1H-pyrrole-1-yl)piperidine-1-yl)pyrimidine-2-yl)-N-((5-fluoro-2-hydroxyphenyl)(1H-indole-2-yl)methyl)-5-methylbenzamide

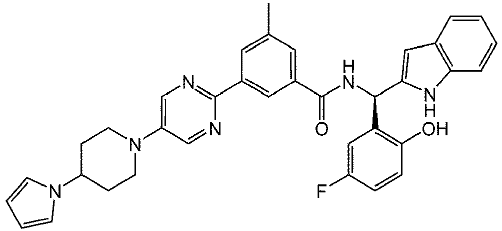

(R)-3-(5-(4-(1H-pyrrole-1-yl)piperidine-1-yl)pyrimidine-2-yl)-N-((5-fluoro-2-hydroxyphenyl)(1H-indole-2-yl)methyl)-5-methylbenzamide [Example 47] (86%) was prepared by a method similar to the method described in [Preparative Example 9].

¹H NMR (400 MHz, DMSO-d6) δ 11.10 (s, 1H), 9.65 (s, 1H), 9.26 (d, J=8.63 Hz, 1H), 8.66-8.67 (m, 1H), 8.65-8.66 (m, 1H), 8.58 (s, 1H), 8.44-8.45 (m, 1H), 8.42-8.43 (m, 1H), 8.38 (s, 2H), 8.19-8.24 (m, 1H), 7.78 (s, 1H), 7.41-7.45 (m, 1H), 7.33 (dd, J=0.75, 8.00 Hz, 1H), 7.26 (dd, J=3.19, 9.82 Hz, 1H), 7.04 (dt, J=1.13, 7.57 Hz, 1H), 6.92-7.01 (m, 2H), 6.79-6.88 (m, 2H), 5.99-6.02 (m, 1H), 3.35-3.43 (m, 3H), 3.07 (br s, 2H), 2.53 (br s, 6H), 2.44 (s, 3H), 2.09 (br s, 1H), 1.90 (br d, J=11.63 Hz, 1H), 1.75-1.86 (m, 1H), 1.72 (br d, J=11.51 Hz, 1H); LC-MS: 601.6 [M+H+].

### [Example 48]

### (R)-3-(5-(4-(1H-1,2,4-triazole-1-yl)piperidine-1-yl)pyrimidine-2-yl)-N-((5-fluoro-2-hydroxyphenyl)(1H-indole-2-yl)methyl)-5-methylbenzamide

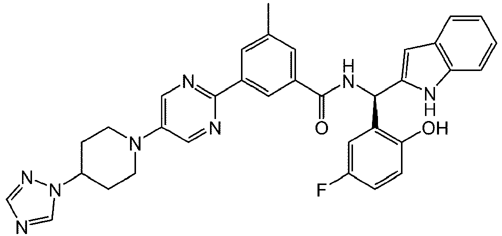

(R)-3-(5-(4-(1H-1,2,4-triazole-1-yl)piperidine-1-yl)pyrimidine-2-yl)-N-((5-fluoro-2-hydroxyphenyl)(1H-indole-2-yl)methyl)-5-methylbenzamide [Example 48] (78%) was prepared by a method similar to the method described in [Preparative Example 9].

¹H NMR (400 MHz, DMSO-d6) δ 11.10 (s, 1H), 9.65 (s, 1H), 9.26 (d, J=8.63 Hz, 1H), 8.66-8.67 (m, 1H), 8.65-8.66 (m, 1H), 8.58 (s, 1H), 8.44-8.45 (m, 1H), 8.42-8.43 (m, 1H), 8.38 (s, 2H), 8.19-8.24 (m, 1H), 7.78 (s, 1H), 7.41-7.45 (m, 1H), 7.33 (dd, J=0.75, 8.00 Hz, 1H), 7.26 (dd, J=3.19, 9.82 Hz, 1H), 7.04 (dt, J=1.13, 7.57 Hz, 1H), 6.92-7.01 (m, 2H), 6.79-6.88 (m, 2H), 5.99-6.02 (m, 1H), 3.35-3.43 (m, 3H), 3.07 (br s, 2H), 2.53 (br s, 6H), 2.44 (s, 3H), 2.09 (br s, 1H), 1.90 (br d, J=11.63 Hz, 1H), 1.75-1.86 (m, 1H), 1.72 (br d, J=11.51 Hz, 1H); LC-MS: 603.6 [M+H+].

### [Example 49]

### (R)-N-((5-fluoro-2-hydroxyphenyl)(1H-indole-2-yl)methyl)-3-(5-(4-hydroxypiperidine-1-yl)pyrimidine-2-yl)-5-methylbenzamide

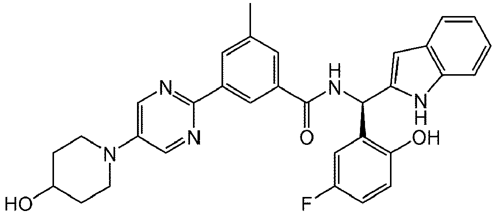

(R)-N-((5-fluoro-2-hydroxyphenyl)(1H-indole-2-yl)methyl)-3-(5-(4-hydroxypiperidine-1-yl)pyrimidine-2-yl)-5-methylbenzamide [Example 49] (15%) was prepared by a method similar to the method described in [Preparative Example 9].

¹H NMR (400 MHz, DMSO-d6) δ 11.10 (s, 1H), 9.65 (s, 1H), 9.26 (d, J=8.63 Hz, 1H), 8.66-8.67 (m, 1H), 8.65-8.66 (m, 1H), 8.58 (s, 1H), 8.44-8.45 (m, 1H), 8.42-8.43 (m, 1H), 8.38 (s, 2H), 8.19-8.24 (m, 1H), 7.78 (s, 1H), 7.41-7.45 (m, 1H), 7.33 (dd, J=0.75, 8.00 Hz, 1H), 7.26 (dd, J=3.19, 9.82 Hz, 1H), 7.04 (dt, J=1.13, 7.57 Hz, 1H), 6.92-7.01 (m, 2H), 6.79-6.88 (m, 2H), 5.99-6.02 (m, 1H), 3.35-3.43 (m, 3H), 3.07 (br s, 2H), 2.53 (br s, 6H), 2.44 (s, 3H), 2.09 (br s, 1H), 1.90 (br d, J=11.63 Hz, 1H), 1.75-1.86 (m, 1H), 1.72 (br d, J=11.51 Hz, 1H); LC-MS: 552.6 [M+H+].

### [Example 50]

### (R)-3-(5-([1,4'-bipiperidine]-1'-yl)pyrimidine-2-yl)-N-((5-fluoro-2-hydroxyphenyl)(1H-indole-2-yl)methyl)-5-methylbenzamide

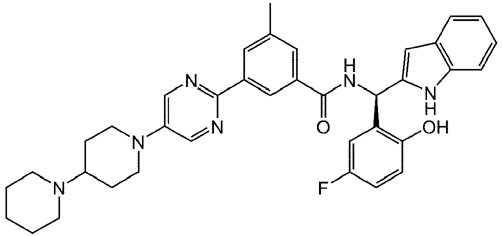

(R)-3-(5-([1,4'-bipiperidine]-1'-yl)pyrimidine-2-yl)-N-((5-fluoro-2-hydroxyphenyl)(1H-indole-2-yl)methyl)-5-methylbenzamide [Example 50] (26%) was prepared by a method similar to the method described in [Preparative Example 9].

¹H NMR (400 MHz, DMSO-d6) δ 11.11 (br s, 1H), 9.66 (br s, 1H), 9.29 (br d, J=8.38 Hz, 1H), 8.56-8.63 (m, 3H), 8.23-8.25 (m, 1H), 7.80 (s, 1H), 7.43 (d, J=7.75 Hz, 1H), 7.33 (dd, J=0.69, 8.07 Hz, 1H), 7.25 (dd, J=3.19, 9.82 Hz, 1H), 7.04 (dt, J=1.13, 7.57 Hz, 1H), 6.92-7.01 (m, 2H), 6.79-6.87 (m, 2H), 6.00 (s, 1H), 3.94 (br d, J=12.76 Hz, 2H), 2.74-2.85 (m, 2H), 2.47 (br d, J=2.75 Hz, 4H), 2.44 (s, 3H), 2.37-2.42 (m, 1H), 1.82 (br d, J=11.38 Hz, 2H), 1.51-1.60 (m, 2H), 1.48 (br d, J=4.88 Hz, 4H), 1.38 (br d, J=5.13 Hz, 2H); LC-MS: 619.7 [M+H+].

### [Example 51]

### (R)-N-((5-fluoro-2-hydroxyphenyl)(1H-indole-2-yl)methyl)-3-methyl-5-(5-(4-(morpholinomethyl)piperidine-1-yl)pyrimidine-2-yl)benzamide

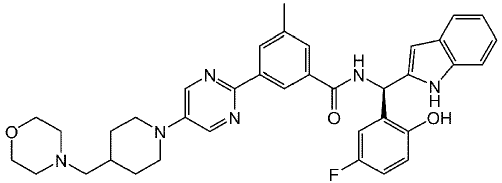

(R)-N-((5-fluoro-2-hydroxyphenyl)(1H-indole-2-yl)methyl)-3-methyl-5-(5-(4-(morpholinomethyl)piperidine-1-yl)pyrimidine-2-yl)benzamide [Example 51] (100%) was prepared by a method similar to the method described in [Preparative Example 9].

¹H NMR (400 MHz, DMSO-d6) δ 11.11 (br s, 1H), 9.66 (br s, 1H), 9.29 (br d, J=8.38 Hz, 1H), 8.56-8.63 (m, 3H), 8.23-8.25 (m, 1H), 7.80 (s, 1H), 7.43 (d, J=7.75 Hz, 1H), 7.33 (dd, J=0.69, 8.07 Hz, 1H), 7.25 (dd, J=3.19, 9.82 Hz, 1H), 7.04 (dt, J=1.13, 7.57 Hz, 1H), 6.92-7.01 (m, 2H), 6.79-6.87 (m, 2H), 6.00 (s, 1H), 3.94 (br d, J=12.76 Hz, 2H), 2.74-2.85 (m, 2H), 2.47 (br d, J=2.75 Hz, 4H), 2.44 (s, 3H), 2.37-2.42 (m, 1H), 1.82 (br d, J=11.38 Hz, 2H), 1.51-1.60 (m, 2H), 1.48 (br d, J=4.88 Hz, 4H), 1.38 (br d, J=5.13 Hz, 2H) ; LC-MS: 635.7 [M+H+].

### [Example 52]

### (R)-N-((5-fluoro-2-hydroxyphenyl)(1H-indole-2-yl)methyl)-3-methyl-5-(5-(4-(pyrrolidine-1-ylmethyl)piperidine-1-yl)pyrimidine-2-yl)benzamide

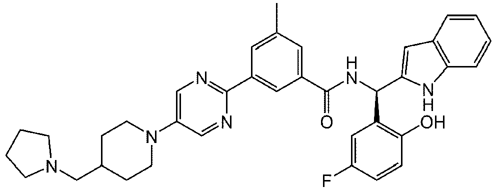

(R)-N-((5-fluoro-2-hydroxyphenyl)(1H-indole-2-yl)methyl)-3-methyl-5-(5-(4-(pyrrolidine-1-ylmethyl)piperidine-1-yl)pyrimidine-2-yl)benzamide [Example 52] (11%) was prepared by a method similar to the method described in [Preparative Example 9].

¹H NMR (400 MHz, DMSO-d6) δ 11.11 (br s, 1H), 9.66 (br s, 1H), 9.29 (br d, J=8.38 Hz, 1H), 8.56-8.63 (m, 3H), 8.23-8.25 (m, 1H), 7.80 (s, 1H), 7.43 (d, J=7.75 Hz, 1H), 7.33 (dd, J=0.69, 8.07 Hz, 1H), 7.25 (dd, J=3.19, 9.82 Hz, 1H), 7.04 (dt, J=1.13, 7.57 Hz, 1H), 6.92-7.01 (m, 2H), 6.79-6.87 (m, 2H), 6.00 (s,1H), 3.94 (br d, J=12.76 Hz, 2H), 2.74-2.85 (m, 2H), 2.47 (br d, J=2.75 Hz, 4H), 2.44 (s, 3H), 2.37-2.42 (m, 1H), 1.82 (br d, J=11.38 Hz, 2H), 1.51-1.60 (m,2H), 1.48 (br d, J=4.88 Hz, 4H), 1.38 (br d, J=5.13 Hz, 2H); LC-MS: 619.7 [M+H+].

### [Example 53]

### 3-[5-[(3R)-3-(dimethylamino)pyrrolidine-1-yl]pyrimidine-2-yl]-N-[(R)-(5-fluoro-2-hydroxyphenyl)-(1H-indole-2-yl)methyl]-5-methyl-benzamide

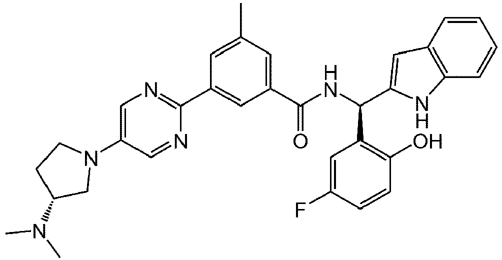

3-[5-[(3R)-3-(dimethylamino)pyrrolidine-1-yl]pyrimidine-2-yl]-N-[(R)-(5-fluoro-2-hydroxyphenyl)-(1H-indole-2-yl)methyl]-5-methyl-benzamide [Example 53] (33%) was prepared by a method similar to the method described in [Preparative Example 9].

¹H NMR (400 MHz, DMSO-d6) δ 11.11 (s, 1H), 9.67 (br d, J=0.75 Hz, 1H), 9.28 (d, J=8.63 Hz, 1H), 8.58 (s, 1H), 8.21-8.26 (m, 3H), 7.76 (d, J=0.63 Hz, 1H), 7.43 (d, J=7.88 Hz, 1H), 7.34 (dd, J=0.75, 8.13 Hz, 1H), 7.26 (dd, J=3.19, 9.82 Hz, 1H), 7.04 (ddd, J=1.19, 7.00, 8.07 Hz, 1H), 6.89-6.99 (m, 2H), 6.78-6.89 (m, 2H), 6.01 (s, 1H), 3.58 (dd, J=7.19, 9.57 Hz, 1H), 3.48-3.55 (m, 1H), 3.34-3.39 (m, 1H), 3.10-3.17 (m, 1H), 3.08 (s, 1H), 2.78-2.88 (m, 1H), 2.44 (s, 3H), 2.22 (s, 6H), 2.14-2.20 (m, 1H), 1.83 (qd, J=9.32, 12.07 Hz, 1H); LC-MS: 565.65 [M+H+].

### [Example 54]

### (R)-N-((5-fluoro-2-hydroxyphenyl)(1H-indole-2-yl)methyl)-3-methyl-5-(5-(4-(methylamino)piperidine-1-yl)pyrimidine-2-yl)benzamide

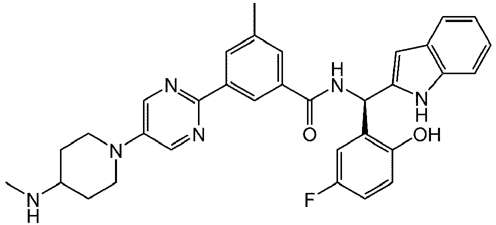

(R)-N-((5-fluoro-2-hydroxyphenyl) (1H-indole-2-yl)methyl)-3-methyl-5-(5-(4-(methylamino)piperidine-1-yl)pyrimidine-2-yl)benzamide [Example 54] (10%) was prepared by a method similar to the method described in [Example 1].

¹H NMR (400 MHz, DMSO-d6)δ 11.10 (s, 1H), 9.61-9.73 (m, 1H), 9.21-9.39 (m, 1H), 8.64 (s, 3H), 8.23-8.26 (m, 1H), 7.83 (s, 1H), 7.41-7.45 (m, 1H), 7.31-7.35 (m, 1H), 7.22-7.28 (m, 1H), 7.02-7.07 (m, 1H), 6.92-7.00 (m, 2H), 6.80-6.88 (m, 2H), 5.98-6.03 (m, 1H), 4.01 (br d, J=12.88 Hz, 1H), 3.98-4.05 (m, 1H), 3.17 (d, J=5.25 Hz, 1H), 2.90 (br s, 2H), 2.58-2.59 (m, 2H), 2.45 (s, 4H), 2.32-2.35 (m, 2H), 2.03-2.10 (m, 3H), 1.53-1.62 (m, 2H); LC-MS: 565.6 [M+H+].

### [Example 55]

### (R)-1-(2-(3-(((5-fluoro-2-hydroxyphenyl)(1H-indole-2-yl)methyl)carbamoyl)-5-methylphenyl)pyrimidine-5-yl)-N-phenylpiperidine-4-carboxamide

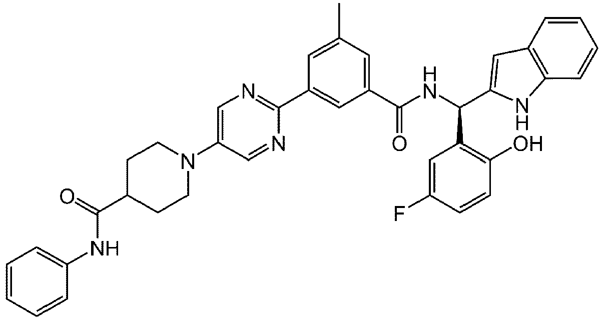

(R)-1-(2-(3-(((5-fluoro-2-hydroxyphenyl)(1H-indole-2-yl)methyl)carbamoyl)-5-methylphenyl)pyrimidine-5-yl)-N-phenylpiperidine-4-carboxamide [Example 55] (10%) was prepared by a method similar to the method described in [Preparative Example 9].

¹H NMR (400 MHz, DMSO-d6) δ 11.11 (d, J=1.63 Hz, 1H), 9.95 (s, 1H), 9.67 (s, 1H), 9.29 (d, J=8.63 Hz, 1H), 8.58-8.66 (m, 3H), 8.26 (d, J=0.63 Hz, 1H), 7.96 (s, 1H), 7.81 (s, 1H), 7.58-7.65 (m, 2H), 7.43 (d, J=7.88 Hz, 1H), 7.23-7.37 (m, 4H), 6.91-7.08 (m, 4H), 6.77-6.89 (m, 2H), 5.96-6.04 (m, 1H), 3.96-4.04 (m, 2H), 3.18 (d, J=5.00 Hz, 1H), 2.89 (s, 2H), 2.45 (s, 3H), 1.92 (br s, 2H), 1.71-1.83 (m, 2H) ; LC-MS: 656.7 [M+H+].

### [Example 56]

### (R)-N-((5-fluoro-2-hydroxyphenyl)(1H-indole-2-yl)methyl)-3-(5-(4-(4-(2-methoxyethoxy)phenyl)piperazine-1-yl)pyrimidine-2-yl)-5-methylbenzamide

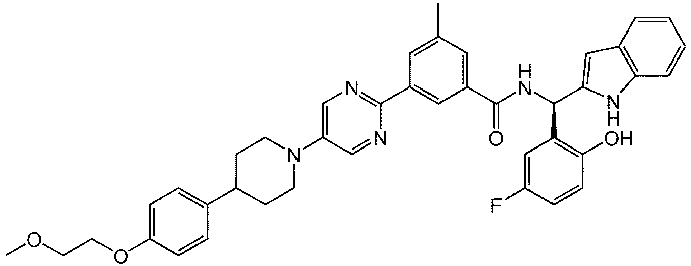

(R)-N-((5-fluoro-2-hydroxyphenyl)(1H-indole-2-yl)methyl)-3-(5-(4-(4-(2-methoxyethoxy)phenyl)piperazine-1-yl)pyrimidine-2-yl)-5-methylbenzamide [Example 56] (10%) was prepared by a method similar to the method described in [Preparative Example 9].

¹H NMR (400 MHz, DMSO-d6) δ 11.11 (d, J=1.63 Hz, 1H), 9.67 (s, 1H), 9.30 (d, J=8.50 Hz, 1H), 8.62-8.68 (m, 3H), 8.27 (d, J=0.63 Hz, 1H), 7.83 (s, 1H), 7.44 (d, J=7.88 Hz, 1H), 7.34 (dd, J=0.81, 8.07 Hz, 1H), 7.27 (dd, J=3.19, 9.82 Hz, 1H), 7.04 (ddd, J=1.19, 7.10, 8.10 Hz, 1H), 6.95-6.99 (m, 3H), 6.94-6.95 (m, 1H), 6.81-6.89 (m, 4H), 5.99-6.03 (m, 1H), 4.00-4.07 (m, 1H), 4.03 (q, J=7.05 Hz, 5H), 3.60-3.66 (m, 2H), 3.45-3.51 (m, 4H), 3.30 (s, 3H), 3.19 (br d, J=4.88 Hz, 4H), 2.87-2.91 (m, 1H), 2.45 (s, 3H), 1.99 (s, 6H), 1.18 (t, J=7.13 Hz, 6H) ; LC-MS: 687.7 [M+H+].

### [Example 57]

### (R)-N-((5-fluoro-2-hydroxyphenyl) (1H-indole-2-yl)methyl)-3-methyl-5-(5-(1,2,3,6-tetrahydropyridine-4-yl)pyrimidine-2-yl)benzamide

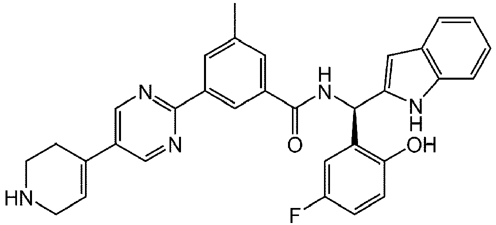

(R)-N-((5-fluoro-2-hydroxyphenyl)(1H-indole-2-yl)methyl)-3-methyl-5-(5-(1,2,3,6-tetrahydropyridine-4-yl)pyrimidine-2-yl)benzamide [Example 57] (81%) was prepared by a method similar to the method described in [Example 1].

¹H NMR (400 MHz, DMSO-d6) δ 11.13 (s, 1H), 9.35 (d, J=8.51 Hz, 1H), 8.94-9.07 (m, 2H), 8.74 (s, 1H), 8.37 (s, 1H), 7.92 (s, 1H), 7.43 (d, J=7.88 Hz, 1H), 7.34 (d, J=8.00 Hz, 1H), 7.25 (dd, J=3.13, 9.76 Hz, 1H), 6.92-7.07 (m, 3H), 6.80-6.88 (m, 2H), 6.53 (br s, 1H), 6.01 (s, 1H), 3.40-3.44 (m, 2H), 2.95 (t, J=5.50 Hz, 2H), 2.46-2.48 (m, 3H), 2.38-2.44 (m, 2H); LC-MS: 534.6 [M+H+].

### [Example 58]

### (R)-N-((5-fluoro-2-hydroxyphenyl) (1H-indole-2-yl)methyl)-3-methyl-5-(5-(piperidine-4-yl)pyrimidine-2-yl)benzamide

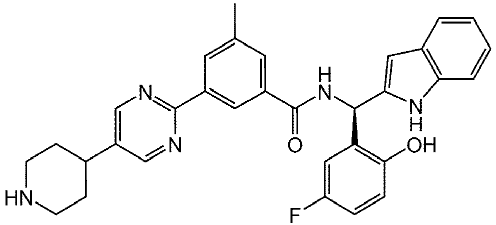

(R)-N-((5-fluoro-2-hydroxyphenyl) (1H-indole-2-yl)methyl)-3-methyl-5-(5-(1,2,3,6-tetrahydropyridine-4-yl)pyrimidine-2-yl)benzamide [Example 57] (150 mg, 0.28 mmol) and 10% palladium/carbon (15 mg, 0.14 mmol) were stirred in methanol and dichloromethane (1:1) under hydrogen at room temperature for 24 hours. The reaction solution was filtered with celite, and then the filtrate was concentrated. The residue was purified by MPLC to give (R)-N-((5-fluoro-2-hydroxyphenyl) (1H-indole-2-yl)methyl)-3-methyl-5-(5-(piperidine-4-yl)pyrimidine-2-yl)benzamide [Example 58](70 mg, 46%) as a white solid.

¹H NMR (400 MHz, DMSO-d6) δ 11.13 (s, 1H), 9.35 (d, J=8.51 Hz, 1H), 8.94-9.07 (m, 2H), 8.74 (s, 1H), 8.37 (s, 1H), 7.92 (s, 1H), 7.43 (d, J=7.88 Hz, 1H), 7.34 (d, J=8.00 Hz, 1H), 7.25 (dd, J=3.13, 9.76 Hz, 1H), 6.92-7.07 (m, 3H), 6.80-6.88 (m, 2H), 6.53 (br s, 1H), 6.01 (s, 1H), 3.40-3.44 (m, 2H), 2.95 (t, J=5.50 Hz, 2H), 2.46-2.48 (m, 3H), 2.38-2.44 (m, 2H); LC-MS: 536.6 [M+H+].

### [Example 59]

### 3-[5-(3-aminoazetidine-1-yl)pyrimidine-2-yl]-N-[(R)-(5-fluoro-2-hydroxy-phenyl)-(1H-indole-2-yl)methyl]-5-methyl-benzamide

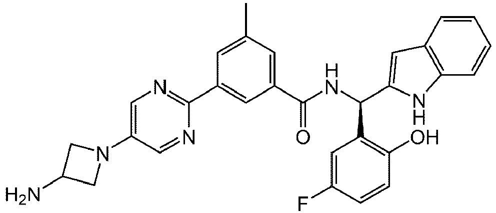

3-[5-(3-Aminoazetidine-1-yl)pyrimidine-2-yl]-N-[(R)-(5-fluoro-2-hydroxy-phenyl)-(1H-indole-2-yl)methyl]-5-methyl-benzamide [Example 59] (54%) was prepared by a method similar to the method described in [Example 1].

¹H NMR (400 MHz, DMSO-d6) δ 11.10 (s, 1H), 8.58 (s, 1H), 8.17-8.25 (m, 1H), 8.13 (s, 2H), 7.79 (s, 1H), 7.43 (d, J=7.88 Hz, 1H), 7.33 (dd, J=0.75, 8.13 Hz, 1H), 7.25 (dd, J=3.13, 9.76 Hz, 1H), 6.91-7.07 (m, 3H), 6.79-6.88 (m, 2H), 5.99-6.02 (m, 1H), 4.20 (t, J=7.44 Hz, 2H), 3.89 (quin, J=6.50 Hz, 1H), 3.54-3.61 (m, 2H), 2.43 (s, 3H)); LC-MS: 523.58 [M+H+].

### [Example 60]

### 3-[5-[(3S)-3-(dimethylamino)pyrrolidine-1-yl]pyrimidine-2-yl]-N-[(R)-(5-fluoro-2-hydroxyphenyl)-(1H-indole-2-yl)methyl]-5-methyl-benzamide

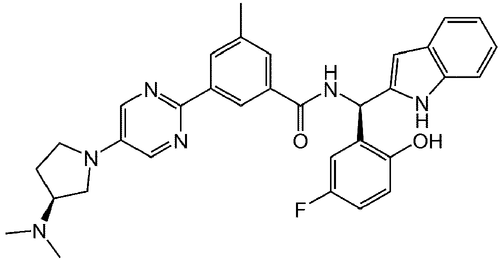

3-[5-[(3S)-3-(dimethylamino)pyrrolidine-1-yl]pyrimidine-2-yl]-N-[(R)-(5-fluoro-2-hydroxyphenyl)-(1H-indole-2-yl)methyl]-5-methyl-benzamide [Example 60] (28%) was prepared by a method similar to the method described in [Preparative Example 9].

¹H NMR (400 MHz, DMSO-d6) δ 11.13 (br s, 1H), 9.31 (br d, J=8.76 Hz, 1H), 8.58 (s, 1H), 8.17-8.29 (m, 3H), 7.76 (s, 1H), 7.43 (d, J=7.75 Hz, 1H), 7.33 (dd, J=0.81, 8.07 Hz, 1H), 7.25 (dd, J=3.25, 9.76 Hz, 1H), 7.04 (ddd, J=1.13, 7.04, 8.10 Hz, 1H), 6.91-7.00 (m, 2H), 6.78-6.87 (m, 2H), 6.01 (s, 1H), 3.50-3.62 (m, 2H), 3.10-3.17 (m, 1H), 3.08 (s, 3H), 2.43 (s, 3H), 2.22 (s, 6H), 2.14-2.20 (m, 1H), 1.78-1.89 (m, 1H); LC-MS: 565.65 [M+H+].

### [Example 61]

### 3-[5-[(3S)-3-aminopyrrolidine-1-yl]pyrimidine-2-yl]-N-[(R)-(5-fluoro-2-hydroxy-phenyl)-(1H-indole-2-yl)methyl]-5-methyl-benzamide

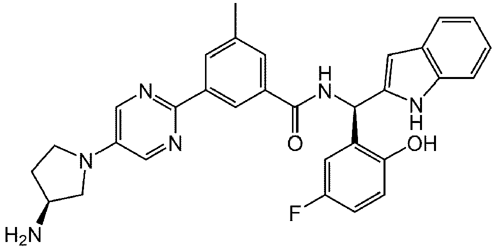

3-[5-[(3S)-3-aminopyrrolidine-1-yl]pyrimidine-2-yl]-N-[(R)-(5-fluoro-2-hydroxy-phenyl)-(1H-indole-2-yl)methyl]-5-methyl-benzamide[Example 61] (21%) was prepared by a method similar to the method described in [Example 1].

¹H NMR (400 MHz, DMSO-d6) δ 11.10 (d, J=1.25 Hz, 1H), 9.26 (d, J=8.63 Hz, 1H), 8.58 (s, 1H), 8.17-8.26 (m, 3H), 7.76 (s, 1H), 7.43 (d, J=7.75 Hz, 1H), 7.30-7.38 (m, 1H), 7.26 (dd, J=3.13, 9.76 Hz, 1H), 7.04 (dt, J=1.13, 7.57 Hz, 1H), 6.91-7.01 (m, 2H), 6.79-6.88 (m, 2H), 6.01 (s, 1H), 3.66 (br d, J=5.25 Hz, 1H), 3.47-3.56 (m, 3H), 3.07 (br dd, J=4.25, 9.88 Hz, 1H), 2.53 (br s, 1H), 2.44 (s, 3H), 2.08-2.15 (m, 1H), 1.75-1.84 (m, 1H); LC-MS: 537.61 [M+H+].

### [Example 62]

### (R)-3-(5-(azetidine-3-yl)pyrimidine-2-yl)-N-((5-fluoro-2-hydroxyphenyl)(1H-indole-2-yl)methyl)-5-methylbenzamide

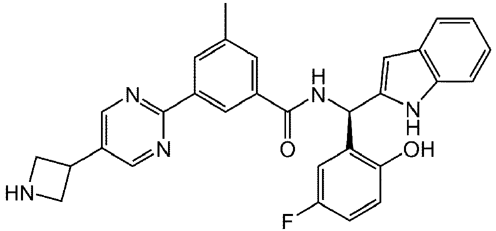

(R)-3-(5-(azetidine-3-yl)pyrimidine-2-yl)-N-((5-fluoro-2-hydroxyphenyl)(1H-indole-2-yl)methyl)-5-methylbenzamide[Example 62] (5%) was prepared by a method similar to the method described in [Example 1].

¹H NMR (400 MHz, Methanol-d4) δ 10.53 (br s, 1H), 9.18 (d, J = 8.4 Hz, 1H), 8.94 (s, 1H), 8.76 (s, 1H), 8.46 (s, 1H), 7.89 (s, 1H), 7.46 (d, J = 8.0 Hz, 1H),7.35 (d, J = 7.2 Hz, 1H), 7.09-7.06 (m, 1H),7.00-6.85 (m, 4H),6.21 (s, 1H), 4.47 - 4.38 (m, 5H), 2.53 (s, 3H); LC-MS: 508.35 [M+H+]

The structures of the compounds prepared in Examples 1-62 are summarized and shown in Table 1 below.

**[Table 1]**

| Example | Structure | Example | Structure |
|---|---|---|---|
| 1 | 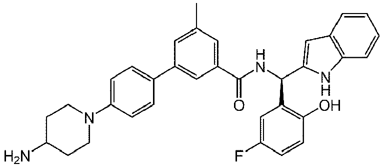 | 2 | 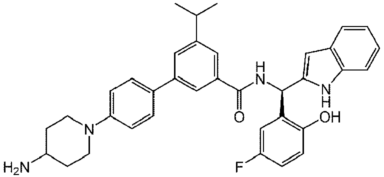 |
| 3 | 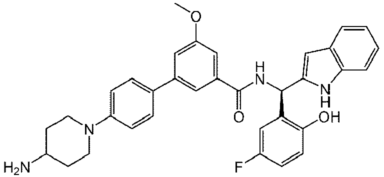 | 4 | 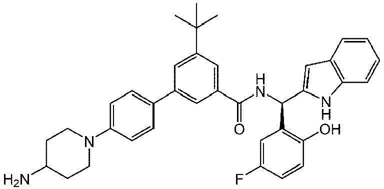 |
| 5 | 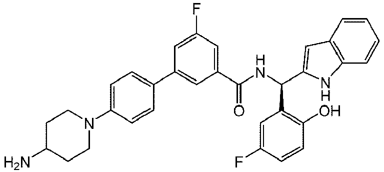 | 6 | 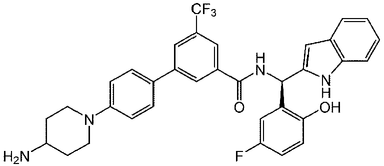 |
| 7 | 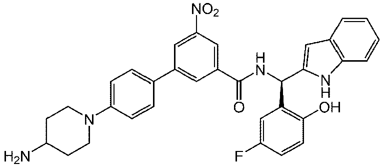 | 8 | 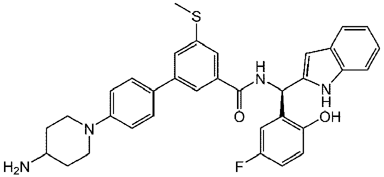 |
| 9 | 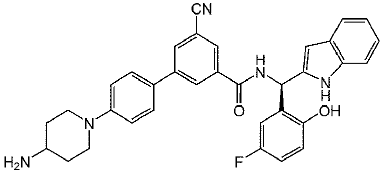 | 10 | 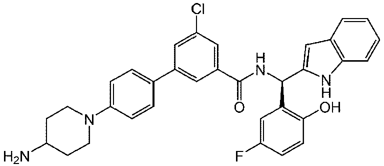 |
| 11 | 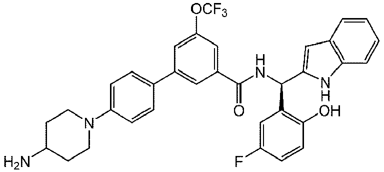 | 12 | 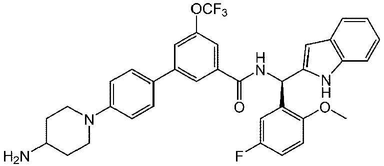 |
| 13 | 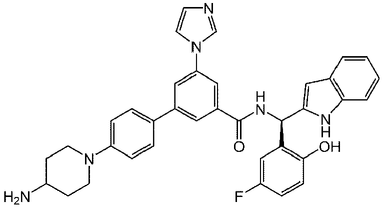 | 14 | 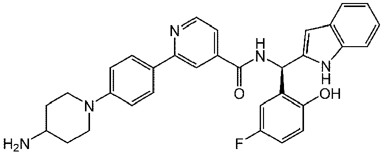 |
| 15 | 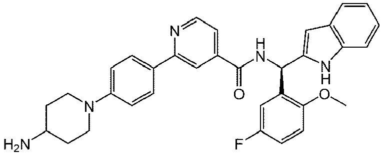 | 16 | 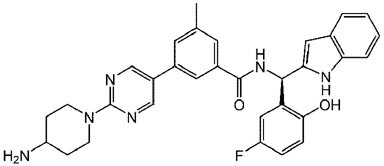 |
| 17 | 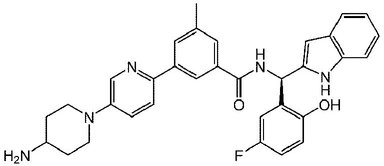 | 18 | 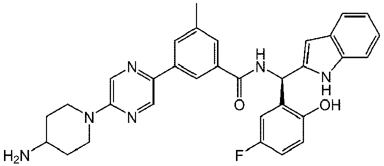 |
| 19 | 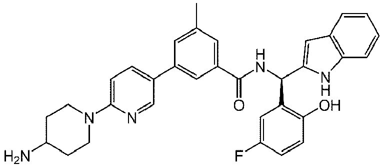 | 20 | 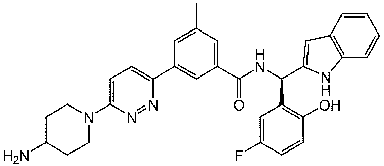 |
| 21 | 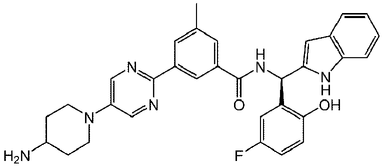 | 22 | 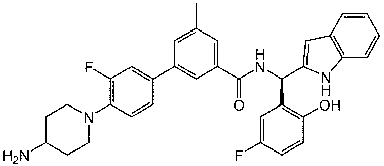 |
| 23 | 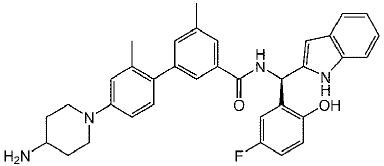 | 24 | 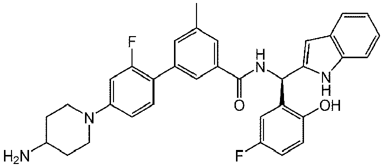 |
| 25 | 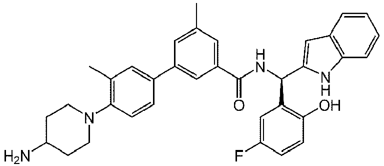 | 26 | 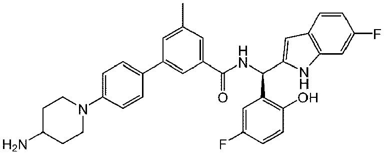 |
| 27 | 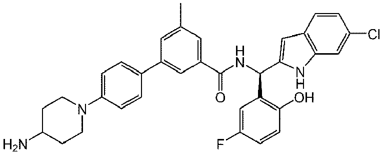 | 28 | 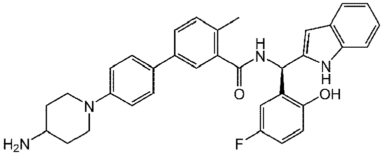 |
| 29 | 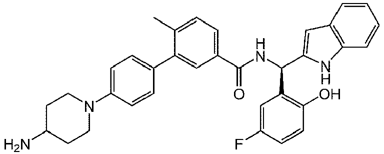 | 30 | 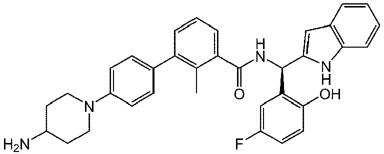 |
| 31 | 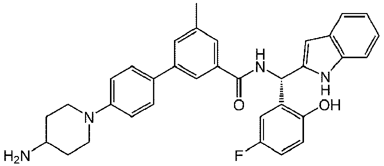 | 32 | 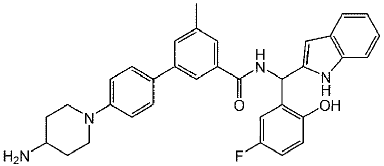 |
| 33 | 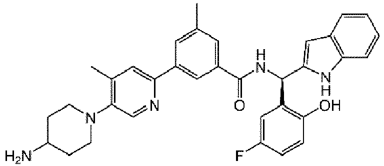 | 34 | 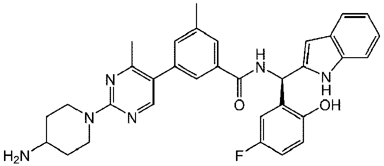 |
| 35 | 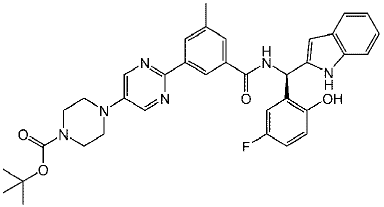 | 36 | 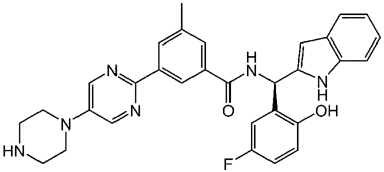 |
| 37 | 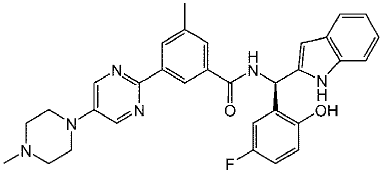 | 38 | 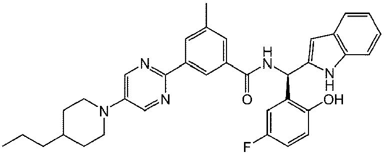 |
| 39 | 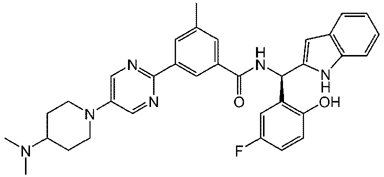 | 40 | 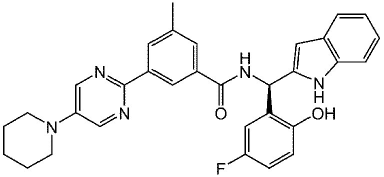 |
| 41 | 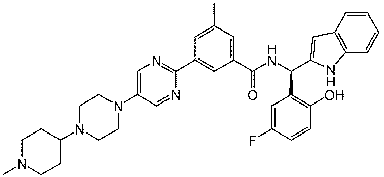 | 42 | 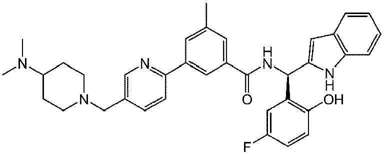 |
| 43 | 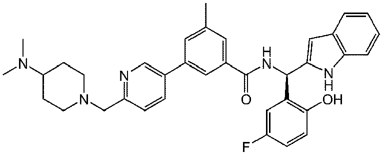 | 44 | 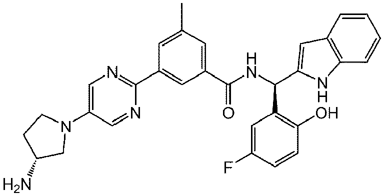 |
| 45 | 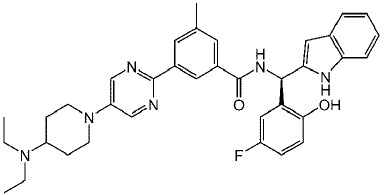 | 46 | 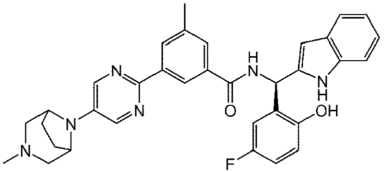 |
| 47 | 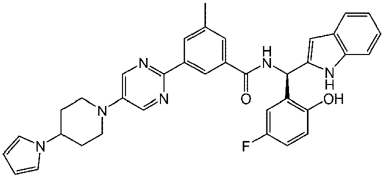 | 48 | 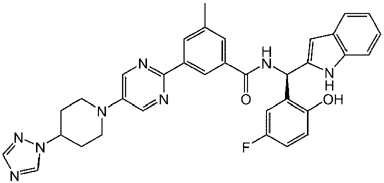 |
| 49 | 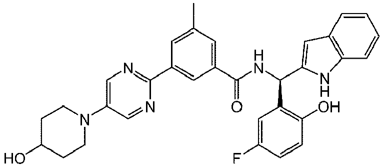 | 50 | 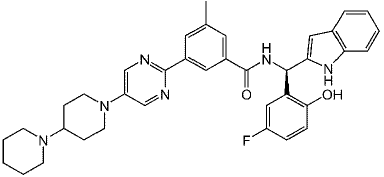 |
| 51 | 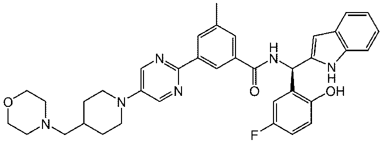 | 52 | 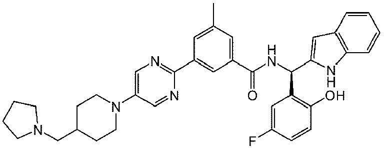 |
| 53 | 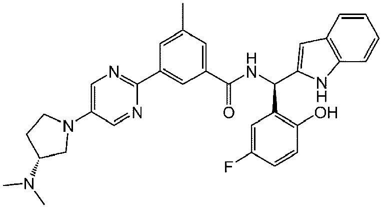 | 54 | 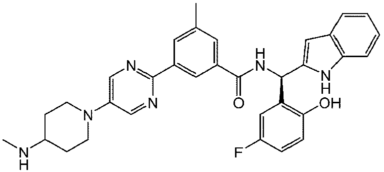 |
| 55 | 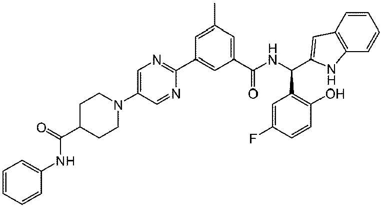 | 56 | 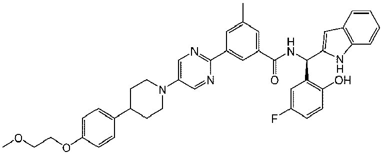 |
| 57 | 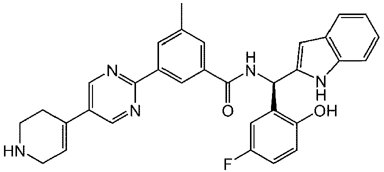 | 58 | 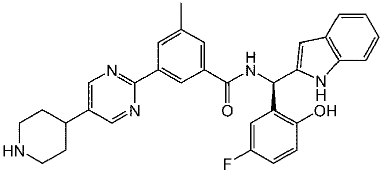 |
| 59 | 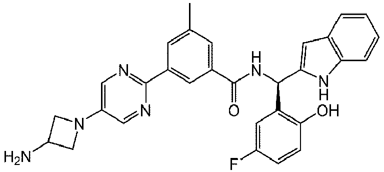 | 60 | 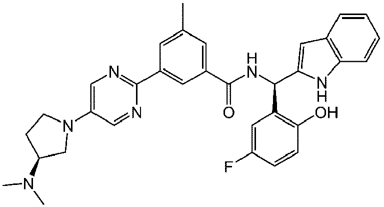 |
| 61 | 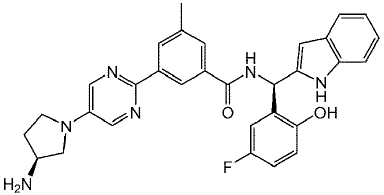 | 62 | 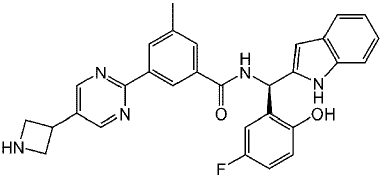 |

### <Experimental Example 1> Evaluation of cell growth inhibitory ability in BaF3 cells overexpressing wild type (WT) and mutant of EGFR

In order to confirm that the compound represented by formula 1 provided in one aspect of the present invention acts as an allosteric inhibitor against WT and mutant of EGFR, the cell growth inhibitory effect was evaluated in BaF3 cells overexpressing WT and mutant of EGFR.

More specifically, the cell growth inhibitory ability of the compound of the present invention to the Ba/F3 cell line overexpressing WT and mutant of EGFR was evaluated using CellTiter 96 AQueous Non-Radioactive Cell Proliferation Assay system of Promega, as follows. AQueous Non-Radioactive Cell Proliferation Assay is a method of confirming cell growth inhibitory ability by measuring the degree of color development by reducing MTS (3-(4,5-Dimethylthiazol-2-yl)-5-(3-carboxymethoxyphenyl)-2-(4-sulfophenyl)-2H-tetrazolium) to Formazan by a reductase secreted by mitochondria present in living cells in a cell culture state. The Ba/F3 EGFR WT and Ba/F3 EGFR del19/T790M/C797S (DTC), Ba/F3 EGFR L858R (L), Ba/F3 EGFR L858R/T790M (LT), and Ba/F3 EGFR L858R/T790M/C797S (LTC) mutation cell lines used in the present invention were purchased from the laboratory of Professor Byung-cheol Cho, Yonsei University. All cell lines were cultured in RPMI supplemented with 10% FBS, 1% penicillin-streptomycin and 1 µg/ml of puromycine in a 5% CO₂ incubator at 37°C.

The cell survival inhibitory effect of the compound according to each EGFR mutation was analyzed by the following analysis method.

BaF3 cells were distributed in a 96-well cell culture plate at the density of 2000-10000 cells/100 µL/well, and cultured for 24 hours. Thereafter, the concentration of the compound represented by formula 1 provided in one aspect of the present invention was prepared to double the final concentrations of 0.1 nM, 1 nM, 10 nM, 100 nM, 1000 nM and 10000 nM, and the compound was treated to the plate (100 µL/well). The plate was reacted in an incubator at 37°C for 72 hours. Then, MTS was treated to the plate (20 µL/well) and the plate was incubated at 37°C for 2 to 4 hours. Finally, after measuring the absorbance at 490 nm using a microplate reader, the relative value compared to 0 nM (% Control) was obtained to confirm the cell growth inhibitory ability of the compound. The measured values were analyzed using Prism program (version 8.0, Graphpad Software, Inc.), and the IC₅₀ value (Inhibition concentration 50), an indicator of the cell growth inhibitory ability of the compound, was calculated.

The results are shown in table 2 below.

**[Table 2]**

| Example | Ba/F3 EGFR double mutant | Ba/F3 EGFR triple mutant |
|---|---|---|
| | L858R/T790M, IC₅₀ (nM) | L858R/T790M/C797S , IC₅₀ (nM) |
| 1 | +++ | +++ |
| 2 | + | + |
| 3 | + | + |
| 4 | + | + |
| 5 | +++ | + |
| 6 | + | + |
| 7 | + | + |
| 8 | + | + |
| 9 | + | + |
| 10 | +++ | ++ |
| 11 | + | + |
| 12 | ++ | + |
| 13 | + | + |
| 14 | ++ | + |
| 15 | + | + |
| 16 | +++ | ++ |
| 17 | +++ | +++ |
| 18 | +++ | +++ |
| 19 | +++ | +++ |
| 20 | ++ | + |
| 21 | +++ | +++ |
| 22 | +++ | +++ |
| 23 | +++ | +++ |
| 24 | +++ | +++ |
| 25 | +++ | +++ |
| 26 | +++ | ++ |
| 27 | ++ | + |
| 28 | + | + |
| 29 | +++ | ++ |
| 30 | + | + |
| 31 | + | + |
| 32 | +++ | +++ |
| 33 | +++ | +++ |
| 34 | +++ | ++ |
| 35 | + | +++ |
| 36 | +++ | +++ |
| 37 | ++ | +++ |
| 38 | + | + |
| 39 | +++ | +++ |
| 40 | +++ | ++ |
| 41 | +++ | +++ |
| 42 | +++ | ++ |
| 43 | +++ | ++ |
| 44 | +++ | +++ |
| 45 | +++ | +++ |
| 46 | - | +++ |
| 47 | - | + |
| 48 | - | ++ |
| 49 | - | +++ |
| 50 | - | +++ |
| 51 | - | + |
| 52 | - | + |
| 53 | - | +++ |
| 54 | - | +++ |
| 55 | - | + |
| 56 | - | + |
| +++: < 30 nM, ++: 30-200 nM, +:>200 nM | | |

As shown in table 2, it was found that the compounds of the present invention exhibit excellent activity against EGFR L858R/T790M and L858R/T790M/C797S mutations.

As mentioned above, the present invention has been described in detail through the preferred preparative examples, examples and experimental examples, but the scope of the present invention is not limited to the specific examples, and should be interpreted by the appended claims. In addition, those of ordinary skill in the art should understand that many modifications and variations are possible without departing from the scope of the present invention.

## Claims

1. A compound represented by formula 1 below, an optical isomer thereof, a solvate thereof, a hydrate thereof, or a pharmaceutically acceptable salt thereof: 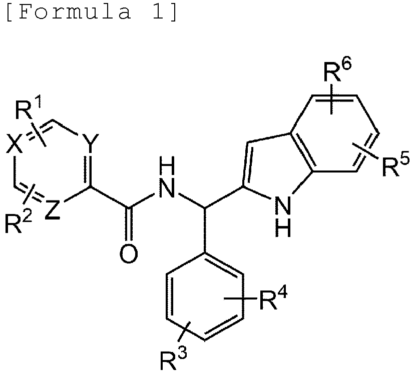 (In formula 1,
X, Y and Z are independently carbon or nitrogen atoms;
R¹ is -H, -OH, halogen, C₁₋₁₀ alkyl unsubstituted or substituted with one or more halogens, C₁₋₁₀ alkoxy unsubstituted or substituted with one or more halogens, nitro, C₁₋₁₀ alkylsulfanyl, cyano, amino, C₁₋₁₀ alkylamino, or 5-6 membered heteroaryl;
R² is substituted C₆₋₁₀ aryl, or substituted 5-10 membered heteroaryl,
wherein the substituted C₆₋₁₀ aryl or substituted 5-10 membered heteroaryl is independently substituted aryl or heteroaryl substituted with one or more substituents selected from the group consisting of halogen, unsubstituted or substituted C₁₋₁₀ alkyl and unsubstituted or substituted 4-10 membered fully or partially saturated heterocycloalkyl,
wherein the substituted C₁₋₁₀ alkyl is substituted with 5-6 membered heterocycloalkyl in which 1 or 2 C₁₋₅ alkyls are substituted with substituted amino,
the substituted 4-10 membered fully or partially saturated heterocycloalkyl is 4-10 membered heterocycloalkyl substituted with one or more substituents selected from the group consisting of - OH, halogen, C₁₋₅ alkyl unsubstituted or substituted with one or more halogens, C₁₋₅ alkyl substituted with 5-6 membered heterocycloalkyl, C₁₋₅ alkoxy unsubstituted or substituted with one or more halogens, nitro, C₁₋₅ alkylsulfanyl, cyano, amino unsubstituted or substituted with 1 or 2 C₁₋₅ alkyls, 5-6 membered heterocycloalkyl unsubstituted or substituted with C₁₋₅ alkyl, C₁₋₅ alkoxycarbonyl, 5-6 membered heteroaryl, C₆₋₁₀ arylaminocarbonyl and C₁₋₅ alkoxy-C₁₋₅ alkoxy-C₆₋₁₀ aryl;
R³ is halogen;
R⁴ is -OH, halogen or C₁₋₁₅ alkoxy;
R⁵ is -H, -OH, halogen, C₁₋₄ alkyl or C₁₋₄ alkoxy; and
R⁶ is -H, -OH, halogen, C₁₋₄ alkyl or C₁₋₄ alkoxy) .

2. The compound, the optical isomer thereof, the solvate thereof, the hydrate thereof, or the pharmaceutically acceptable salt thereof according to claim 1, wherein:
X, Y and Z are independently carbon or nitrogen atoms;
R¹ is -H, -OH, halogen, C₁₋₅ alkyl unsubstituted or substituted with one or more halogens, C₁₋₅ alkoxy unsubstituted or substituted with one or more halogens, nitro, C₁₋₅ alkylsulfanyl, cyano, amino, C₁₋₅ alkylamino, or 5-6 membered heteroaryl;
R² is substituted C₆₋₁₀ aryl, or substituted 5-6 membered heteroaryl,
wherein the substituted C₆₋₁₀ aryl or substituted 5-6 membered heteroaryl is independently aryl or heteroaryl substituted with one or more substituents selected from the group consisting of halogen, unsubstituted or substituted C₁₋₅ alkyl and unsubstituted or substituted 4-8 membered fully or partially saturated heterocycloalkyl containing at least one N,
wherein the substituted C₁₋₅ alkyl is substituted with 6 membered heterocycloalkyl in which 2 C₁₋₅ alkyls are substituted with substituted amino,
the substituted 4-8 membered fully or partially saturated heterocycloalkyl is 4-8 membered heterocycloalkyl substituted with one or more substituents selected from the group consisting of - OH, halogen, C₁₋₅ alkyl unsubstituted or substituted with one or more halogens, C₁₋₅ alkyl substituted with 5-6 membered heterocycloalkyl, C₁₋₅ alkoxy unsubstituted or substituted with one or more halogens, nitro, C₁₋₅ alkylsulfanyl, cyano, amino unsubstituted or substituted with 1 or 2 C₁₋₅ alkyls, 5-6 membered heterocycloalkyl unsubstituted or substituted with C₁₋₅ alkyl, C₁₋₅ alkoxycarbonyl, 5-6 membered heteroaryl, C₆₋₁₀ arylaminocarbonyl and C₁₋₅ alkoxy-C₁₋₅ alkoxy-C₆₋₁₀ aryl;
R³ is halogen;
R⁴ is -OH, halogen or C₁₋₁₀ alkoxy;
R⁵ is -H, -OH or halogen; and
R⁶ is -H, halogen, C₁₋₄ alkyl or C₁₋₄ alkoxy.

3. The compound, the optical isomer thereof, the solvate thereof, the hydrate thereof, or the pharmaceutically acceptable salt thereof according to claim 1, wherein:
X, Y and Z are independently carbon or nitrogen atoms;
R¹ is -H, -OH, halogen, C₁₋₅ alkyl unsubstituted or substituted with one or more halogens, C₁₋₅ alkoxy unsubstituted or substituted with one or more halogens, nitro, C₁₋₅ alkylsulfanyl, cyano, amino, C₁₋₅ alkylamino, or 5 membered heteroaryl containing at least one N;
R² is substituted phenyl, or substituted 6 membered heteroaryl containing at least one N,
wherein the substituted phenyl or substituted 6 membered heteroaryl is independently phenyl or heteroaryl substituted with one or more substituents selected from the group consisting of halogen, unsubstituted or substituted C₁₋₃ alkyl and unsubstituted or substituted 4-8 membered fully or partially saturated heterocycloalkyl containing at least one N,
wherein the substituted C₁₋₃ alkyl is substituted with 6 membered heterocycloalkyl in which 2 C₁₋₃ alkyls are substituted with substituted amino,
the substituted 4-8 membered heterocycloalkyl is 4-8 membered heterocycloalkyl substituted with one or more substituents selected from the group consisting of -OH, C₁₋₃ alkyl unsubstituted or substituted with 5-6 membered heterocycloalkyl, amino unsubstituted or substituted with 1 or 2 C₁₋₃ alkyls, 5-6 membered heterocycloalkyl unsubstituted or substituted with C₁₋₃ alkyl, C₁₋₄ alkoxycarbonyl, 5 membered heteroaryl containing at least one N, phenylaminocarbonyl and C₁₋₃ alkoxy-C₁₋₃ alkoxy-phenyl;
R³ is halogen;
R⁴ is -OH, halogen or C₁₋₅ alkoxy;
R⁵ is -H, -OH or halogen; and
R⁶ is -H, halogen, C₁₋₄ alkyl or C₁₋₄ alkoxy.

4. The compound, the optical isomer thereof, the solvate thereof, the hydrate thereof, or the pharmaceutically acceptable salt thereof according to claim 1, wherein:
X, Y and Z are independently carbon or nitrogen atoms;
R¹ is -H, -F, -Cl, -OH, -CH₃, -CF₃, -OCH₃, -OCF₃, iso-propyl, tert-butyl, -SCH₃, -NO₂, -CN or imidazole,
R² is 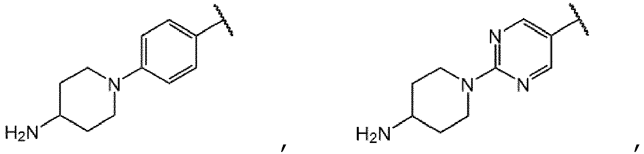 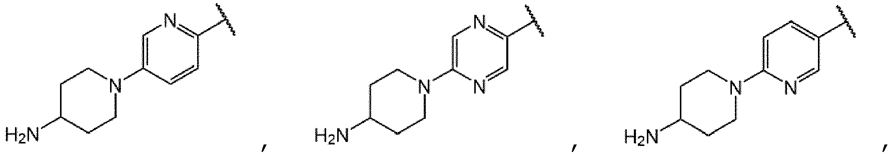 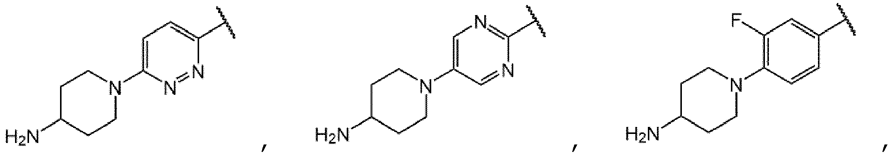 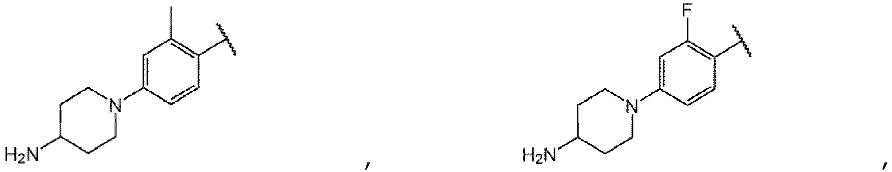 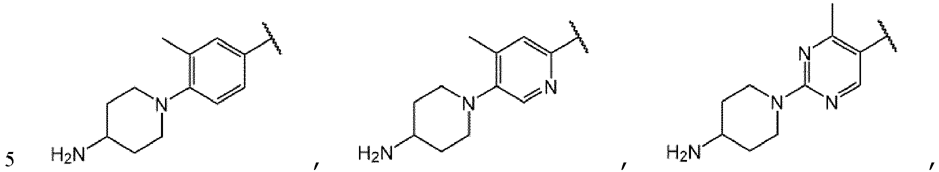 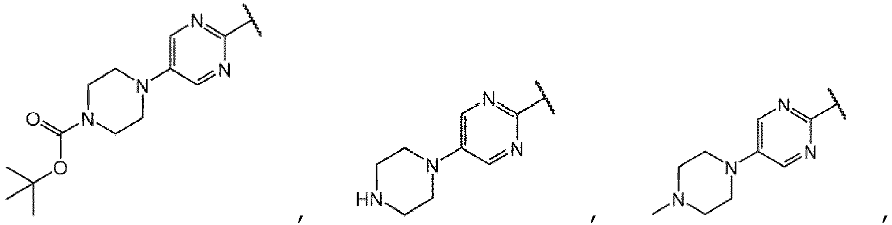 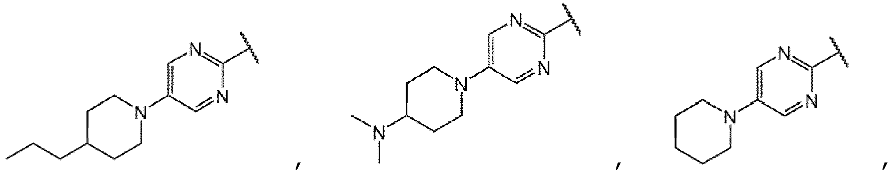 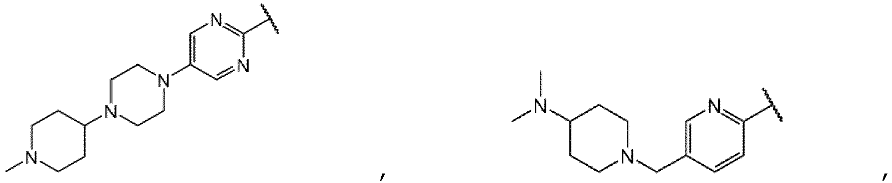 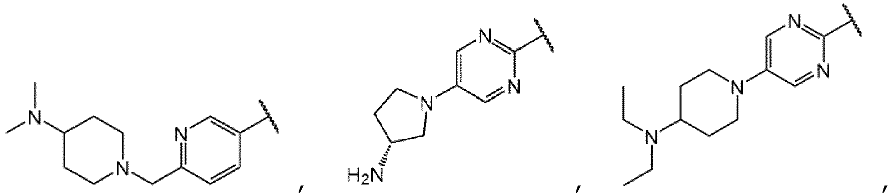 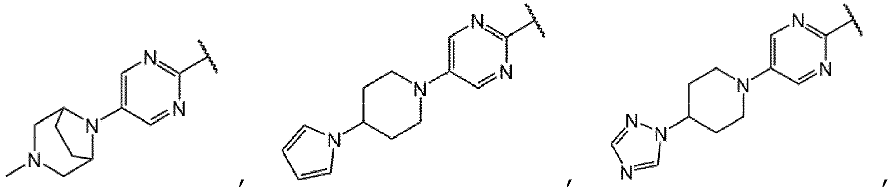 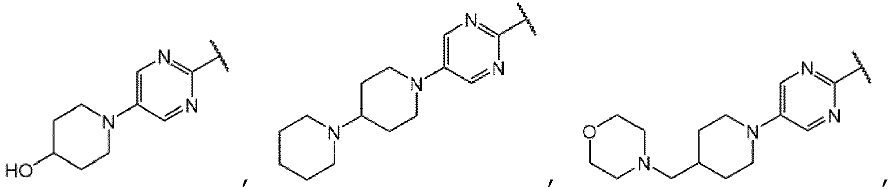 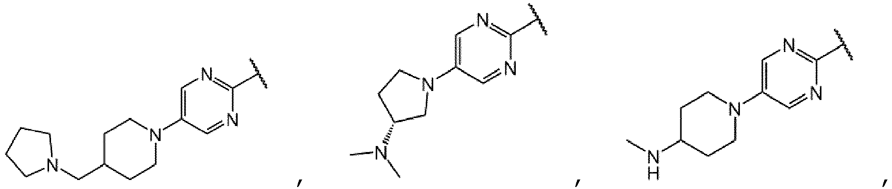 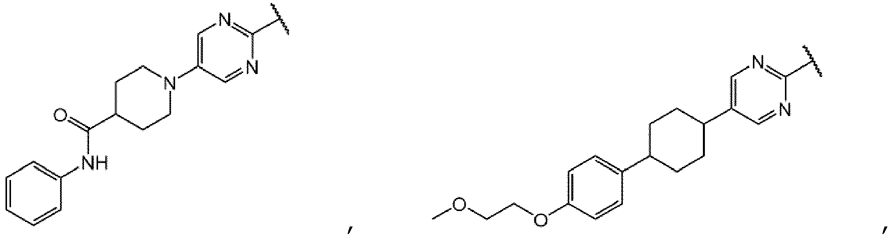 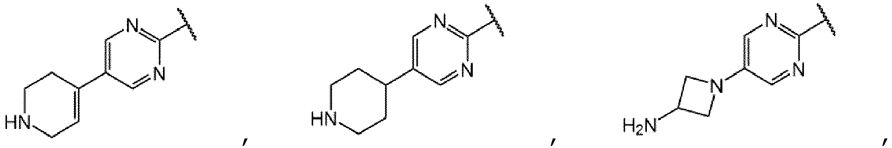 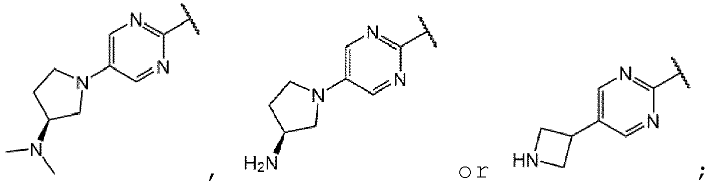
R³ is -F or -Cl;
R⁴ is -OH or -OCH₃;
R⁵ is -H, -OH, -F, -Cl, C₁₋₄ alkyl or C₁₋₄ alkoxy; and
R⁶ is -H, -OH, -F, -Cl, C₁₋₄ alkyl or C₁₋₄ alkoxy.

5. The compound, the optical isomer thereof, the solvate thereof, the hydrate thereof, or the pharmaceutically acceptable salt thereof according to claim 1, wherein the compound represented by formula 1 is a compound represented by formula 2 below: 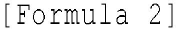 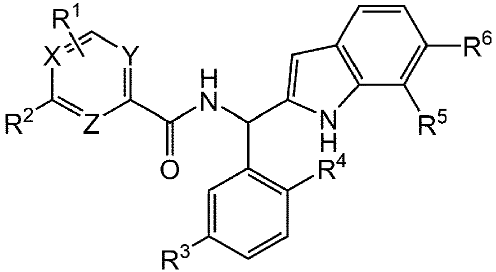 (In formula 2, X, Y, Z, R¹, R², R³, R⁴, R⁵ and R⁶ are independently as defined in formula 1 of claim 1).

6. The compound, the optical isomer thereof, the solvate thereof, the hydrate thereof, or the pharmaceutically acceptable salt thereof according to claim 1, wherein:
X is carbon or nitrogen atoms;
Y and Z are carbon atoms;
R¹ is -H, -F, -Cl, -OH, -CH₃, -CF₃, -OCH₃, -OCF₃, iso-propyl, tert-butyl, -SCH₃, -NO₂, -CN or imidazole,
R² is 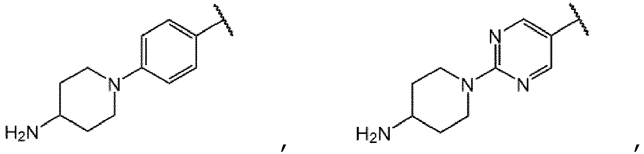 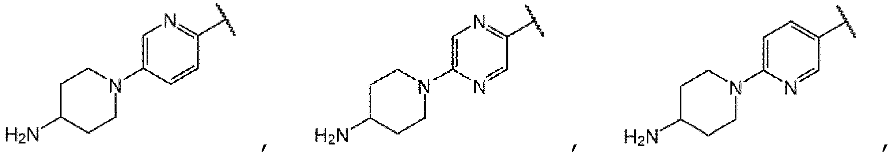 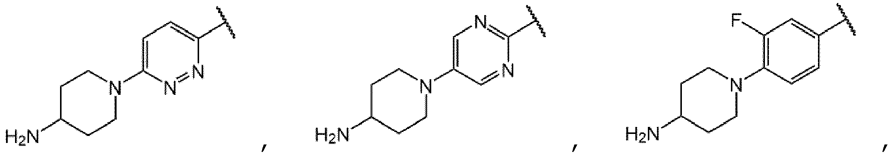 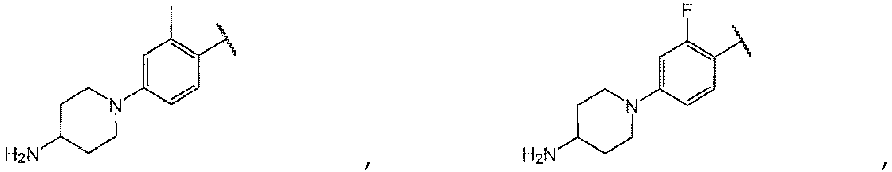 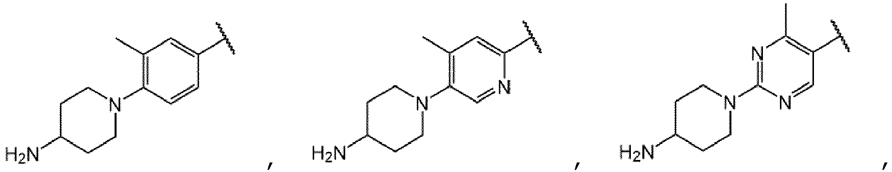 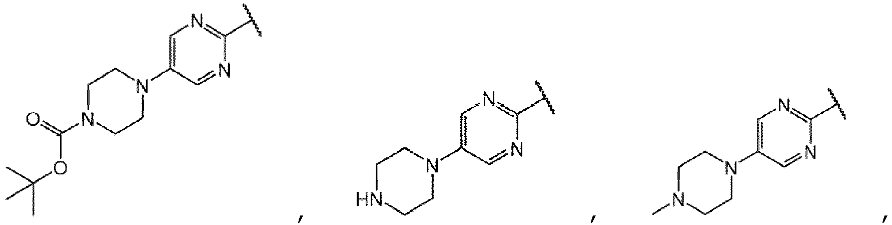 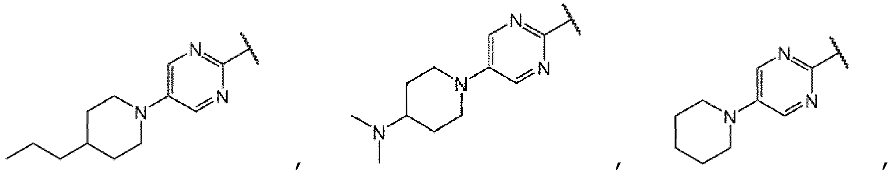 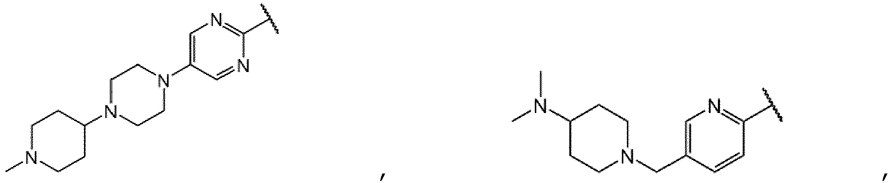 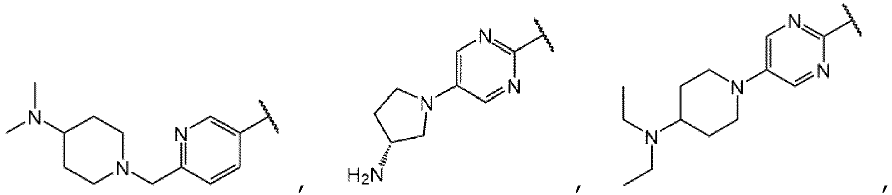 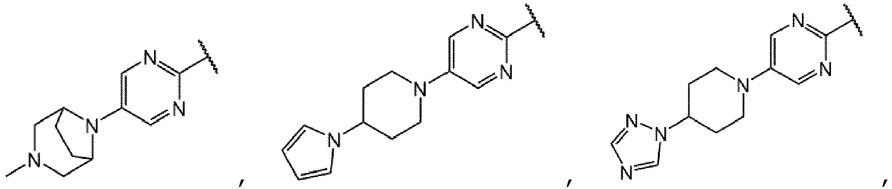 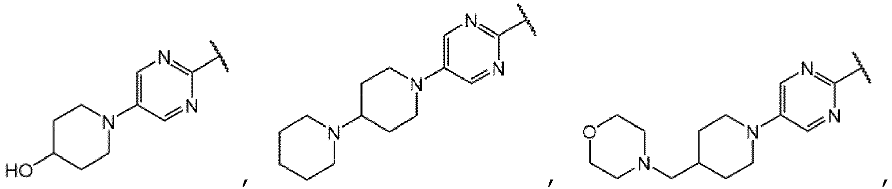 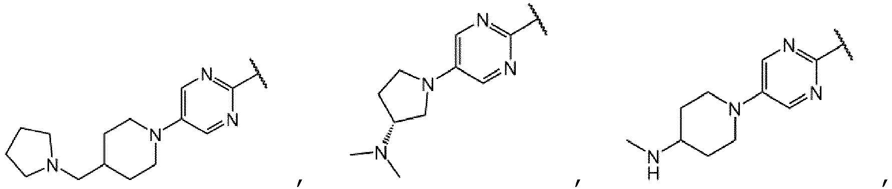 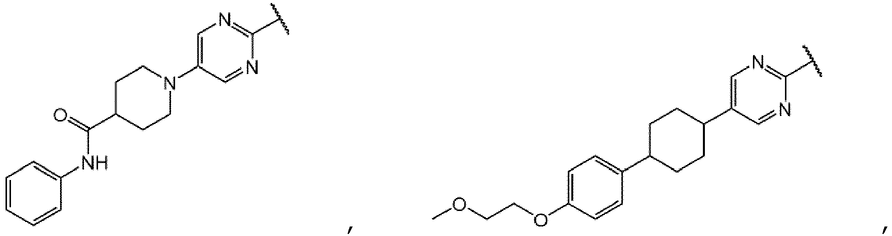 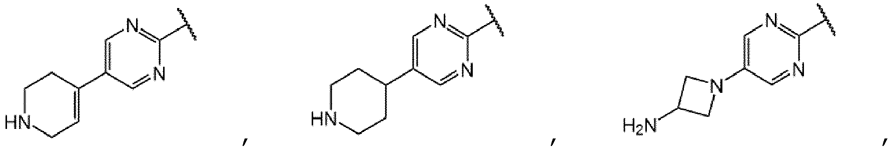 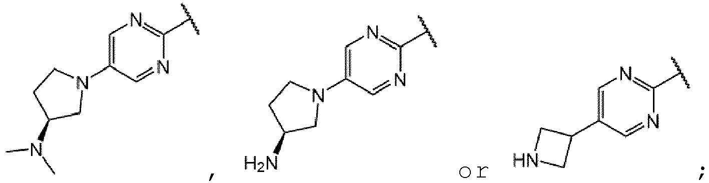
R³ is -F;
R⁴ is -OH or -OCH₃;
R⁵ is -H; and
R⁶ is -H, -F or -Cl.

7. The compound, the optical isomer thereof, the solvate thereof, the hydrate thereof, or the pharmaceutically acceptable salt thereof according to claim 1, wherein the compound represented by formula 1 is selected from the group consisting of the following compounds:
(1) (R)-4'-(4-aminopiperidine-1-yl)-N-((5-fluoro-2-hydroxyphenyl)(1H-indole-2-yl)methyl)-5-methyl-[1,1'-biphenyl]-3-carboxamide;
(2) (R)-4'-(4-aminopiperidine-1-yl)-N-((5-fluoro-2-hydroxyphenyl)(1H-indole-2-yl)methyl)-5-isopropyl-[1,1'-biphenyl]-3-carboxamide;
(3) (R)-4'-(4-aminopiperidine-1-yl)-N-((5-fluoro-2-hydroxyphenyl)(1H-indole-2-yl)methyl)-5-methoxy-[1,1'-biphenyl]-3-carboxamide;
(4) (R)-4'-(4-aminopiperidine-1-yl)-5-(tert-butyl)-N-((5-fluoro-2-hydroxyphenyl)(1H-indole-2-yl)methyl)-[1,1'-biphenyl]-3-carboxamide;
(5) (R)-4'-(4-aminopiperidine-1-yl)-5-fluoro-N-((5-fluoro-2-hydroxyphenyl)(1H-indole-2-yl)methyl)-[1,1'-biphenyl]-3-carboxamide;
(6) (R)-4'-(4-aminopiperidine-1-yl)-N-((5-fluoro-2-hydroxyphenyl)(1H-indole-2-yl)methyl)-5-(trifluoromethyl)-[1,1'-biphenyl]-3-carboxamide;
(7) (R)-4'-(4-aminopiperidine-1-yl)-N-((5-fluoro-2-hydroxyphenyl)(1H-indole-2-yl)methyl)-5-nitro-[1,1'-biphenyl]-3-carboxamide;
(8) (R)-4'-(4-aminopiperidine-1-yl)-N-((5-fluoro-2-hydroxyphenyl)(1H-indole-2-yl)methyl)-5-(methylthio)-[1,1'-biphenyl]-3-carboxamide;
(9) (R)-4'-(4-aminopiperidine-1-yl)-5-cyano-N-((5-fluoro-2-hydroxyphenyl)(1H-indole-2-yl)methyl)-[1,1'-biphenyl]-3-carboxamide;
(10) (R)-4'-(4-aminopiperidine-1-yl)-5-chloro-N-((5-fluoro-2-hydroxyphenyl)(1H-indole-2-yl)methyl)-[1,1'-biphenyl]-3-carboxamide;
(11) (R)-4'-(4-aminopiperidine-1-yl)-N-((5-fluoro-2-hydroxyphenyl)(1H-indole-2-yl)methyl)-5-(trifluoromethoxy)-[1,1'-biphenyl]-3-carboxamide;
(12) (R)-4'-(4-aminopiperidine-1-yl)-N-((5-fluoro-2-methoxyphenyl)(1H-indole-2-yl)methyl)-5-(trifluoromethoxy)-[1,1'-biphenyl]-3-carboxamide;
(13) (R)-4'-(4-aminopiperidine-1-yl)-N-((5-fluoro-2-hydroxyphenyl)(1H-indole-2-yl)methyl)-5-(1H-imidazole-1-yl)-[1,1'-biphenyl]-3-carboxamide;
(14) (R)-2-(4-(4-aminopiperidine-1-yl)phenyl)-N-((5-fluoro-2-hydroxyphenyl)(1H-indole-2-yl)methyl)isonicotinamide;
(15) (R)-2-(4-(4-aminopiperidine-1-yl)phenyl)-N-((5-fluoro-2-methoxyphenyl)(1H-indole-2-yl)methyl)isonicotinamide;
(16) (R)-3-(2-(4-aminopiperidine-1-yl)pyrimidine-5-yl)-N-((5-fluoro-2-hydroxyphenyl)(1H-indole-2-yl)methyl)-5-methylbenzamide;
(17) (R)-3-(5-(4-aminopiperidine-1-yl)pyridine-2-yl)-N-((5-fluoro-2-hydroxyphenyl)(1H-indole-2-yl)methyl)-5-methylbenzamide;
(18) (R)-3-(5-(4-aminopiperidine-1-yl)pyrazine-2-yl)-N-((5-fluoro-2-hydroxyphenyl)(1H-indole-2-yl)methyl)-5-methylbenzamide;
(19) (R)-3-(6-(4-aminopiperidine-1-yl)pyridine-3-yl)-N-((5-fluoro-2-hydroxyphenyl)(1H-indole-2-yl)methyl)-5-methylbenzamide;
(20) (R)-3-(6-(4-aminopiperidine-1-yl)pyridazine-3-yl)-N-((5-fluoro-2-hydroxyphenyl)(1H-indole-2-yl)methyl)-5-methylbenzamide;
(21) (R)-3-(5-(4-aminopiperidine-1-yl)pyrimidine-2-yl)-N-((5-fluoro-2-hydroxyphenyl)(1H-indole-2-yl)methyl)-5-methylbenzamide;
(22) (R)-4'-(4-aminopiperidine-1-yl)-3'-fluoro-N-((5-fluoro-2-hydroxyphenyl)(1H-indole-2-yl)methyl)-5-methyl-[1,1'-biphenyl]-3-carboxamide;
(23) (R)-4'-(4-aminopiperidine-1-yl)-N-((5-fluoro-2-hydroxyphenyl)(1H-indole-2-yl)methyl)-2',5-dimethyl-[1,1'-biphenyl]-3-carboxamide;
(24) (R)-4'-(4-aminopiperidine-1-yl)-2'-fluoro-N-((5-fluoro-2-hydroxyphenyl)(1H-indole-2-yl)methyl)-5-methyl-[1,1'-biphenyl]-3-carboxamide;
(25) (R)-4'-(4-aminopiperidine-1-yl)-N-((5-fluoro-2-hydroxyphenyl)(1H-indole-2-yl)methyl)-3',5-dimethyl-[1,1'-biphenyl]-3-carboxamide;
(26) (R)-4'-(4-aminopiperidine-1-yl)-N-((6-fluoro-1H-indole-2-yl)(5-fluoro-2-hydroxyphenyl)methyl)-5-methyl-[1,1'-biphenyl]-3-carboxamide;
(27) (R)-4'-(4-aminopiperidine-1-yl)-N-((6-chloro-1H-indole-2-yl)(5-fluoro-2-hydroxyphenyl)methyl)-5-methyl-[1,1'-biphenyl]-3-carboxamide;
(28) (R)-4'-(4-aminopiperidine-1-yl)-N-((5-fluoro-2-hydroxyphenyl)(1H-indole-2-yl)methyl)-6-methyl-[1,1'-biphenyl]-3-carboxamide;
(29) (R)-4'-(4-aminopiperidine-1-yl)-N-((5-fluoro-2-hydroxyphenyl)(1H-indole-2-yl)methyl)-4-methyl-[1,1'-biphenyl]-3-carboxamide;
(30) (R)-4'-(4-aminopiperidine-1-yl)-N-((5-fluoro-2-hydroxyphenyl)(1H-indole-2-yl)methyl)-2-methyl-[1,1'-biphenyl]-3-carboxamide;
(31) (S)-4'-(4-aminopiperidine-1-yl)-N-((5-fluoro-2-hydroxyphenyl)(1H-indole-2-yl)methyl)-5-methyl-[1,1'-biphenyl]-3-carboxamide;
(32) 4'-(4-aminopiperidine-1-yl)-N-((5-fluoro-2-hydroxyphenyl)(1H-indole-2-yl)methyl)-5-methyl-[1,1'-biphenyl]-3-carboxamide;
(33) (R)-3-(5-(4-aminopiperidine-1-yl)-4-methylpyridine-2-yl)-N-((5-fluoro-2-hydroxyphenyl)(1H-indole-2-yl)methyl)-5-methylbenzamide;
(34) (R)-3-(2-(4-aminopiperidine-1-yl)-4-methylpyrimidine-5-yl)-N-((5-fluoro-2-hydroxyphenyl)(1H-indole-2-yl)methyl)-5-methylbenzamide;
(35) tert-butyl (R)-4-(2-(3-(((5-fluoro-2-hydroxyphenyl)(1H-indole-2-yl)methyl)carbamoyl)-5-methylphenyl)pyrimidine-5-yl)piperazine-1-carboxylate;
(36) (R)-N-((5-fluoro-2-hydroxyphenyl)(1H-indole-2-yl)methyl)-3-methyl-5-(5-(piperazine-1-yl)pyrimidine-2-yl)benzamide;
(37) (R)-N-((5-fluoro-2-hydroxyphenyl)(1H-indole-2-yl)methyl)-3-methyl-5-(5-(4-methylpiperazine-1-yl)pyrimidine-2-yl)benzamide;
(38) N-[(R)-(5-fluoro-2-hydroxy-phenyl)-(1H-indole-2-yl)methyl]-3-methyl-5-[5-(4-propyl-1-piperidyl)pyrimidine-2-yl]benzamide;
(39) 3-[5-[4-(dimethylamino)-1-piperidyl]pyrimidine-2-yl]-N-[(R)-(5-fluoro-2-hydroxyphenyl)-(1H-indole-2-yl)methyl]-5-methyl-benzamide;
(40) N-[(R)-(5-fluoro-2-hydroxy-phenyl)-(1H-indole-2-yl)methyl]-3-methyl-5-[5-(1-piperidyl)pyrimidine-2-yl]benzamide;
(41) N-[(R)-(5-fluoro-2-hydroxy-phenyl)-(1H-indole-2-yl)methyl]-3-methyl-5-[5-[4-(1-methyl-4-piperidyl)piperazine-1-yl]pyrimidine-2-yl]benzamide;
(42) (R)-3-(5-((4-(dimethylamino)piperidine-1-yl)methyl)pyridine-2-yl)-N-((5-fluoro-2-hydroxyphenyl)(1H-indole-2-yl))methyl)-5-methylbenzamide;
(43) (R)-3-(6-((4-(dimethylamino)piperidine-1-yl)methyl)pyridine-3-yl)-N-((5-fluoro-2-hydroxyphenyl)(1H-indole-2-yl))methyl)-5-methylbenzamide;
(44) 3-[5-[(3R)-3-aminopyrrolidine-1-yl]pyrimidine-2-yl]-N-[(R)-(5-fluoro-2-hydroxyphenyl)-(1H-indole-2-yl)methyl]-5-methyl-benzamide;
(45) (R)-3-(5-(4-(diethylamino)piperidine-1-yl)pyrimidine-2-yl)-N-((5-fluoro-2-hydroxyphenyl)(1H-indole-2-yl)methyl)-5-methylbenzamide;
(46) N-((R)-(5-fluoro-2-hydroxyphenyl)(1H-indole-2-yl)methyl)-3-methyl-5-(5-(3-methyl-3,8-diazabicyclo[3.2.1]octane-8-yl)pyrimidine-2-yl)benzamide;
(47) (R)-3-(5-(4-(1H-pyrrole-1-yl)piperidine-1-yl)pyrimidine-2-yl)-N-((5-fluoro-2-hydroxyphenyl)(1H-indole-2-yl)methyl)-5-methylbenzamide;
(48) (R)-3-(5-(4-(1H-1,2,4-triazole-1-yl)piperidine-1-yl)pyrimidine-2-yl)-N-((5-fluoro-2-hydroxyphenyl)(1H-indole-2-yl)methyl)-5-methylbenzamide;
(49) (R)-N-((5-fluoro-2-hydroxyphenyl)(1H-indole-2-yl)methyl)-3-(5-(4-hydroxypiperidine-1-yl)pyrimidine-2-yl)-5-methylbenzamide;
(50) (R)-3-(5-([1,4'-bipiperidine]-1'-yl)pyrimidine-2-yl)-N-((5-fluoro-2-hydroxyphenyl)(1H-indole-2-yl)methyl)-5-methylbenzamide;
(51) (R)-N-((5-fluoro-2-hydroxyphenyl)(1H-indole-2-yl)methyl)-3-methyl-5-(5-(4-(morpholinomethyl)piperidine-1-yl)pyrimidine-2-yl)benzamide;
(52) (R)-N-((5-fluoro-2-hydroxyphenyl)(1H-indole-2-yl)methyl)-3-methyl-5-(5-(4-(pyrrolidine-1-ylmethyl)piperidine-1-yl)pyrimidine-2-yl)benzamide;
(53) 3-[5-[(3R)-3-(dimethylamino)pyrrolidine-1-yl]pyrimidine-2-yl]-N-[(R)-(5-fluoro-2-hydroxyphenyl)-(1H-indole-2-yl)methyl]-5-methyl-benzamide;
(54) (R)-N-((5-fluoro-2-hydroxyphenyl)(1H-indole-2-yl)methyl)-3-methyl-5-(5-(4-(methylamino)piperidine-1-yl)pyrimidine-2-yl)benzamide;
(55) (R)-1-(2-(3-(((5-fluoro-2-hydroxyphenyl)(1H-indole-2-yl)methyl)carbamoyl)-5-methylphenyl)pyrimidine-5-yl)-N-phenylpiperidine-4-carboxamide;
(56) (R)-N-((5-fluoro-2-hydroxyphenyl)(1H-indole-2-yl)methyl)-3-(5-(4-(4-(2-methoxyethoxy)phenyl)piperazine-1-yl)pyrimidine-2-yl)-5-methylbenzamide;
(57) (R)-N-((5-fluoro-2-hydroxyphenyl)(1H-indole-2-yl)methyl)-3-methyl-5-(5-(1,2,3,6-tetrahydropyridine-4-yl)pyrimidine-2-yl)benzamide;
(58) (R)-N-((5-fluoro-2-hydroxyphenyl)(1H-indole-2-yl)methyl)-3-methyl-5-(5-(piperidine-4-yl)pyrimidine-2-yl)benzamide;
(59) (R)-3-(5-(3-aminoazetidine-1-yl)pyrimidine-2-yl)-N-((5-fluoro-2-hydroxyphenyl)(1H-indole-2-yl)methyl)-5-methylbenzamide;
(60) 3-(5-((S)-3-(dimethylamino)pyrrolidine-1-yl)pyrimidine-2-yl)-N-((R)-(5-fluoro-2-hydroxyphenyl)(1H-indole-2-yl)methyl)-5-methylbenzamide;
(61) 3-(5-((S)-3-aminopyrrolidine-1-yl)pyrimidine-2-yl)-N-((R)-(5-fluoro-2-hydroxyphenyl)(1H-indole-2-yl) methyl)-5-methylbenzamide; and
(62) (R)-3-(5-(azetidine-3-yl)pyrimidine-2-yl)-N-((5-fluoro-2-hydroxyphenyl)(1H-indole-2-yl)methyl)-5-methylbenzamide.

8. A pharmaceutical composition comprising a compound represented by formula 1 of claim 1, an optical isomer thereof, a solvate thereof, a hydrate thereof, or a pharmaceutically acceptable salt thereof as an active ingredient for the prevention or treatment of cancer.

9. The pharmaceutical composition according to claim 8, wherein the compound inhibits EGFR (epidermal growth factor receptor) mutation to prevent or treat cancer.

10. The pharmaceutical composition according to claim 9, wherein the EGFR (epidermal growth factor receptor) mutation is at least one selected from the group consisting of EGFR L858R/T790M and EGFR L858R/T790M/C797S.

11. A health functional food composition comprising a compound represented by formula 1 of claim 1, an optical isomer thereof, a solvate thereof, a hydrate thereof, or a pharmaceutically acceptable salt thereof as an active ingredient for the prevention or amelioration of cancer.

12. A combination preparation comprising a compound represented by formula 1 of claim 1, an optical isomer thereof, a solvate thereof, a hydrate thereof, or a pharmaceutically acceptable salt thereof as an active ingredient for the prevention or treatment of cancer.

13. The combination preparation according to claim 12, wherein the combination preparation is administered in combination with at least one selected from the group consisting of the following components:
cisplatin, oxaliplatin, carboplatin, cyclophosphamide, nitrogen mustard, melphalan, chlorambucil, busulfan, temozolamide, nitrosourea, gemcitabine, antifolate, 5-fluorouracil, tegafur, raltitrexed, methotrexate, cytosine arabinoside, hydroxyurea, adriamycin, bleomycin, doxorubicin, daunomycin, epirubicin, idarubicin, mitomycin C, dactinomycin, mithramycin, vincristine, vinblastine, vindesine, vinorelbine, taxol, taxotere, etoposide, teniposide, amsacrine, topotecan, camptothecin, tamoxifen, fulvestrant, toremifene, raloxifene, droloxifene, iodooxyphene, bicalutamide, flotamide, nilutamide, cyproterone acetate, goserelin, leuprorelin, buserellin, megestrol acetate, anastrozole, letrozole, borazole, eximestane, finasteride, saracatinib, dasatinib, bosutinib, marimastat, cetuximab, gefitinib, erlotinib, panitumumab, osimertinib, lapatinib, imatinib, nilotinib, sorafenib, tipifarnib, lonafarnib, bevacizumab, vandetanib, vatalanib, sunitinib, axitinib, pazopanib, 4-(4-fluoro-2-methylindole-5-yloxy)-6-methoxy-7-(3-pyrrolidine -1-ylpropoxy)quinazolineline, combretastatin A4, zibotentan and atrasentan.

14. A method for preventing or treating cancer comprising a step of administering a compound represented by formula 1 of claim 1, an optical isomer thereof, a solvate thereof, a hydrate thereof, or a pharmaceutically acceptable salt thereof to a subject in need.

15. A use of a compound represented by formula 1 of claim 1, an optical isomer thereof, a solvate thereof, a hydrate thereof, or a pharmaceutically acceptable salt thereof for the manufacture of a medicament for the prevention or treatment of cancer.
